(19) Europäisches Patentamt
European Patent Office
Office européen des brevets

(11) EP 4 501 359 A1

(12) **EUROPEAN PATENT APPLICATION**
published in accordance with Art. 153(4) EPC

(43) Date of publication:
**05.02.2025 Bulletin 2025/06**

(21) Application number: **23778866.6**

(22) Date of filing: **06.02.2023**

(51) International Patent Classification (IPC):
***A61K 47/18*** (2017.01) ***A61K 9/127*** (2025.01)
***A61K 9/51*** (2006.01) ***A61K 31/7088*** (2006.01)
***A61K 31/7105*** (2006.01) ***A61K 31/713*** (2006.01)
***A61K 48/00*** (2006.01) ***A61P 1/16*** (2006.01)
***A61P 43/00*** (2006.01) ***C07C 219/06*** (2006.01)
***C12N 15/09*** (2006.01) ***C12N 15/113*** (2010.01)
***C12N 15/88*** (2006.01)

(52) Cooperative Patent Classification (CPC):
**A61K 9/127; A61K 9/51; A61K 31/7088; A61K 31/7105; A61K 31/713; A61K 47/18; A61K 48/00; A61P 1/16; A61P 43/00; C07C 219/06; C12N 15/09; C12N 15/113; C12N 15/88**

(86) International application number:
**PCT/JP2023/003844**

(87) International publication number:
**WO 2023/188830 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022060960**

(71) Applicant: **National University Corporation Hokkaido University**
**Hokkaido 060-0808 (JP)**

(72) Inventors:
- **SATO Yusuke**
**Sapporo-shi, Hokkaido 060-0808 (JP)**
- **HARASHIMA Hideyoshi**
**Sapporo-shi, Hokkaido 060-0808 (JP)**
- **ONUMA Haruno**
**Sapporo-shi, Hokkaido 060-0808 (JP)**

(74) Representative: **Forstmeyer, Dietmar**
**Boeters & Lieck**
**Oberanger 32**
**80331 München (DE)**

Remarks:
The complete document including Reference Table(s) and the Sequence Listing(s) can be downloaded from the EPO website

(54) **LIPID NANOPARTICLES**

(57) The present invention provides lipid nanoparticles each containing a pH-sensitive cationic lipid represented by formula (I) [wherein a represents an integer of 3 to 5; b represents 0 or 1; $R^1$ and $R^2$ each independently represent a group represented by general formula (A) (wherein $R^{11}$ and $R^{12}$ each independently represent a linear or branched C1-15 alkyl group; c represents an integer of 1 to 7: and e represents an integer of 4 to 12); and X represents a group represented by general formula (B) (wherein d represents an integer of 0 to 3; and $R^3$ and $R^4$ each independently represent a $C_{1-4}$ alkyl group or a $C_{2-4}$ alkenyl group, in which $R^3$ and $R^4$ may be bonded to each other to form a 5- to 7-membered nonaromatic hetero ring) or a 5- to 7-membered nonaromatic hetero ring group]. $(R^1)(R^2)C(OH)-(CH_2)a-(O-CO)b-X$ (I) $(R^{11})(R^{12})HC-(CH_2)c-(CO-O)-(CH_2)e-$(A) $-(CH_2)d-N(R^3)(R^4)$ (B)
[Chem. 1]

$$(R^1)\ (R^2)C(OH)-(CN_2)a-(O-CO)b-X \qquad (I)$$



EP 4 501 359 A1

$(R^{11})(R^{12})HC-(CH_2)_c-(CO-O)-(CH_2)_e-$ (A)

$-(CH_2)d-N(R^3)(R^4)$ (B)

## Description

[TECHNICAL FIELD]

The present invention relates to lipid nanoparticles and their constituent lipids that are useful as gene delivery carriers for liver cells and spleen cells.

This application claims priority based on Japanese Patent Application No. 2022-060960, filed in Japan on March 31, 2022, the contents of which are incorporated herein by reference.

[BACKGROUND ART]

Lipid nanoparticles (LNPs) are utilized as carriers for encapsulating lipid-soluble drugs, or nucleic acids such as short interfering RNA (siRNA) and mRNA, and delivering them to target cells. For instance, a lipid nanoparticle comprising as a constituent lipid a pH-sensitive cationic lipid, which is electrically neutral at physiological pH and turns cationic under weakly acidic pH environment such as in an endosome, is reported as a lipid nanoparticle that can be used as a carrier for efficiently delivering a nucleic acid such as siRNAs to a target cell (Patent Literature 1).

As a pH-sensitive cationic lipid, for example, Jayaraman et al. developed DLin-MC3-DMA (hereinafter, may be abbreviated as "MC3"), which achieved an $ED_{50}$ of 0.005 mg siRNA/kg in knockdown of factor 7 (F7) in mouse liver (Non-Patent Literature 1). The present inventors have also previously developed unique pH-sensitive cationic lipids YSK05 and YSK13-C3, which achieved $ED_{50}$ values of 0.06 and 0.015 mg siRNA/kg, respectively, in F7 knockdown (Non-Patent Literatures 2 to 4). In addition, Maier et al. have developed L319, which is a biodegradability-rendered version of MC3-DMA, and have reported that it achieved both an $ED_{50}$ of 0.01 mg siRNA/kg and high safety (Non-Patent Literatures 5 to 7). However, it has been revealed that the endosomal escape efficiency of lipid nanoparticles containing these pH-sensitive cationic lipids is still only about a few percent (Non-Patent Literature 8), and the development of a technology that can further improve bioavailability is desired.

On the other hand, there is a method for producing lipid nanoparticles encapsulating nucleic acids or the like, which is based on the alcohol dilution method using a flow path. For instance, it has been reported that lipid nanoparticles with a diameter of about 30 nm can be produced with good reproducibility by using a micro-flow path equipped with a three-dimensional micromixer that is capable of achieving instantaneous mixing of two liquids (Non-Patent Literature 9). It has also been reported that by using a flow path structure having a simple two-dimensional structure provided with a micro-sized flow path through which feedstock solutions are to be run and in which baffles (baffle plates) of specified widths in relation to the width of the flow path are alternately disposed from the two side faces, it is possible to form a nano-sized lipid particle formation system with higher particle size controllability than a flow path structure using a conventional three-dimensional mixer (Patent Literature 2). In recent years, these methods for producing nanoparticle formulations have been mainly adopted for producing lipid nanoparticles (LNPs) loaded with lipid-soluble drugs or nucleic acids such as siRNA (short interfering RNA) or mRNA.

[PRIOR ART LITERATURES]

[PATENT LITERATURES]

[Patent Literature 1] WO 2018/230710
[Patent Literature 2] WO 2018/190423

[NON-PATENT LITERATURES]

[Non-Patent Literature 1] Jayaraman et al., Angewandte Chemie International Edition, 2012, vol.51, p.8529-8533.
[Non-Patent Literature 2] Watanabe et al., Scientific Reports, 2014, 4:4750, DOI:10.1038/srep04750.
[Non-Patent Literature 3] Yamamoto et al., Journal of Hepatology, 2016, vol.64, p.547-555.
[Non-Patent Literature 4] Sato et al., Molecular Therapy, 2016, vol.24, p.788-795.
[Non-Patent Literature 5] Maier et al., Molecular Therapy, 2013, vol.21(8), p.1570-1578.
[Non-Patent Literature 6] Wittrup et al., Nature Biotechnology, 2015, vol.33(8), p.870-876.
[Non-Patent Literature 7] Xu et al., Molecular Pharmaceutics, 2014, vol.11, p.1424-1434.
[Non-Patent Literature 8] Gilleron et al., Nature Biotechnology, 2013, vol.31(7), p.638-646.

[Non-Patent Literature 9] Leung et al., Journal of Physical Chemistry C Nanomater Interfaces, 2012, vol.116(34), p.18440-18450.

[SUMMARY OF INVENTION]

[PROBLEMS TO BE SOLVED BY INVENTION]

An object of the present invention is to provide lipid nanoparticles that serve as gene delivery carriers for liver cells and spleen cells.

[MEANS TO SOLVE THE PROBLEMS]

The present inventors have discovered that lipid nanoparticles comprising as a constituent lipid a pH-sensitive cationic lipid having a branched hydrocarbon chain are useful as gene delivery carriers for liver cells and spleen cells, and have completed the present invention.

Accordingly, the present invention is to provide a lipid nanoparticle, etc., as follows.

[1] A lipid nanoparticle comprising a pH-sensitive cationic lipid represented by the formula (I):
[Chem. 1]

$$(R^1)(R^2)C(OH)\text{-}(CH_2)a\text{-}(O\text{-}CO)b\text{-}X \qquad (I)$$

[in the formula (I), a denotes an integer from 3 to 5; b denotes 0 or 1; $R^1$ and $R^2$ each independently denote a group represented by the following general formula (A):
[Chem. 2]

$$(R^{11})\ (R^{12})HC\text{-}(CH_2)c\text{-}(CO\text{-}O)\text{-}(CH_2)e\text{-} \qquad (A)$$

(in the formula (A), $R^{11}$ and $R^{12}$ each independently denote a linear or branched $C_{1-15}$ alkyl group; c denotes an integer from 1 to 7; and e denotes an integer from 4 to 12; provided that $R^{11}$ and $R^{12}$ may be joined to each other to form a ring);
X denotes a group represented by the following general formula (B):
[Chem. 3]

$$\text{-}(CH_2)d\text{-}N(R^3)(R^4) \qquad (B)$$

(in formula (B), d denotes an integer from 0 to 3; $R^3$ and $R^4$ each independently denote a $C_{1-4}$ alkyl group or a $C_{2-4}$ alkenyl group (in the $C_{1-4}$ alkyl group or the $C_{2-4}$ alkenyl group, one or two hydrogen atoms may be substituted by a phenyl group), or $R^3$ and $R^4$ may be joined to each other to form a 5- to 7-membered non-aromatic heterocycle (in the heterocycle, one or two hydrogen atoms may be substituted by a $C_{1-4}$ alkyl group or $C_{2-4}$ alkenyl group)), or a 5- to 7-membered non-aromatic heterocyclic group (provided that the group is bound to (O-CO)b- via a carbon atom, and one or two hydrogen atoms of the ring may be substituted by a $C_{1-4}$ alkyl group or a $C_{2-4}$ alkenyl group).

[2] The lipid nanoparticle according to [1], wherein c is an integer from 4 to 7.
[3] The lipid nanoparticle according to [1] or [2], wherein the sum of the number of carbon atoms of the group having a larger carbon number out of $R^{11}$ and $R^{12}$ plus c is 5 to 17.
[4] The lipid nanoparticle according to any one of [1] to [3], further comprising a sterol and a polyalkylene glycol-modified lipid.
[5] The lipid nanoparticle according to any one of [1] to [4], comprising a nucleic acid.
[6] The lipid nanoparticle according to [5], wherein the nucleic acid is an siRNA, mRNA, or plasmid DNA.
[7] The lipid nanoparticle according to any one of [1] to [5], comprising a DNA nuclease, a guide RNA, and a single-stranded oligonucleotide comprising a region capable of paring with a part of the guide RNA.
[8] The lipid nanoparticle according to [7], wherein 30 to 60% of the bases in the region of the single-stranded oligonucleotide that can pair with a part of the guide RNA are complementary to the bases in the guide RNA.
[9] The lipid nanoparticle according to [7] or [8], wherein the DNA nuclease is a Cas9 protein, and the guide RNA consists of crRNA and tracrRNA.
[10] A lipid nanoparticle comprising a lipid ingredient, a DNA nuclease, a guide RNA, and a single-stranded

oligonucleotide comprising a region capable of paring with a part of the guide RNA, wherein 30 to 60% of the bases in the region of the single-stranded oligonucleotide that can pair with a part of the guide RNA are complementary to the bases in the guide RNA.

[11] A pharmaceutical composition, wherein the lipid nanoparticle according to any one of [1] to [10] is an active ingredient.

[12] The pharmaceutical composition according to [11] for use in gene therapy.

[13] A method for expressing an exogenous gene, comprising administering the lipid nanoparticles according to any one of [1] to [10] in which an exogenous gene of interest to be expressed in liver cells or spleen cells is encapsulated to a subject animal (excluding human) and expressing the exogenous gene in the liver or spleen of the subject animal.

[14] A method for genome editing, comprising introducing the lipid nanoparticle according to any one of [7] to [10] into a cell.

[15] A pH-sensitive cationic lipid represented by the above formula (I).

[16] The pH-sensitive cationic lipid according to [15], that is

7-(4-(dipropylamino)butyl)-7-hydroxy-13-((3-propylhexanoyl)oxy)tridecyl 3-ethylheptanoic acid,
7-(4-(dipropylamino)butyl)-7-hydroxytridecane-1,13-diylbis(3-butylheptanoate),
7-(4-(dipropylamino)butyl)-7-hydroxytridecane-1,13-diylbis(3-pentyloctanoate),
7-(4-(dipropylamino)butyl)-7-hydroxytridecane-1,13-diylbis(3-hexylnonanoate),
7-(4-(dipropylamino)butyl)-7-hydroxytridecane-1,13-diylbis(3-heptyldecanoate),
7-(4-(dipropylamino)butyl)-7-hydroxytridecane-1,13-diylbis(3-octylundecanoate),
7-(4-(dipropylamino)butyl)-7-hydroxytridecane-1,13-diylbis(2-cycloheptyl acetate),
7-(4-(dipropylamino)butyl)-7-hydroxytridecane-1,13-diylbis(2-cyclododecyl acetate),
7-(4-(dipropylamino)butyl)-7-hydroxytridecane-1,13-diylbis(2-cyclopentadecyl acetate),
7-(4-(dipropylamino)butyl)-7-hydroxytridecane-1,13-diylbis(6-ethyldecanoate),
7-(4-(dipropylamino)butyl)-7-hydroxytridecane-1,13-diylbis(6-propylundecanoate),
7-(4-(dipropylamino)butyl)-7-hydroxytridecane-1,13-diylbis(6-butyldodecanoate),
7-(4-(dipropylamino)butyl)-7-hydroxytridecane-1,13-diylbis(6-hexyldodecanoate),
7-(4-(dipropylamino)butyl)-7-hydroxytridecane-1,13-diylbis(7-ethylundecanoate),
7-(4-(dipropylamino)butyl)-7-hydroxytridecane-1,13-diylbis(8-ethyldodecanoate),
7-(4-(dipropylamino)butyl)-7-hydroxytridecane-1,13-diylbis(8,10,10-trimethylundecanoate),
7-(4-(dipropylamino)butyl)-7-hydroxytridecane-1,13-diylbis(7,9,9-trimethyldecanoate),
7-(4-(dipropylamino)butyl)-7-hydroxytridecane-1,13-diylbis(9,11,11-trimethyldodecanoate),
7-(4-(dipropylamino)butyl)-7-hydroxytridecane-1,13-diylbis(7,11-dimethyldodecanoate), or
7-(4-(dipropylamino)butyl)-7-hydroxytridecane-1,13-diylbis(8,12-dimethyltridecanoate).

[ADVANTAGEOUS EFFECTS OF INVENTION]

The lipid nanoparticles according to the present invention is capable of high-level expression of encapsulated genes in liver cells and spleen cells. Therefore, the lipid nanoparticles are useful as gene delivery carriers which can be used in gene therapy.

[BRIEF DESCRIPTION OF DRAWINGS]

[FIG. 1] FIG. 1 is a diagram showing the results of measuring Flue activity (RLU/mg protein) in the liver and spleen of mice administered with each Flue mRNA-loaded lipid nanoparticle in Example 1.

[FIG. 2] FIG. 2 is a diagram showing the results of measuring Fluc activity (RLU/mg protein) in the liver and spleen of mice administered with each Flue mRNA-loaded lipid nanoparticle in Example 1.

[FIG. 3] FIG. 3 is a diagram showing the results of measuring Fluc activity (RLU/mg protein) in the liver and spleen of mice administered with each Flue mRNA-loaded lipid nanoparticle in Example 2.

[FIG. 4] FIG. 4 is a diagram showing the results of measuring the amount of lipids in the liver of mice administered with each Flue mRNA-loaded lipid nanoparticle in Example 3 (percentage to the total amount administered per gram of liver; %/g liver).

[FIG. 5] FIG. 5 is a diagram showing the results of measuring TTR activity (μg/mL) in mouse serum 7 days after administration of TTR-RNP-ssODN-loaded lipid nanoparticles in Example 4.

[FIG. 6] FIG. 6 is a diagram showing the results of measuring TTR activity (μg/mL) in mouse serum 7 days after administration of TTR-RNP-ssODN-loaded lipid nanoparticles in Example 4.

[FIG. 7] FIG. 7 is a diagram showing the results of measuring the in vitro DNA cleavage activity of TTR-RNP-ssODN-loaded lipid nanoparticles in Example 6.

[FIG. 8] FIG. 8 is a diagram showing the results of measuring the amount of TTR protein (μg/mL) in mouse serum 7 days after administration of TTR-RNP-ssODN-loaded lipid nanoparticles in Example 6.

[FIG. 9] FIG. 9 is a diagram showing the results of measuring Flue activity (RLU/mg protein) in each tissue of mice administered with each Fluc mRNA-loaded lipid nanoparticle in Example 7.

[FIG. 10] FIG. 10 is a diagram showing the results of measuring Fluc activity (RLU/mg protein) in each tissue of mice administered with each Flue mRNA-loaded lipid nanoparticle in Example 7.

[FIG. 11] FIG. 11 is a diagram showing the results of measuring Fluc activity (RLU/mg protein) in each tissue of mice administered with a frozen sample or a non-frozen sample in Example 7.

[FIG. 12] FIG. 12 is a diagram showing the results of measuring the amount of TTR protein (μg/mL) in mouse serum 7 days after administration of TTR-RNP-ssODN-loaded lipid nanoparticles in Example 8.

[FIG. 13] FIG. 13 is a diagram showing the results of measuring the amount of TTR protein (μg/mL) in mouse serum 7 days after administration of lipid nanoparticles loaded with TTR-RNP-ssODN having different PEG-DMG content ratios and [RNP weight]/[total lipid weight] weight ratios in Example 8.

[FIG. 14] FIG. 14 is a diagram showing the results of measuring the amount of TTR protein (μg/mL) in mouse serum 7 days after administration of lipid nanoparticles loaded with TTR-RNP-ssODN having different CL4F11-ζ-2 and DSPC content ratios in Example 8.

[FIG. 15] FIG. 15 is a diagram showing the results of measuring the amount of TTR protein (μg/mL) in mouse serum 7 days after administration of lipid nanoparticles loaded with TTR-RNP-ssODN containing CL4F11-ζ-2 in Example 8 (FIG. 15(A)), and the gene knockout efficiency (%) calculated from the results (FIG. 15(B)).

[FIG. 16] FIG. 16 is a diagram showing the results of measuring the amount of TTR protein (μg/mL) in the serum of mice 1, 4, or 12 weeks after administration of TTR-RNP-ssODN-loaded lipid nanoparticles containing CL4F11-ζ-2 or GFP-RNP-ssODN-loaded lipid nanoparticles containing CL4F11-ζ-2 in Example 8.

[FIG. 17] FIG. 17 is a diagram showing the results of measuring the amount of TTR protein (μg/mL) in mouse serum 7 days after administration of lipid nanoparticles loaded with TTR-RNP-ssODN containing CL4F11-ζ-2, SM, ALC, or MC3 in Example 8.

[FIG. 18] FIG. 18 is a diagram showing the results of measuring Fluc activity (RLU/mg protein) in the liver or spleen of mice administered with Fluc mRNA-loaded lipid nanoparticles containing CL4F11-ζ-2, SM, ALC, or MC3 in Example 8.

[FIG. 19]FIG. 19 is a diagram showing the results of measuring the amount of TTR protein (μg/mL) in mouse serum 7 days after administration of frozen or non-frozen samples of TTR-RNP-ssODN-loaded lipid nanoparticles containing CL4F11-ζ-2, CL4F11-e-3, or CL4F12-e-4 in Example 8.

[FIG. 20] FIG. 20 is a diagram showing the results of measuring the DiR fluorescence intensity in each tissue of mice 1 hour after administration of DiR-labeled TTR-RNP-ssODN-loaded lipid nanoparticles or DiR-labeled siTTR-loaded lipid nanoparticles diluted to have the same DiR fluorescence intensity in Example 8.

[FIG. 21] FIG. 21 is a diagram showing a plot of fluorescence intensity of the reaction solutions containing sgTTR-G211-5FAM and ssODN-80%-3IBFQ, ssODN-40%-3IBFQ or ssODN-0%-3IBFQ at different temperatures from 11 to 70 °C in Example 9 (FIG. 21(A)), and the amount of change in fluorescence intensity at each temperature (FIG. 21(B)).

[FIG. 22] FIG. 22 is a diagram showing the results of in vitro measurement of DNA cleavage activity at 37 °C (FIG. 22(A)) or 15 °C (FIG. 22(B)) of RNPs formed using sgTTR-G211 and ssODN-TTR_3-80%, ssODN-TTR_3-40% or ssODN-TTR_3-0% in Example 9.

[FIG. 23] FIG. 23 is a diagram showing the results of measuring the amount of TTR protein (μg/mL) in mouse serum 7 days after administration of TTR-RNP-ssODN-loaded lipid nanoparticles prepared at 4 °C in Example 9.

[DESCRIPTION OF EMBODIMENTS]

Hereinbelow, the embodiments of the present invention are to be explained in specific. Herein, "from X1 to X2 (X1 and X2 are real numbers that satisfy X1 < X2)" means "equal to or greater than X1 and equal to or less than X2".

[pH-Sensitive Cationic Lipids]

The lipid nanoparticles according to the present invention are lipid nanoparticles containing a pH-sensitive cationic lipid represented by the following general formula (I) (hereinbelow may be referred to as the "pH-sensitive cationic lipid of the present invention"). By comprising a pH-sensitive cationic lipid represented by general formula (I) as a constituent lipid of the lipid nanoparticle, the lipid nanoparticle of the present invention has good introduction efficiency and expression efficiency in liver cells and spleen cells, and is useful as a gene carrier.

[Chem. 4]

$(R^1)(R^2)C(OH)-(CH_2)a-(O-CO)b-X$ (I)

In the general formula (I), a denotes an integer from 3 to 5, though it is preferably 4.

b denotes 0 or 1. When b is 0, the -O-CO-group is absent, meaning that it is a single bond.

In the general formula (I), $R^1$ and $R^2$ each independently denote a group expressed by the following general formula (A). In the generic formula (A), $R^{11}$ and $R^{12}$ each independently denote a linear or branched $C_{1-15}$ alkyl group (an alkyl group having 1 to 15 carbon atoms).

[Chem. 5]

$(R^{11})(R^{12})HC-(CH_2)c-(CO-O)-(CH_2)e-$ (A)

The linear or branched $C_{1-15}$ alkyl group includes:

a methyl group, an ethyl group, a propyl group, an isopropyl group, an n-butyl group, an isobutyl group, and a tert-butyl group.The $C_{2-4}$ alkenyl group includes a vinyl group, 1-propenyl group, 2-propenyl group, 1-methylvinyl group, 2-methyl-1-propenyl group, 1-butenyl group, 2-butenyl group, and 3-butenyl group;

n-pentyl group, 1-methylbutyl group, 2-methylbutyl group, 3-methylbutyl group, 1-ethylpropyl group, 1,1-dimethylpropyl group, 2,2-dimethylpropyl group;

n-hexyl group, 1-methylpentyl group, 2-methylpentyl group, 3-methylpentyl group, 4-methylpentyl group, 1-ethylbutyl group, 1,1-dimethylbutyl group, 2,2-dimethylbutyl group, 3,3-dimethylbutyl group, 1,2-dimethylbutyl group, 1-methyl-2,2-dimethylbutyl group;

n-heptyl group, 1-methylhexyl group, 2-methylhexyl group, 3-methylhexyl group, 4-methylhexyl group, 5-methylhexyl group, 1-ethylpentyl group, 1,1-dimethylpentyl group, 2,2-dimethylpentyl group, 3,3-dimethylpentyl group, 4,4-dimethylpentyl group, 1-methyl-3,3-dimethylbutyl group, 2-methyl-3,3-dimethylbutyl group;

n-octyl group, 1-methylheptyl group, 2-methylheptyl group, 3-methylheptyl group, 4-methylheptyl group, 5-methylheptyl group, 6-methylheptyl group, 1-ethylhexyl group, 1,1-dimethylhexyl group, 2,2-dimethylhexyl group, 3,3-dimethylhexyl group, 4,4-dimethylhexyl group, 5,5-dimethylhexyl group, 1-methyl-4,4-dimethylpentyl group, 2-methyl-4,4-dimethylpentyl group, 3-methyl-4,4-dimethylpentyl group;

n-nonyl group, 1-methyloctyl group, 2-methyloctyl group, 3-methyloctyl group, 4-methyloctyl group, 5-methyloctyl group, 6-methyloctyl group, 7-methyloctyl group, 1-ethylheptyl group, 1,1-dimethylheptyl group, 2,2-dimethylheptyl group, 3,3-dimethylheptyl group, 4,4-dimethylheptyl group, 5,5-dimethylheptyl group, 6,6-dimethylheptyl group, 1-methyl-5,5-dimethylhexyl group, 2-methyl-5,5-dimethylhexyl group, 3-methyl-5,5-dimethylhexyl group, 4-methyl-5,5-dimethylhexyl group;

n-decyl group, 1-methylnonyl group, 2-methylnonyl group, 3-methylnonyl group, 4-methylnonyl group, 5-methylnonyl group, 6-methylnonyl group, 7-methylnonyl group, 8-methylnonyl group, 1-ethyloctyl group, 1,1-dimethyloctyl group, 2,2-dimethyloctyl group, 3,3-dimethyloctyl group, 4,4-dimethyloctyl group, 5,5-dimethyloctyl group, 6,6-dimethyloctyl group, 7,7-dimethyloctyl group, 1-methyl-6,6-dimethylheptyl group, 2-methyl-6,6-dimethylheptyl group, 3-methyl-6,6-dimethylheptyl group, 4-methyl-6,6-dimethylheptyl group, 5-methyl-6,6-dimethylheptyl group;

n-undecyl group, 1-methyldecyl group, 2-methyldecyl group, 3-methyldecyl group, 4-methyldecyl group, 5-methyldecyl group, 6-methyldecyl group, 7-methyldecyl group, 8-methyldecyl group, 9-methyldecyl group, 1-ethylnonyl group, 1,1-dimethylnonyl group, 2,2-dimethylnonyl group, 3,3-dimethylnonyl group, 4,4-dimethylnonyl group, 5,5-dimethylnonyl group, 6,6-dimethylnonyl group, 7,7-dimethylnonyl group, 8,8-dimethylnonyl group, 1-methyl-7,7-dimethyloctyl group, 2-methyl-7,7-dimethyloctyl group, 3-methyl-7,7-dimethyloctyl group, 4-methyl-7,7-dimethyloctyl group, 5-methyl-7,7-dimethyloctyl group, 6-methyl-7,7-dimethyloctyl group;

n-dodecyl group, 1-methylundecyl group, 2-methylundecyl group, 3-methylundecyl group, 4-methylundecyl group, 5-methylundecyl group, 6-methylundecyl group, 7-methylundecyl group, 8-methylundecyl group, 9-methylundecyl group, 10-methylundecyl group, 1-ethyldecyl group, 1,1-dimethyldecyl group, 2,2-dimethyldecyl group, 3,3-dimethyldecyl group, 4,4-dimethyldecyl group, 5,5 -dimethyldecyl group, 6,6-dimethyldecyl group, 7,7-dimethyldecyl group, 8,8-dimethyldecyl group, 9,9-dimethyldecyl group, 1-methyl-8,8-dimethylnonyl group, 2-methyl-8,8-dimethylnonyl group, 3-methyl-8,8-dimethylnonyl group, 4-methyl-8,8-dimethylnonyl group, 5-methyl-8,8-dimethylnonyl group, 6-methyl-8,8-dimethylnonyl group, 7-methyl-8,8-dimethylnonyl group;

n-tridecyl group, 1-methyldodecyl group, 2-methyldodecyl group, 3-methyldodecyl group, 4-methyldodecyl group, 5-methyldodecyl group, 6-methyldodecyl group, 7-methyldodecyl group, 8-methyldodecyl group, 9-methyldodecyl group, 10-methyldodecyl group, 11-methyldodecyl group, 1-ethylundecyl group, 1,1-dimethylundecyl group, 2,2-dimethylundecyl group, 3,3-dimethylundecyl group, 4,4-dimethylundecyl group, 5,5-dimethylundecyl group, 6,6-dimethylundecyl group, 7,7-dimethylundecyl group, 8,8-dimethylundecyl group, 9,9-dimethylundecyl group, 10,10-dimethylundecyl group, 1-methyl-9,9-dimethyldecyl group, 2-methyl-9,9-dimethyldecyl group, 3-methyl-9,9-dimethyldecyl group, 4-methyl-9,9-dimethyldecyl group, 5-methyl-9,9-dimethyldecyl group, 6-methyl-9,9-dimethyldecyl group, 7-methyl-9,9-dimethyldecyl group, 8-methyl-9,9-dimethyldecyl group;

n-tetradecyl group, 1-methyltridecyl group, 2-methyltridecyl group, 3-methyltridecyl group, 4-methyltridecyl group, 5-methyltridecyl group, 6-methyltridecyl group, 7-methyltridecyl group, 8-methyltridecyl group, 9-methyltridecyl group, 10-methyltridecyl group, 11-methyltridecyl group, 12-methyltridecyl group, 1-ethyldodecyl group, 1,1-dimethyldodecyl group, 2,2-dimethyldodecyl group, 3,3-dimethyldodecyl group, 4,4-dimethyldodecyl group, 5,5-dimethyldodecyl group, 6,6-dimethyldodecyl group, 7,7-dimethyldodecyl group, 8,8-dimethyldodecyl group, 9,9-dimethyldodecyl group, 10,10-dimethyldodecyl group, 11,11-dimethyldodecyl group, 1-methyl-10,10-dimethylundecyl group, 2-methyl-10,10-dimethylundecyl group, 3-methyl-10,10-dimethylundecyl group, 4-methyl-10,10-dimethylundecyl group, 5-methyl-10,10-dimethylundecyl group, 6-methyl-10,10-dimethylundecyl group, 7-methyl-10,10-dimethylundecyl group, 8-methyl-10,10-dimethylundecyl group, 9-methyl-10,10-dimethylundecyl group;

n-pentadecyl group, 1-methyltetradecyl group, 2-methyltetradecyl group, 3-methyltetradecyl group, 4-methyltetradecyl group, 5-methyltetradecyl group, 6-methyltetradecyl group, 7-methyltetradecyl group, 8-methyltetradecyl group, 9-methyltetradecyl group, 10-methyltetradecyl group, 11-methyltetradecyl group, 12-methyltetradecyl group, 13-methyltetradecyl group, 1-ethyltridecyl group, 1,1-dimethyltridecyl group, 2,2-dimethyltridecyl group, 3,3-dimethyltridecyl group, 4,4-dimethyltridecyl group, 5,5-dimethyltridecyl group, 6,6-dimethyltridecyl group, 7,7-dimethyltridecyl group, 8,8-dimethyltridecyl group, 9,9-dimethyltridecyl group, 10,10-dimethyltridecyl group, 11,11-dimethyltridecyl group, 12,12-dimethyltridecyl group, 1-methyl-11,11-dimethyldodecyl group, 2-methyl-11,11-dimethyldodecyl group, 3-methyl-11,11-dimethyldodecyl group, 4-methyl-11,11-dimethyldodecyl group, 5-methyl-11,11-dimethyldodecyl group, 6-methyl-11,11-dimethyldodecyl group, 7-methyl-11,11 -dimethyldodecyl group, 8-methyl-11,11-dimethyldodecyl group, 9-methyl-11,11-dimethyldodecyl group, and 10-methyl-11,11-dimethyldodecyl group.

In the general formula (A), $R^{11}$ and $R^{12}$ are each independently preferably a linear or branched $C_{1-12}$ alkyl group (an alkyl group having 1 to 12 carbon atoms), more preferably a linear or branched $C_{1-10}$ alkyl group (an alkyl group having 1 to 10 carbon atoms), and further preferably a linear or branched $C_{1-8}$ alkyl group (an alkyl group having 1 to 8 carbon atoms). In the pH-sensitive cationic lipid of the present invention, $R^1$ and $R^2$ may be the same group or different groups to each other as long as they are groups represented by general formula (A).

In the general formula (A), the group having a larger carbon number out of $R^{11}$ and $R^{12}$ is the main chain, the number of carbon atoms of the group having a larger carbon number out of $R^{11}$ and $R^{12}$ is $Nc_L$, and the number of carbon atoms of the group having a smaller carbon number out of the two is $Nc_S$ . When $R^{11}$ and $R^{12}$ have the same number of carbon atoms, $Nc_L$ and $Nc_S$ have the same value. In the general formula (A), the sum of $Nc_L$+ c is preferably 5 to 17, more preferably 5 to 12, and even more preferably 6 to 11, since higher delivery efficiency can be achieved. The sum of $Nc_L$+ c is preferably 10 to 15, and more preferably 13-15. From the viewpoint of delivery efficiency, Ncs is preferably 1 to 8, more preferably 1 to 5, and even more preferably 1 to 3. Moreover, $Nc_L$ is preferably 4 to 8, and more preferably 4 to 6. From the standpoint of delivery efficiency, the sum of $Nc_L$+ $Nc_S$ + c is preferably 10 to 17, more preferably 10 to 15, and even more preferably 11 to 13.

In the general formula (A), $R^{11}$ and $R^{12}$ may be linked to each other to form a ring. The ring includes, for example, a cycloalkane having 5 to 15 carbon atoms. Specific examples include cyclopentane, cyclohexane, cycloheptane, cyclooctane, cyclononane, cyclodecane, cycloundecane, cyclododecane, cyclotridecane, cyclotetradecane, and cyclopentadecane. These cycloalkanes may have an alkyl group substituent.

In the general formula (A), c denotes an integer from 1 to 7. From the viewpoint of improving biodegradability, in the pH-sensitive cationic lipid of the present invention, it is preferred that c is an integer from 4 or more, i.e., that the chain of the general formula (A) is branched at a carbon atom at the ε-position or farther.

In the general formula (A), e denotes an integer from 4 to 12. In the pH-sensitive cationic lipid of the present invention, e is preferably an integer from 4 to 10, and more preferably an integer from 6 to 10.

In the general formula (I), X denotes a group represented by the following general formula (B) or a 5- to 7-membered non-aromatic heterocyclic group. The 5- to 7-membered non-aromatic heterocyclic group denoted by X is bound to (O-CO)b- via a carbon atom.

[Chem. 6]

$$-(CH_2)d\text{-}N(R^3)(R^4) \qquad (B)$$

In the general formula (B), d denotes an integer from 0 to 3. When d is 0, $-(CH_2)-$ group is absent, meaning that it is a single bond.

In the general formula (B), $R^3$ and $R^4$ each independently denote a $C_{1-4}$ alkyl group (an alkyl group having from 1 to 4 carbon atoms) or a $C_{2-4}$ alkenyl group (an alkenyl group having from 1 to 4 carbon atoms). In the $C_{1-4}$ alkyl group or $C_{2-4}$ alkenyl group denoted by $R^3$ and $R^4$, one or two hydrogen atom(s) may be substituted by (a) phenyl group(s). $R^3$ and $R^4$

may be the same or different groups to each other as long as they are (a) $C_{1-4}$ alkyl group(s) or (a) $C_{2-4}$ alkenyl group(s).

The $C_{1-4}$ alkyl group includes a methyl group, ethyl group, propyl group, isopropyl group, n-butyl group, isobutyl group, and tert-butyl group. The $C_{2-4}$ alkenyl group includes a vinyl group, 1-propenyl group, 2-propenyl group, 1-methylvinyl group, 2-methyl-1-propenyl group, 1-butenyl group, 2-butenyl group, and 3-butenyl group.

In the general formula (B), $R^3$ and $R^4$ may be bound to each other to form a 5- to 7-membered non-aromatic heterocycle. The 5- to 7-membered non-aromatic heterocycle formed by $R^3$ and $R^4$ bound to each other includes, for example, a 1-pyrrolidinyl group, 1-piperidinyl group, 1-morpholinyl group, and 1-piperazinyl group. In the 5- to 7-membered non-aromatic heterocycle formed by $R^3$ and $R^4$ bound to each other, one or two hydrogen atom(s) in the ring may be substituted by (a) $C_{1-4}$ alkyl group(s) or (a) $C_{2-4}$ alkenyl group(s). When two hydrogen atoms in the ring have been substituted by (a) $C_{1-4}$ alkyl group(s) or (a) $C_{2-4}$ alkenyl group(s), they may be substituted by the same groups, or they may be substituted by different groups to each other.

In the general formula (I), when X is a 5- to 7-membered non-aromatic heterocyclic group, the heteroatom contained in the heterocyclic group includes a nitrogen atom, oxygen atom or sulfur atom, etc. The heterocycle in the heterocyclic group may comprise as (a) constituent(s) one heteroatom, or two or more heteroatoms, which are the same or different. The heterocycle in the heterocyclic group may be a saturated heterocycle and may comprise one or two or more double bond(s), provided that the heterocycle is never an aromatic ring.

The pH-sensitive cationic lipid of the present invention is preferably a compound in which $R^1$ and $R^2$ in the general formula (I) are each independently a group of the general formula (A) in which $R^{11}$ and $R^{12}$ are each independently a linear or branched $C_{1-12}$ alkyl group, c is an integer from 1 to 7, and e is an integer from 6 to 10, and a is an integer from 3 to 5, b is 1, and X is a 5- to 7-membered non-aromatic heterocyclic group (provided that it is bound to (O-CO)b- via a carbon atom in the heterocyclic group), preferably 1-pyrrolidinyl group, 1-piperidinyl group, 1-morpholinyl group, or 1 -piperazinyl group (which is bound to (O-CO)b- via a carbon atom in the ring, and one hydrogen atom may be substituted with a $C_{1-4}$ alkyl group or a $C_{2-4}$ alkenyl group), or a compound in which $R^1$ and $R^2$ in the general formula (I) are each independently a group of the general formula (A) in which $R^{11}$ and $R^{12}$ are each independently a linear or branched $C_{1-12}$ alkyl group, c is an integer from 1 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group or a $C_{2-4}$ alkenyl group (the $C_{1-4}$ alkyl group or the $C_{2-4}$ alkenyl group indicated by $R^3$ and $R^4$ may have one or two hydrogen atom(s) substituted by (a) phenyl group(s)). In particular, the pH-sensitive cationic lipid of the present invention is preferably a compound in which $R^1$ and $R^2$ in the general formula (I) are each independently a group of the general formula (A) in which $R^{11}$ and $R^{12}$ are each independently a linear or branched $C_{1-8}$ alkyl group, c is an integer from 1 to 7, and e is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group), or a compound in which $R^1$ and $R^2$ in the general formula (I) are each independently a group of the general formula (A) in which $R^{11}$ and $R^{12}$ are each independently a linear or branched $C_{1-8}$ alkyl group, c is an integer from 1 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group.

The pH-sensitive cationic lipid of the present invention is, more preferably, a compound in which $R^1$ and $R^2$ in the general formula (I) are each independently a group of the general formula (A) in which $R^{11}$ and $R^{12}$ are each independently a linear or branched $C_{1-8}$ alkyl group, c is an integer from 4 to 7, and e is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (I) are each independently a group of the general formula (A) in which $R^{11}$ and $R^{12}$ are linked to each other to form a cycloalkane having 5 to 15 carbon atoms, c is an integer from 4 to 7, and e is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (I) are each independently a group of the general formula (A) in which $R^{11}$ and $R^{12}$ are each independently a $C_{1-8}$ alkyl group, c is an integer from 4 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group; a compound in which $R^1$ and $R^2$ in the general formula (I) are each independently a group of the general formula (A) in which $R^{11}$ and $R^{12}$ are linked to each other to form a cycloalkane having 5 to 15 carbon atoms, c is an integer from 4 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group.

Particularly preferred pH-sensitive cationic lipid of the present invention is a compound in which $R^1$ and $R^2$ in the general formula (I) are the same group, and which is a group of the general formula (A) in which $R^{11}$ and $R^{12}$ are each independently a linear or branched $C_{1-8}$ alkyl group, c is an integer from 4 to 7, and e is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- by a carbon atom in the ring, and one hydrogen atom may be substituted with a $C_{1-4}$ alkyl group

or a $C_{2-4}$ alkenyl group); a compound in which $R^1$ and $R^2$ in the general formula (I) are the same group, and which is a group of the general formula (A) in which $R^{11}$ and $R^{12}$ are linked to each other to form a cycloalkane having 5 to 15 carbon atoms, c is an integer from 4 to 7, and e is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (I) are the same group, and which is a group of the general formula (A) in which $R^{11}$ and $R^{12}$ are each independently a linear or branched $C_{1-8}$ alkyl group, c is an integer from 4 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group; a compound in which $R^1$ and $R^2$ in the general formula (I) are the same group, and which is a group of the general formula (A) in which $R^{11}$ and $R^{12}$ are linked to each other to form a cycloalkane having 5 to 15 carbon atoms, c is an integer from 4 to 7, and e is an integer from 6 to 10, and a is an integer from 3 to 5, b is 0, and X is a group of the general formula (B) in which d is 0, and $R^3$ and $R^4$ are each independently a $C_{1-4}$ alkyl group.

As the pH-sensitive cationic lipid of the present invention, in particularly, a compound in which $R^1$ and $R^2$ in the general formula (I) are the same group, and which is a group of the general formula (A) in which $R^{11}$ and $R^{12}$ are each independently a linear or branched $C_{1-8}$ alkyl group, c is an integer from 4 to 7, the sum of $Nc_L$+ c is from 8 to 12, $Nc_S$ is from 1 to 6, and e is an integer from 6 to 10, and a is an integer from 3 to 5, b is 0, and X is a group of the general formula (B) in which d is 0, and $R^3$ and $R^4$ are each independently a $C_{1-4}$ alkyl group is preferred; a compound in which $R^1$ and $R^2$ in the general formula (I) are the same group, and which is a group of the general formula (A) in which $R^{11}$ and $R^{12}$ are each independently a linear or branched $C_{1-8}$ alkyl group, c is an integer from 4 to 7, the sum of $Nc_L$+ c is from 9 to 11, $Nc_S$ is from 1 to 3, and e is an integer from 6 to 10, and a is an integer from 3 to 5, b is 0, and X is a group of the general formula (B) in which d is 0, and $R^3$ and $R^4$ are each independently a $C_{1-4}$ alkyl group is more preferred; anda compound in which $R^1$ and $R^2$ in the general formula (I) are the same group, and which is a group of the general formula (A) in which $R^{11}$ and $R^{12}$ are each independently a linear or branched $C_{1-8}$ alkyl group, c is an integer from 4 to 6, the sum of $Nc_L$+ c is from 9 to 11, $Nc_S$ is from 1 to 3, and e is an integer from 6 to 10, and a is an integer from 3 to 5, b is 0, and X is a group of the general formula (B) in which d is 0, and $R^3$ and $R^4$ are each independently a $C_{1-4}$ alkyl group is further preferred.

The pKa of the pH-sensitive cationic lipid represented by the general formula (I) is not particularly limited, though it can be selected, for example, from about 4.0 to 9.0, preferably from about 4.5 to 8.5, and it is preferable to select the type of each substituent so as to give a pKa in this range.

The pH-sensitive cationic lipid represented by the general formula (I) can easily be produced, for example, by a method specifically shown in Examples herein. A skilled artisan can easily produce any lipid encompassed within the scope of the general formula (I) by referring to this method for production and appropriately selecting ingredient compounds, reagents and reaction conditions, etc.

The group represented by the general formula (A) is a group that is provided with a branched structure in which two hydrocarbon chains ($R^{11}$ and $R^{12}$ ) are linked to a -CO-O- group. That is, the pH-sensitive cationic lipid of the present invention is provided with two branched hydrocarbon chains ($R^1$ and $R^2$), and these hydrocarbon chains form a hydrophobic scaffold that is to be embedded in the lipid membrane of the lipid nanoparticle. The lipid nanoparticle according to the present invention is characterized in that it has high liver selectivity by comprising as a lipid component the pH-sensitive cationic lipid of the present invention that is provided with a hydrophobic scaffold consisting of a branched chain structure.

[Lipid Nanoparticles]

The lipid nanoparticles according to the present invention have as their constituent lipid the pH-sensitive cationic lipid of the present invention, i.e., they have a lipid component in which two hydrocarbon chains ($R^{11}$ and $R^{12}$) are linked to a carbon atom at the β-position relative to the -CO-O- group or farther from this. Therefore, the lipid nanoparticles according to the present invention also have high selectivity for the spleen.

The lipid nanoparticle according to the present invention can comprise only one, or two or more types of pH-sensitive cationic lipid(s) of the present invention as (a) constituent(s). When the lipid nanoparticles according to the present invention comprise two or more types of pH-sensitive cationic lipids of the present invention, the amount of the pH-sensitive cationic lipids of the present invention means the total amount of lipid molecules corresponding to the pH-sensitive cationic lipids of the present invention among the lipid molecules constituting the lipid nanoparticles.

The higher the ratio of the pH-sensitive cationic lipid of the present invention to the lipid molecules constituting the lipid nanoparticles, the higher the efficiency of uptake of the lipid nanoparticles into target cells. For this reason, in the lipid nanoparticles according to the present invention, the ratio of the amount of the pH-sensitive cationic lipid of the present invention to the total amount of lipids constituting the lipid nanoparticles ([amount (mol) of the pH-sensitive cationic lipid of the present invention]/([amount (mol) of the total lipids constituting the lipid nanoparticles])×100%) is preferably 20 mol% or more. On the other hand, if the ratio of pH-sensitive cationic lipids to the lipid molecules constituting the lipid nanoparticles is too high, it may be difficult to sufficiently reduce the particle diameter. Since the lipid nanoparticles that

has a sufficient uptake efficiency into target cells and a sufficiently small particle diameter can be obtained, the ratio of the amount of the pH-sensitive cationic lipid of the present invention to the total amount of lipids constituting the lipid nanoparticles in the lipid nanoparticles of the present invention is more preferably 30 mol % or more, further preferably 30 to 70 mol %, and even more preferably 40 to 60 mol %.

Of the constituent lipids of the lipid nanoparticles according to the present invention, lipids that are generally used when forming liposomes may be used as lipids other than the pH-sensitive cationic lipid of the present invention. Such lipids include, for example, phospholipids, sterols, glycolipids, or saturated or unsaturated fatty acids, etc. These can be used alone or in combination of two or more.

Phospholipids can include glycerophospholipids such as phosphatidyl serine, phosphatidyl inositol, phosphatidyl glycerol, phosphatidyl ethanol amine, phosphatidyl choline, cardiolipin, plasmalogen, ceramide phosphoryl glycerol phosphate, and phosphatidic acid; and sphingophospholipids such as sphingomyelin, ceramide phosphoryl glycerol, and ceramide phosphoryl ethanol amine, etc. Moreover, phospholipids derived from natural ingredients such as egg yolk lecithin and soybean lecithin can also be used. The fatty acid residue in the glycerophospholipid and sphingophospholipid is not particularly limited, though it includes, for example, a saturated or unsaturated fatty acid residue having from 12 to 24 carbon atoms, of which a saturated or unsaturated fatty acid residue having from 14 to 20 carbon atoms is preferred. In specific, it includes an acyl group derived from a fatty acid such as lauric acid, myristic acid, palmitic acid, palmitoleic acid, stearic acid, oleic acid, linoleic acid, linolenic acid, arachidic acid, arachidonic acid, behenic acid and lignoceric acid. When the glycerolipid or sphingolipid has two or more fatty acid residues, the fatty acids residues may all be the same groups, or they may be different groups from each other.

Sterols include, for example, animal-derived sterols such as cholesterol, cholesterol succinic acid, lanosterol, dihydrolanosterol, desmosterol and dihydrocholesterol; plant-derived sterols (phytosterols) such as stigmasterol, sitosterol, campesterol and brassicasterol; and microorganism-derived sterol such as zymosterol and ergosterol, and the like. Glycolipids include, for example, glyceroglycolipids such as sulfoxy ribosylglyceride, diglycosyl diglyceride, digalactosyl diglyceride, galactosyl diglyceride, and glycosyl diglyceride; sphingoglycolipids such as galactosyl cerebroside, lactosyl cerebroside, and ganglioside. Saturated or unsaturated fatty acids include, for example, saturated or unsaturated fatty acids having from 12 to 20 carbon atoms such as palmitic acid, oleic acid, stearic acid, arachidonic acid and myristic acid.

The lipid nanoparticles according to the present invention preferably comprises as a constituent lipid, in addition to the pH-sensitive cationic lipid of the present invention, a neutral lipid, more preferably a phospholipid or a sterol, further preferably a sterol, and even more preferably cholesterol.

The lipid nanoparticles according to the present invention preferably contain a polyalkylene glycol-modified lipid as a lipid component. Polyalkylene glycol is a hydrophilic polymer. By constructing lipid nanoparticles using a polyalkylene glycol-modified lipid as a lipid membrane-constituting lipid, the surface of the lipid nanoparticles can be modified with polyalkylene glycol. Surface modification with polyalkylene glycol may increase the stability of lipid nanoparticles, such as their retention in blood.

The polyalkylene glycol that can be used includes, for example, a polyethylene glycol, polypropylene glycol, polytetramethylene glycol, polyhexamethylene glycol, etc. The molecular weight of the polyalkylene glycol is, for example, approximately from 300 to 10,000, preferably approximately from 500 to 10,000, further preferably approximately from 1,000 to 5,000.

For instance, in order to modify a lipid with a polyethylene glycol, a stearylated polyethylene glycol (e.g., PEG45 stearate (STR-PEG45), etc.) can be used. In addition, polyethylene glycol derivatives can also be used, such as N-[carbonyl-methoxypolyethylene glycol-2000]-1,2-dipalmitoyl-sn-glycero-3-phosphoethanol amine, n-[carbonyl-methoxypolyethylene glycol-5000]-1,2-dipalmitoyl-sn-glycero-3-phosphoethanol amine, N-[carbonyl-methoxypolyethylene glycol-750]-1,2-distearoyl-sn-glycero-3-phosphoethanol amine, N-[carbonyl-methoxypolyethylene glycol-2000]-1,2-distearoyl-sn-glycero-3-phosphoethanol amine, N-[carbonyl-methoxypolyethylene glycol-5000]-1,2-distearoyl-sn-glycero-3-phosphoethanol amine, 1,2-dimyristoyl-rac-glycero-3-methoxypolyethylene glycol-2000 (PEG2k-DMG), though the polyalkylene glycol-modified lipid is not to be limited thereto.

The ratio of the polyalkylene glycol-modified lipid to the total amount of lipid constituting the lipid nanoparticle according to the present invention is not particularly limited, as long as it is an amount that does not impair the liver selectivity of the pH-sensitive cationic lipid of the present invention, specifically, the liver-specific gene expression activity when the lipid nanoparticle of the present invention is used as a gene carrier. For example, the ratio of the polyalkylene glycol-modified lipid to the total amount of lipid constituting the lipid nanoparticle is preferably 0.5 to 3 mol %.

The lipid nanoparticle according to the present invention can undergo an appropriate surface modification, etc., as necessary.

Surface modification of the lipid nanoparticles according to the present invention with a hydrophilic polymer, etc., can increase their retention in blood. The surface modification may also be performed by using a lipid that has been modified with these modifying groups as a lipid constituent for the lipid nanoparticle.

Upon producing the lipid nanoparticle according to the present invention, for example, a glycophorin, ganglioside GM1, phosphatidyl inositol, ganglioside GM3, glucuronic acid derivative, glutamic acid derivative, and polyglycerin

phospholipid derivative, etc., can be utilized as a lipid derivative for increasing their retention in blood. Moreover, dextran, pullulan, Ficoll, polyvinyl alcohols, styrene-maleic anhydride alternating copolymers, divinyl ether-maleic anhydride alternating copolymers, amylose, amylopectin, chitosan, mannan, cyclodextrin, pectin, carrageenan, etc., can also be used for the surface modification, as a hydrophilic polymer for increasing the retention in blood, in addition to the polyalkylene glycol.

Moreover, in order to promote the nuclear translocation of the lipid nanoparticle according to the present invention, for example, the surface of the lipid nanoparticle can be modified with a trisaccharide or a higher oligosaccharide compound. The type of the trisaccharide or a higher oligosaccharide compound is not particularly limited, however, for example, an oligosaccharide compound in which approximately from 3 to 10 sugar units are bound can be used, and an oligosaccharide compound in which approximately from 3 to 6 sugar units are bound can preferably be used. In particular, an oligosaccharide compound which is from a trimer to a hexamer of glucoses can preferably used, and an oligosaccharide compound which is a trimer or tetramer of glucoses can further preferably be used. More specifically, isomaltotriose, isopanose, maltotriose, maltotetraose, maltopentaose or maltohexaose, etc., can suitably be used, of which further preferred are maltotriose, maltotetraose, maltopentaose or maltohexaose to which glucoses are bound via $\alpha$1-4 linkage. Particularly preferred is maltotriose or maltotetraose, and the most preferred is maltotriose. The level of the surface modification of the lipid nanoparticle with the oligosaccharide compound is not particularly limited, though it is, for example, approximately from 1 to 30 mol %, preferably approximately from 2 to 20 mol %, and more preferably approximately from 5 to 10 mol % in relation to the total lipid amount.

A method for modifying the surface of the lipid nanoparticle with an oligosaccharide compound is not particularly limited. For instance, a liposome lipid nanoparticle whose surface has been modified with a monosaccharide such as galactose or mannose has been known (WO 2007/102481), and the method of surface modification described in this publication can be employed. The entire disclosure of the publication described above is incorporated herein by reference as the disclosure of the specification of the present application.

Moreover, the lipid nanoparticle according to the present invention can be conferred any one or more function(s) such as, for example, a temperature change-sensing function, a membrane-penetrating function, a gene-expressing function and a pH-sensing function. Conferring these functions as appropriate enables an improvement in the retention of the lipid nanoparticle in blood, an efficient endosomal escape by the lipid nanoparticle after endocytosis in the target cell, and more efficient expression of the encapsulated nucleic acid in the liver cell.

The lipid nanoparticle according to the present invention may comprise one or more substance(s) selected from a group consisting of antioxidants such as tocopherol, propyl gallate, ascorbyl palmitate or butylated hydroxytoluene, charged substances, and membrane polypeptides, etc. A charged substance that gives a positive charge can includes, for example, saturated or unsaturated aliphatic amines such as stearylamine and oleylamine. A charged substance that gives a negative charge includes, for example, dicetyl phosphate, cholesteryl hemisuccinate, phosphatidyl serine, phosphatidyl inositol and phosphatidic acid. Membrane polypeptides include, for example, membrane superficial polypeptides or membrane intrinsic polypeptides. The amounts of these substances combined are not particularly limited, and they are appropriately selected for the purpose.

The size of the lipid nanoparticles according to the present invention is such that, because this tends to provide high delivery efficiency to liver cells in vivo, the average particle diameter is preferably 400 nm or less, more preferably 300 nm or less, further preferably 200 nm or less, and even more preferably 150 nm or less. The average particle diameter of the lipid nanoparticles means the number-average particle diameter measured by dynamic light scattering (DLS). A measurement by the dynamic light scattering method can be performed conventionally using a commercially available DLS apparatus, etc.

The Polydispersity Index (PDI) of the lipid nanoparticle according to the present invention is approximately from 0.01 to 0.7, preferably approximately from 0.01 to 0.6, further preferably approximately from 0.01 to 0.3. The zeta potential can be in the range between -50 mV and 5 mV, preferably in the range between -10 mV and 5 mV.

The form of the lipid nanoparticle according to the present invention is not particularly limited, though it can include, for example, a single-membrane liposome, multilayered liposome or spherical micelle in a form being dispersed in an aqueous solvent, or an amorphous layered structure, etc. The lipid nanoparticle according to the present invention is preferably a unilamellar liposome or a multilayer liposome.

The lipid nanoparticle according to the present invention preferably includes an ingredient of interest to be delivered into the target cell within the particle which is covered by a lipid membrane. The ingredient that the lipid nanoparticle according to the present invention includes within the particle is not particularly limited as long as it has a size that can be included. The lipid nanoparticle according to the present invention can encapsulate any substance such as a nucleic acid, a saccharide, a peptide, a low molecular weight compound and a metal compound.

A preferred ingredient to be included in the lipid nanoparticle according to the present invention is a nucleic acid. The nucleic acid may be a DNA or an RNA, including analogues or derivatives thereof (e.g., a peptide nucleic acid (PNA) and phosphorothioated DNA, etc.). The nucleic acid to be included in the lipid nanoparticle according to the present invention may be a single-stranded nucleic acid or double-stranded nucleic acid, and may be in a linear or cyclic form.

The nucleic acid to be included in the lipid nanoparticle according to the present invention preferably comprises an exogenous gene to be expressed in the target cell. More preferably, it is a nucleic acid that is incorporated into a cell and thereby functions to express the exogenous gene in the cell. The exogenous gene may be a gene that is intrinsically included in the genome DNA of the target cell (preferably a liver cell), or a gene that is not included in the genome DNA. Such nucleic acid includes a gene expression vector comprising a nucleic acid that consists of a base sequence encoding the gene of interest to be expressed. The gene expression vector may be present as an extrachromosomal gene in the cell into which it is introduced, or may be incorporated into the genome DNA by homologous recombination.

A gene expression vector to be included in the lipid nanoparticle according to the present invention is not particularly limited, and vectors that are generally used in gene therapy, etc. can be used. The gene expression vector to be included in the lipid nanoparticle according to the present invention is preferably a nucleic acid vector such as a plasmid vector. The plasmid vector may be in a cyclic form as it is, or it may be cut beforehand into a linear form before being encapsulated into the lipid nanoparticle according to the present invention. The gene expression vector can be designed by a conventional method utilizing molecular biological tools that are generally used, based on the base sequence information of the target gene to be expressed, and can be produced by various known methods.

It is also preferred that the nucleic acid to be included in the lipid nanoparticle according to the present invention is a functional nucleic acid to control the expression of the target gene that is present in the target cell. Such a functional nucleic acid includes an antisense oligonucleotide, an antisense DNA, an antisense RNA, an siRNA, a microRNA, an mRNA, etc. Alternatively, it may be a plasmid DNA (pDNA) that serves as an siRNA expression vector for expressing siRNA in cells. The siRNA expression vector can be prepared from a commercially available siRNA expression vector, which may also be altered as appropriate. The nucleic acid to be encapsulated in the lipid nanoparticles according to the present invention is preferably mRNA or pDNA, due to their particularly good selectivity for the liver.

The method for producing a lipid nanoparticle according to the present invention is not particularly limited, and any method that can be available to a skilled artisan can be employed. As an example, it can be produced by dissolving all lipid components in an organic solvent such as chloroform; forming a lipid membrane by exsiccating the mixture under reduced pressure using an evaporator or by spray-drying it using a spray drier; and subsequently adding an aqueous solvent comprising the ingredient to be encapsulated in the lipid nanoparticle (e.g., a nucleic acid, etc.) to the mixture dried as described above; and further emulsifying it using a emulsifier such as a homogenizer, a ultrasonic emulsifier or a high-pressure splaying emulsifier, etc. It may also be produced by a method that is well known as a method for producing a liposome, for example, the reverse-phase evaporation method, etc. When it is desired to control the size of the liposome, an extrusion (extruding filtration) may be performed under a high pressure using a membrane filter having uniform pore diameter, etc.

The composition of the aqueous solvent (dispersion medium) is not particularly limited, and can include, for example, buffers such as a phosphate buffer, citrate buffer and phosphate buffered physiological saline, a physiological saline, and a medium for cell-culturing, etc. These aqueous solvents (dispersion media) can stably disperse the lipid nanoparticles, though, furthermore, sugars (solutions) such as monosaccharides (e.g., glucose, galactose, mannose, fructose, inositol, ribose and xylose), disaccharides (e.g., lactose, sucrose, cellobiose, trehalose and maltose), trisaccharides (e.g., raffinose and melezitose), polysaccharides (e.g., cyclodextrin), and a sugar alcohol (e.g., erythritol, xylitol, sorbitol, mannitol and maltitol), and a polyhydric alcohol (solution) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether and 1 ,3-butylene glycol, etc., may be added. In order to stably preserve the lipid nanoparticle dispersed in this aqueous solvent for a prolonged period, it is desirable to exclude the electrolytes in the aqueous solvent to the utmost, from a viewpoint of physical stability (e.g., for suppressing an aggregation). Moreover, from a viewpoint of chemical stability of the lipid, it is desirable to set the pH of the aqueous solvent from weak acid to near neutral (approximately from pH 3.0 to 8.0), and/or to eliminate dissolved oxygen by bubbling with nitrogen gas, etc.

The lipid nanoparticle according to the present invention can be produced by the alcohol dilution method using a flow path. This method produces lipid nanoparticles by introducing a solution in which a lipid component is dissolved in an alcohol solvent and a solution in which a water-soluble component to be included in the lipid nanoparticles is dissolved in an aqueous solvent through separate flow paths and allowing the two to join together. By using a micro-flow path with a built-in three-dimensional micromixer that can achieve instantaneous mixing of two liquids, lipid nanoparticles with a diameter of about 30 nm can be produced with good reproducibility (Non-Patent Literature 9). As the flow path that is used for production, it is preferable to use a simple two-dimensional flow path structure having a simple two-dimensional structure provided with a micro-sized flow path through which feedstock solutions are to be run and in which baffles (baffle plates) of specified widths in relation to the width of the flow path are alternately disposed from the two side faces as described in Patent Literature 2, because this enables the formation of a nano-sized lipid particle formation system with higher particle size controllability. As the aqueous solvent used in the alcohol dilution method, those mentioned above can be used.

When the obtained aqueous dispersion of the lipid nanoparticles is freeze-dried or spray-dried, its stability might be improved, for example, by using sugars (aqueous solutions) such as monosaccharides (e.g., glucose, galactose, mannose, fructose, inositol, ribose and xylose sugar), disaccharides (e.g., lactose, sucrose, cellobiose, trehalose and

maltose), trisaccharides (e.g., raffinose and melezitose), polysaccharides (e.g., cyclodextrin), and a sugar alcohol (e.g., erythritol, xylitol, sorbitol, mannitol and maltitol). Moreover, when the above-described dispersion is to be frozen, its stability might be improved, for example, by using the above-described sugars as well as polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether and 1 ,3-butylene glycol.

When the lipid nanoparticles according to the present invention in which a gene expression vector has been encapsulated are administered to an animal individual, the gene expression vector encapsulated in the lipid nanoparticles will be selectively expressed in the liver and spleen rather than in other organs. For example, by administering the lipid nanoparticles according to the present invention in which an exogenous gene of interest intended to be expressed in liver cells or spleen cells has been encapsulated to a subject animal, the exogenous gene can be expressed in the liver or spleen of the subject animal. Similarly, when the lipid nanoparticles according to the present invention in which an siRNA expression vector has been encapsulated are administered to an individual animal, the siRNA expression vector encapsulated in the lipid nanoparticles will selectively express in the liver and spleen rather than in other organs, thereby suppress the expression of the gene targeted by the expression vector.

Due to this highly selective gene expression activity in the liver or spleen, the lipid nanoparticles according to the present invention function as gene expression carriers for targeting the liver or spleen. By encapsulating an exogenous gene intended to be expressed in liver cells or spleen cells in the lipid nanoparticles according to the present invention and administering them to a subject animal, the exogenous gene is expressed in the liver or spleen of the subject animal. Therefore, the lipid nanoparticles of the present invention are useful as an active ingredient of a pharmaceutical composition used in gene therapy, and, in particular, are useful as an active ingredient of a pharmaceutical composition used in gene therapy for the liver or spleen as a target organ.

The animal to which the lipid nanoparticle according to the present invention is administered is not particularly limited, and it may be a human, or may be an animal other than human. Non-human animals include mammals such as cows, pigs, horses, sheep, goats, monkeys, dogs, cats, rabbits, mice, rats, hamsters and guinea pigs, and birds such as chickens, quails and ducks. Moreover, the route of administration for administrating the lipid nanoparticle according to the present invention to an animal is not particularly limited, though it is preferably a parenteral administration such as an intravenous administration, enteral administration, intramuscular administration, subcutaneous administration, transdermal administration, nasal administration and pulmonary administration.

[Gene Carrier for Genome Editing]

The lipid nanoparticles according to the present invention can also be used as gene carriers for gene therapy using genome editing by encapsulating therein nucleic acids and the like that are necessary for genome editing. For instance, the genome editing by the CRISPR/Cas9 system uses an RNP, which is a complex of crRNA, tracrRNA, and Cas9 protein. By expressing an RNP in a target cell, or by delivering the RNP itself into the target cell, it is possible to induce a knockout of the targeted gene. Furthermore, a gene knock-in can also be induced by simultaneously delivering a donor DNA. Therefore, the lipid nanoparticles according to the present invention can be used as gene carriers for genome editing targeting liver cells or spleen cells by encapsulating therein an RNP that consists of a guide RNA (gRNA) consisting of crRNA and tracrRNA and a Cas9 protein. Since the RNP itself is directly loaded onto the lipid nanoparticles and introduced into target cells, it causes less off-target effect than with methods in which a gene encoding a DNA nuclease such as Cas9 protein is introduced into target cells and expressed.

In the inventions and the specification of this application, the DNA nuclease to be loaded onto the lipid nanoparticle is an enzyme that binds to a DNA in gRNA-dependent manner and recognizes and cleaves the double-stranded DNA formed by the pairing of the DNA with a part of the gRNA. Examples of such DNA nucleases include Cas9, Cpf1, etc.

In the inventions and the specification of this application, the Cas9 protein is a protein that binds to a DNA in a gRNA-dependent manner and has at least one of RuvC nuclease activity and HNH nuclease activity. The Cas9 protein that has both RuvC and HNH nuclease activities cleaves a double-strand in the genomic DNA. The Cas9 protein that has only either RuvC nuclease activity or HNH nuclease activity cleaves only one of the two chains of the genomic DNA.

The Cas9 protein used in the present invention may be a wild-type Cas9 protein derived from a bacterium having a CRISPR system, or a mutant protein obtained by modifying the wild-type protein. The bacteria having a CRISPR system include, for example, Streptococcus pyogenes, Staphylococcus aureus, Neisseria meningitidis, Campylobacter jejuni, Geobacillus stearothermophilus, Streptococcus thermophilus, and Treponema denticola, etc. The mutant proteins obtained by modifying the wild-type Cas9 proteins include a mutant into which a mutation has been introduced that inactivates either the RuvC nuclease activity or the HNH nuclease activity. Examples of such mutants include a mutant in which the 10th aspartic acid in the wild-type Cas9 protein is replaced with alanine (Cas9(D10A)). Cas9(D10A) functions as a DNA nickase and is therefore also called Cas9 nickase (Cas9n). Alternatively, a mutant protein in which a mutation that

does not affect the nuclease activity of the wild-type Cas9 protein has been introduced may also be used.

The Cas9 protein used in the present invention may be a wild-type Cas9 protein or a mutant protein thereof to which various peptides have been added, or may be a chimeric protein fused with another protein. Examples of such peptides include tag peptides such as His-tag, Myc-tag and Flag-tag, and signal peptides such as nuclear translocation signal peptides. Examples of other proteins to be fused to the wild-type or mutant Cas9 protein include GST, fluorescent proteins, and the like.

In the invention and this specification of this application, the Cpf1 protein is a protein that binds to a DNA in gRNA-dependent manner and has only RuvC nuclease activity. The Cas9 protein that has both RuvC and HNH nuclease activities cleaves the double strands of the genomic DNA to form blunt ends, whereas the Cpf1 protein forms 5' overhanging ends.

The Cpf1 protein used in the present invention may be a wild-type Cpf1 protein derived from a bacterium having a CRISPR/Cpf1 system, or may be a mutant protein obtained by modifying the wild-type protein. Examples of bacteria having the CRISPR/Cpf1 system include Acidaminococcus sp. and Lachnospiraceae bacterium. Furthermore, examples of mutant proteins modified from wild-type Cpf1 protein include a mutant into which a mutation has been introduced that increases nuclease activity, and a mutant protein into which a mutation has been introduced that does not affect the nuclease activity.

The Cpf1 protein used in the present invention may be a wild-type Cpf1 protein or a mutant protein thereof to which various peptides have been added, or may be a chimeric protein fused with another protein. As the peptides and other proteins, those that are similar to the peptides and other proteins listed for the Cas9 protein can be used.

In the inventions and the specification of this application, the gRNA is an RNA having a base sequence that is capable of pairing with a target sequence (a base sequence to be a subject of genome-edition) on a genomic DNA to be cleaved by a DNA nuclease. The "base sequence that is capable of pairing with a target sequence" is usually a base sequence that is homologous (identical) or complementary to the target sequence in order to suppress recognition of a region other than the target sequence. The gRNA may consist of a single RNA, or may consist of two or more RNAs forming a complex.

When the DNA nuclease loaded onto the lipid nanoparticle is a Cas9 protein, crRNA and tracrRNA can be used as the gRNA. The crRNA is derived from bacteria having the CRISPR system and is a single-stranded RNA that comprises a region consisting of a base sequence complementary to a part of tracrRNA (tracrRNA-binding region), and a region consisting of a nucleotide sequence homologous or complementary to a target sequence on genomic DNA(genomic DNA-binding region). The tracrRNA is also derived from bacteria having the CRISPR system, and is a single-stranded RNA that has a region consisting of a base sequence complementary to a part of the crRNA (crRNA-binding region), and hybridizes with the crRNA in this region to form a hairpin structure. The Cas9 protein recognizes the hairpin structure, thereby forming an RNP. The crRNA and tracrRNA may each be an independent single-stranded RNA, or may be a single-stranded RNA in which the two are linked via a suitable RNA linker. Examples of bacteria having a CRISPR system include those described above. The Cas9 protein, the crRNA and the tracrRNA may all be derived from the same species of bacteria, or may be derived from different species of bacteria. Furthermore, the crRNA and tracrRNA may consist only of natural RNA, or may contain modified RNA or artificial nucleic acids in part or in whole, as long as the function of the CRISPR/Cas9 system is not impaired.

When the DNA nuclease loaded onto the lipid nanoparticle is Cpf1 protein, the gRNA may be any RNA containing a target sequence, as with the crRNA, and tracrRNA is not necessary. Because of this, the gRNA can be made shorter than when using the Cas9 protein. Moreover, the gRNA may consist only of natural RNA, or may contain modified RNA or artificial nucleic acid in part or in whole, as long as the function of the CRISPR/Cpf1 system is not impaired.

The target sequence is usually selected from the base sequence of the region immediately followed by the PAM sequence. The PAM sequence is a sequence recognized by a DNA nuclease such as the Cas9 protein, and is determined depending on the DNA nuclease to be used (e.g., the Cas9 protein). An example of the PAM sequence recognized by the Cas9 protein is 5'-NGG (N: A, G, C, T), and an example of the PAM sequence recognized by the Cpf1 protein is 5'-TTTV (V: A, G, C) and 5'-TTTN (N: A, G, C, T). The nucleotide length of the target sequence in a gRNA such as crRNA is not particularly limited, and can be, for example, about 15 to 30 bases long, preferably 18 to 22 bases long.

When the lipid nanoparticles according to the present invention are used as gene carriers for genome editing using the CRISPR/Cas9 system, in order to further improve genome editing efficiency, it is preferable that the lipid nanoparticles according to the present invention further comprise a DNA nuclease, a gRNA, and a single-stranded oligonucleotide (ssON) containing a region that is capable of paring with a part of the gRNA. The region in an ssON that is capable of pairing with a gRNA is called a "gRNA-pairing region," and a region in a gRNA that pairs with a part of an ssON is called an "ssON-pairing region." Pairing of the gRNA-binding region and the ssON-binding region forms an RNP, which is a complex of the DNA nuclease, gRNA, and ssON.

The ssON may be an oligonucleotide consisting of only DNA, an oligonucleotide consisting of only RNA, or a chimeric oligonucleotide containing both DNA and RNA. Moreover, the ssONs may consist only of natural RNA or DNA, or may contain modified nucleic acids or artificial nucleic acids in part or in whole, as long as the function of the CRISPR

system to be used is not impaired.

Unlike nucleic acids, the Cas9 protein and the Cpf1 protein are positively charged, and therefore have low loading efficiency onto the lipid nanoparticles that contain the pH-sensitive cationic lipids as lipid components that constitute the lipid membrane. In contrast, when the nucleic acid that complexes with a DNA nuclease such as Cas9 protein includes not only gRNA(e.g., crRNA and tracrRNA) but also ssON, the amount of nucleic acid contained in the RNP is large, so the positive charge of the RNP is suppressed and the negative charge becomes stronger, and the RNP can efficiently be loaded onto the lipid nanoparticles comprising the pH-sensitive cationic lipids.

The nucleotide length of the ssON used in the present invention can be appropriately determined taking into consideration the base length of the gRNA, for example, the crRNA and tracrRNA, the type of Cas9 protein, etc., as long as it is long enough to negatively charge the RNP. The length of the ssON can be, for example, about 50 to 500 bases long.

When the DNA nuclease loaded onto the lipid nanoparticle is a Cas9 protein, it includes a region consisting of a base sequence complementary to a partial region of the crRNA other than the tracrRNA-binding region. The ssON hybridizes with the crRNA via the region complementary to the crRNA (crRNA-binding region). In other words, crRNA and tracrRNA, and crRNA and ssON hybridize with each other to form a ternary complex, which is then complexed with the Cas9 protein. The region in the crRNA that hybridizes with the ssON (ssON-binding region) is not particularly limited, as long as the crRNA can simultaneously hybridize with the tracrRNA and with the ssON to form a ternary complex, and this ternary complex can form a hairpin structure recognized by the Cas9 protein. For instance, the ssON-binding region in the crRNA may include a part or all of the genomic DNA-binding region, or may be identical to the genomic DNA-binding region. In the case of the double nicking method using Cas9 nickase (Cas9n) in which one of the RuvC nuclease activity and the HNH nuclease activity has been inactivated, two types of guide RNA are used. Here, if the DNA nuclease to be loaded onto the lipid nanoparticles is the Cas9n and the lipid nanoparticles is loaded with an RNP for the double nicking method, two types of ssONs that hybridize to the respective guide RNAs are used.

When a gene fragment of interest is knocked into the cleaved genomic DNA using the CRISPR system, the ssON can also be used as the donor DNA to be knocked in. For example, an ssON that has, in addition to a binding region for gRNA such as crRNA, a region in which regions of homologous sequence (homology arms) of 40 to 60 bp for homologous recombination are added to both ends of the gene fragment to be knocked in functions as a donor DNA.

Determination of target sequences on genomic DNA, designing of base sequences of gRNAs such as crRNA and tracrRNA, and designing of base sequences of ssONs, etc., can be performed by conventional methods using commonly used molecular biology tools based on the base sequence information of genomic DNA. For example, gRNA can be designed using design tools such as CRISPR Design Tool (Horizon Discovery) and TrueDesign Genome Editor (Invitrogen).

In addition, the lipid nanoparticle encapsulating the RNP, which is a complex of the DNA nuclease, gRNA, and ssON, may be a lipid nanoparticle other than those according to the present invention. For instance, lipid nanoparticles as disclosed in Patent Literatures 1 and 2 can also be used for encapsulation as a gene carrier suitable for genome editing.

The gRNA pairing region in the ssON used in the present invention may be a base sequence that is completely complementary to the ssON pairing region in the gRNA (perfect match sequence), but it is preferable that the base sequence has some non-complementary bases (mismatch bases). When the gRNA pairing region is a perfect match sequence with the ssON pairing region, the RNP, which is a complex of DNA nuclease, gRNA, and ssON, has excellent stability, and lipid nanoparticles encapsulating the RNP can be produced stably; however, when the lipid nanoparticles are taken up into cells, the RNP may be difficult to dissociate within the cells. When the gRNA pairing region has a suitable number of mismatched bases with respect to the ssON pairing region, the balance between the stability during production of the RNP and the ease of dissociation within the cell is good, making it possible to create a gene carrier which is excellent in both production stability and genome editing efficiency.

In the gRNA pairing region in the ssON, it is preferable that 30 to 60% of the bases are complementary to the corresponding bases in the ssON pairing region, and it is more preferable that 35 to 55% of the bases are complementary to the corresponding bases in the ssON pairing region. In addition, the Tm value between the gRNA-pairing region in the ssON and the ssON-pairing region in the gRNA is preferably from 25 to 40 °C, more preferably from 25 to 35 °C, and even more preferably from 25 to 30 °C. The Tm value can be calculated, for example, by the Wallace method when the length is less than 18 bases, and by Nearest-Neighbor method for base pairs when the length is 18 bases or more.

As a lipid nanoparticle containing the above-described DNA nuclease, gRNA, and a single-stranded oligonucleotide (ssON) containing a region capable of pairing with a part of the gRNA as a gene carrier for genome editing using the CRISPR/Cas9 system, it is also preferable to use lipid nanoparticles comprising a pH-sensitive cationic lipid represented by the following general formula (II) (hereinbelow, may be referred to as "pH-sensitive cationic lipid (II)") and/or a pH-sensitive cationic lipid represented by the following general formula (III) (hereinbelow, may be referred to as "pH-sensitive cationic lipid (III)") as a constituent lipid, instead of or in addition to the pH-sensitive cationic lipid of the present invention. Even when lipid nanoparticles containing these pH-sensitive cationic lipids are used as gene carriers, it is preferable that 30 to 60% of the bases in the gRNA pairing region of the ssON are complementary to the corresponding bases in the ssON pairing region, and it is more preferable that 35 to 55% of the bases are complementary to the

corresponding bases in the ssON pairing region. In addition, the Tm value between the gRNA-pairing region in the ssON and the ssON-pairing region in the gRNA is preferably from 25 to 40 °C, more preferably from 25 to 35 °C, and even more preferably from 25 to 30 °C.

[Chem. 7]

$$(R^{21})(R^{22})C(OH)\text{-}(CH_2)a\text{-}(O\text{-}CO)b\text{-}X \qquad (II)$$

In the general formula (II), a denotes an integer from 3 to 5, though it is preferably 4. b denotes 0 or 1. When b is 0, the -O-CO-group is absent, meaning that it is a single bond.

In the general formula (I), $R^{21}$ and $R^{22}$ each independently denote a group expressed by the following general formula (A'). In general formula (A'), q represents an integer from 1 to 9; r represents 0 or 1; s represents an integer from 1 to 3; t represents 0 or 1; u represents an integer from 1 to 8; f represents 0 or 1; and v represents an integer from 4 to 12. However, when b and f are simultaneously 0, the cases where q is an integer from 3 to 5, r and t are 1, s is 1, and u + v is an integer from 6 to 10 are excluded.

[Chem. 8]

$$CH_3\text{-}(CH_2)q\text{-}(CH{=}CH)_r\text{-}(CH_2)_s\text{-}(CH{=}CH)_t\text{-}(CH_2)_u\text{-}(CO\text{-}O)_f\text{-}(CH_2)v\text{-} \qquad (A')$$

With respect to the pH-sensitive cationic lipid (II), it is preferred that a hydrocarbon chain of $R^{21}$ and $R^{22}$ is a relatively long chain in view of the stability of the lipid nanoparticle. In specific, in the pH-sensitive cationic lipid (II), it is preferred that $R^{21}$ and $R^{22}$ are groups having 20 or more carbon atoms. Namely, it is preferred that, in the general formula (A'), q + 2r + s + 2t + u + f + v is an integer equal to or greater than 19. In particular, with respect to the pH-sensitive cationic lipid (II), q + 2r + s + 2t + u + f + v is preferably an integer from 19 to 33, more preferably an integer from 19 to 31, further preferably an integer from 21 to 31, yet further preferably an integer from 21 to 27.

In the pH-sensitive cationic lipid (II), $R^{21}$ and $R^{22}$ are, in the context of the general formula (A'), preferably groups in which r is 1, t is 0, q is an integer from 5 to 11, preferably an integer from 6 to 10, s + u is an integer from 5 to 11, preferably an integer from 6 to 10, f is 1, v is an integer from 4 to 12, and q + s + u + v is an integer equal to or greater than 16; groups in which r is 0, t is 1, q + s is an integer from 5 to 11, preferably an integer from 6 to 10, u is an integer from 5 to 8, f is 1, v is an integer from 4 to 12, and q + s + u + v is an integer equal to or greater than 16; groups in which r and t are 0, q + s + u is an integer from 13 to 23, preferably from 15 to 21, f is 1, v is an integer from 4 to 12, and q + s + u + v is an integer equal to or greater than 18; groups in which r and t are 0, q + s + u is an integer from 13 to 23, preferably from 15 to 21, f is 1, v is an integer from 6 to 10, and q + s + u + v is an integer equal to or greater than 18.

In the pH-sensitive cationic lipid (II), $R^{21}$ and $R^{22}$ may be any group as long as being represented by the general formula (A'). $R^{21}$ and $R^{22}$ may be different groups from each other, though preferably they are the same groups.

In the general formula (II), X is the same as X in the general formula (I).

The pH-sensitive cationic lipid of the present invention is preferably a lipid of the general formula (II) in which a is an integer from 3 to 5, b is 1, X is a 5- or 7-membered non-aromatic heterocyclic group (provided that it is bound to (O-OC)b- via a carbon atom in the heterocyclic group), preferably 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group), and $R^{21}$ and $R^{22}$ are each independently a group of the general formula (A') in which r is 1, t is 0, q is an integer from 5 to 11, preferably an integer from 6 to 10, s + u is an integer from 5 to 11, preferably an integer from 6 to 10, f is 1, v is an integer from 4 to 12, and q + s + u + v is an integer equal to or greater than 16; a lipid of the general formula (II) in which a is an integer from 3 to 5, b is 1, X is a 5- or 7-membered non-aromatic heterocyclic group (provided that it is bound to (O-OC)b- via a carbon atom in the heterocyclic group), preferably 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group), and $R^{21}$ and $R^{22}$ are each independently a group of the general formula (A') in which r is 0, t is 1, q + s is an integer from 5 to 11, preferably an integer from 6 to 10, u is an integer from 5 to 8, f is 1, v is an integer from 4 to 12, and q + s + u + v is an integer equal to or greater than 16; a lipid of the general formula (II) in which a is an integer from 3 to 5, b is 0, X is a group of the general formula (B) in which d is 0, $R^3$ and $R^4$ is are each independently a $C_{1-4}$ alkyl group or $C_{2-4}$ alkenyl group (the $C_{1-4}$ alkyl group or $C_{2-4}$ alkenyl group indicated by $R^3$ and $R^4$ may have one or two hydrogen atom(s) substituted by (a) phenyl group(s)), and $R^{21}$ and $R^{22}$ are each independently a group of the general formula (A') in which r is 1, t is 0, q is an integer from 5 to 11, preferably an integer from 6 to 10, s + u is an integer from 5 to 11, preferably an integer from 6 to 10, f is 1, v is an integer from 4 to 12, and q + s + u + v is an integer equal to or greater than 16; a lipid of the general formula (II) in which a is an integer from 3 to 5, b is 0, X is a group of the general formula (B) in which d is 0, $R^3$ and $R^4$ is are each independently a $C_{1-4}$ alkyl group or $C_{2-4}$ alkenyl group (the $C_{1-4}$ alkyl group or $C_{2-4}$ alkenyl group indicated by $R^3$ and $R^4$ may have one or two hydrogen atom(s) substituted by (a) phenyl group(s)), and $R^{21}$ and $R^{22}$ are each independently a group of the general formula (A') in which r is 0, t is 1 q + s is an integer from 5 to 11, preferably an integer from 6 to 10, u is an integer from 5 to 8, f is 1, v is an integer from 4 to 12, and q

+ s + u + v is an integer equal to or greater than 16. Among these lipids, lipids in which $R^{21}$ and $R^{22}$ are the same group are more preferred as the pH-sensitive cationic lipid represented by the general formula (II), and lipids in which $R^{21}$ and $R^{22}$ are the same group and a is 4 are particularly preferred.

[Chem. 9]

$$(R^{31})(R^{32})C(OH)\text{-}(CH_2)a\text{-}(O\text{-}CO)b\text{-}X \qquad \text{(III)}$$

In the general formula (III), a denotes an integer from 3 to 5, though it is preferably 4.

b denotes 0 or 1. When b is 0, the -O-CO-group is absent, meaning that it is a single bond.

In the general formula (III), $R^{31}$ and $R^{32}$ each independently denote a group expressed by the following general formula (A"). In the general formula (A"), $R^{33}$ and $R^{34}$ each independently denote a linear or branched $C_{5-15}$ alkyl group (an alkyl group having 5 to 15 carbon atoms); g denotes 0 or 1; and h denotes an integer from 4 to 12.

[Chem. 10]

$$(R^{33})(R^{34})\text{-}CH\text{-}(CO\text{-}O)g\text{-}(CH_2)h\text{-} \qquad \text{(A")}$$

As the linear or branched $C_{5-15}$ alkyl group, similar groups to those listed for $R^{11}$ can be used.

In the general formula (A"), $R^{33}$ and $R^{34}$ are each independently preferably a linear or branched $C_{5-12}$ alkyl group (an alkyl group having 5 to 12 carbon atoms), more preferably a linear or branched $C_{5-10}$ alkyl group (an alkyl group having 5 to 10 carbon atoms), and further preferably a linear or branched $C_{6-9}$ alkyl group (an alkyl group having 6 to 9 carbon atoms). In the pH-sensitive cationic lipid (III), $R^{31}$ and $R^{32}$ may be the same group or different groups as long as they are groups represented by the general formula (A").

In the general formula (III), X is the same as X In the general formula (I).

The pH-sensitive cationic lipid (III) is, more preferably, a compound in which $R^1$ and $R^2$ in the general formula (III) are each independently a group of the general formula (A") in which $R^{33}$ and $R^{34}$ are each independently a linear $C_{6-9}$ alkyl group, g is 1, and h is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (III) are each independently a group of the general formula (A") in which $R^{33}$ and $R^{34}$ are each independently a branched $C_{6-9}$ alkyl group, g is 1, and h is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (III) are each independently a group of the general formula (A") in which $R^{33}$ and $R^{34}$ are each independently a linear $C_{6-9}$ alkyl group, g is 1, and h is an integer from 6 to 10, and a is an integer from 3 to 5, b is 0, and X is a group of the general formula (B) in which d is 0, and $R^3$ and $R^4$ are each independently a $C_{1-4}$ alkyl group; a compound in which $R^1$ and $R^2$ in the general formula (III) are each independently a group of the general formula (A") in which $R^{33}$ and $R^{34}$ are each independently a branched $C_{6-9}$ alkyl group, g is 1, and h is an integer from 6 to 10, and a is an integer from 3 to 5, b is 0, and X is a group of the general formula (B) in which d is 0, and $R^3$ and $R^4$ are each independently a $C_{1-4}$ alkyl group.

The pH-sensitive cationic lipid (III) is, particularly preferably, a compound in which $R^1$ and $R^2$ in the general formula (III) are the same group, and which is a group of the general formula (A") in which $R^{33}$ and $R^{34}$ are each independently a linear $C_{6-9}$ alkyl group, g is 1, and h is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (III) are the same group, and which is a group of the general formula (A") in which $R^{33}$ and $R^{34}$ are each independently a branched $C_{6-9}$ alkyl group, g is 1, and h is an integer from 6 to 10, and a is an integer from 3 to 5, b is 1, and X is 1-pyrrolidinyl group, 1-piperidinyl group, 1-morpholinyl group, or 1-piperazinyl group (which is bound to (O-CO)b- via a carbon atom in the ring, and one hydrogen atom may be substituted with a $C_{1-4}$ alkyl group or a $C_{2-4}$ alkenyl group); a compound in which $R^1$ and $R^2$ in the general formula (III) are the same group, and which is a group of the general formula (A") in which $R^{33}$ and $R^{34}$ are each independently a linear $C_{6-9}$ alkyl group, g is 1, and h is an integer from 6 to 10, and a is an integer from 3 to 5, b is 0, and X is a group of the general formula (B) in which d is 0, and $R^3$ and $R^4$ are each independently a $C_{1-4}$ alkyl group; a compound in which $R^1$ and $R^2$ in the general formula (III) are the same group, and which is a group of the general formula (A") in which $R^{33}$ and $R^{34}$ are each independently a branched $C_{6-9}$ alkyl group, g is 1, and h is an integer from 6 to 10, and a is an integer from 3 to 5, b is 0, and X is a group of the general formula (B) in which d is 0, and $R^3$ and $R^4$ are each independently a $C_{1-4}$ alkyl group.

The pKa of the pH-sensitive cationic lipid (II) and the pH-sensitive cationic lipid (III) is not particularly limited, but can be selected, for example, from about 4.0 to 9.0, preferably from about 4.5 to 8.5, and more preferably from about 6 to 8. It is preferable to select the type of each substituent so as to give a pKa in this range.

The pH-sensitive cationic lipid (II) and the pH-sensitive cationic lipid (III) can be easily produced, for example, by the method specifically shown in the Examples of this specification. A skilled artisan can easily produce any lipid encompassed within the scope of the general formula (II) or the general formula (III) by referring to this method for production and appropriately selecting ingredient compounds, reagents and reaction conditions, etc.

## EXAMPLES

Next, the present invention will be described in more detail with reference to Examples, though the present invention is not to be limited by the following Examples.

<NMR>

In the following experiments, unless otherwise specified, $^1$H and $^{13}$C NMR spectra were obtained using a JEOL ECA500 or ECS400 instrument with tetramethylsilane as the internal standard (0 ppm). Noted that d, t, and m are abbreviations for doublet, triplet, and multiplet, respectively. Chemical shifts for $^1$H NMR spectra are reported on the $\sigma$ scale in ppm downfield from tetramethylsilane.

<Thin Layer Chromatography (TLC)>

In the experiments that follow, unless otherwise stated, the reactants in all reactions were monitored by TLC using precoated TLC plates (Millipore). Visualization of TLC was carried out by UV light (254 nm), phosphomolybdic acid staining, or p-anisaldehyde staining. Reaction products were purified by automated combiflash® Rf chromatography system (Teledyne ISCO) or Selekt system (Biotage).

<Constituent Lipids of Lipid Nanoparticles>

Cholesterols was manufactured by SIGMA Aldrich, and 1,2-distearoyl-sn-glycero-3-phosphocholine (DSPC), 1,2-dioleoyl-sn-glycero-3-phosphoethanolamine (DOPE), polyethylene glycol 2000-modified 2-dimyristoyl-rac-glycero, and polyethylene glycol 2000-modified dimyristoyl glycerol (PEG-DMG) were manufactured by NOF Corporation. SM-102 (hereinbelow may be abbreviated as "SM") was manufactured by Cayman Chemical (catalog number: PIN 33474), ALC-0315 (hereinbelow may be abbreviated as "ALC") by Ambeed (catalog number: PIN A1375212), and DLin-MC3-DMA (MC3) by MedChemExpress (catalog number: PIN HY-112251), respectively.

<mRNA>

Among the nucleic acids to be encapsulated in the lipid nanoparticles, the mRNA encoding firefly luciferase (Flue) (CleanCap FLuc mRNA, 5 moU) was manufactured by TriLink Biotechnologies.

<Preparation of RNP Complexes>

Recombinant spCas9 nuclease (TrueCut Cas9 Protein v2) was manufactured by Thermo Fisher Scientific, and single guide RNA (sgRNA) and single-stranded oligodeoxyribonucleotide (ssODN) by Integrated DNA Technologies, respectively. As sgRNA, two types of sgRNA (sgTTR-G211, sgTTR-G269) targeting the TTR gene were used. The base sequences of the sgRNAs are shown in Table 1.

[Table 1]

| sgRNA | Base sequence | SEQ ID NO |
|---|---|---|
| sgTTR-G211 | mU*mU*mA*CAGCCACGUCUACAGCAGUUUUAGAGCUAGAAAUAGCAAGUUAAAAUAAGGCU AGUCCGUUAUCAACUUGAAAAAGUGGCACCGAGUCGGUGCmU*mU*mU*U | 1 |
| sgTTR-G269 | mC*mC*mC*AUACUCCUACAGCACCAGUUUUAGAGCUAGAAAUAGCAAGUUAAAAUAAGGCU AGUCCGUUAUCAACUUGAAAAAGUGGCACCGAGUCGGUGCmU*mU*mU*U | 2 |

As ssODNs that pair with sgTTR-G211, seven types listed in Table 2 (ssODN-TTR_3-100%, ssODN-TTR_3-80%, ssODN-TTR_3-60%, ssODN-TTR_3-50%, ssODN-TTR_3-40%, ssODN-TTR_3-30%, ssODN-TTR_3-0%), which have from 30% to 100% matched sequences with the ssON-pairing region in the sgRNA, were used. As ssODNs that pair with sgTTR-G269, four types listed in Table 2 (ssODN-TTR_4-60%, ssODN-TTR_4-50%, ssODN-TTR_4-40%, ssODN-TTR_4-30%), which have from 30% to 100% matched sequences with the ssON-pairing region in the sgRNA, were used.

[Table 2]

| ssODN | Base sequence | SEQ ID NO |
|---|---|---|
| ss0DN-TTR_3-100% | AAAAAAAAAAAAAAAAAAAAAAAAAAAAAAAAAAAAAAAAAAAAAAAAAA<br>ATGCTGTAGACGTGGCTGTAAAAAAAAAAAAAAAAAAAAAAAAAAAAAAA<br>AAAAAAAAAAAAAAAAAAAA | 3 |
| ssODN-TTR_3-80% | AAAAAAAAAAAAAAAAAAAAAAAAAAAAAAAAAAAAAAAAAAAAAAAAAA<br>AAACTGTAGACGTGGCTGTTTAAAAAAAAAAAAAAAAAAAAAAAAAAAAA<br>AAAAAAAAAAAAAAAAAAAA | 4 |
| ssODN-TTR_3-60% | AAAAAAAAAAAAAAAAAAAAAAAAAAAAAAAAAAAAAAAAAAAAAAAAAA<br>AAAAAGTAGACGTGGCTAATTAAAAAAAAAAAAAAAAAAAAAAAAAAAAA<br>AAAAAAAAAAAAAAAAAAAA | 5 |
| ssODN-TTR_3-50% | AAAAAAAAAAAAAAAAAAAAAAAAAAAAAAAAAAAAAAAAAAAAAAAAAA<br>AAAAAATAGACGTGGCAAATTAAAAAAAAAAAAAAAAAAAAAAAAAAAAA<br>AAAAAAAAAAAAAAAAAAAA | 6 |
| ssODN-TTR_3-40% | AAAAAAAAAAAAAAAAAAAAAAAAAAAAAAAAAAAAAAAAAAAAAAAAAA<br>AAAAAAAAGACGTGGAAAATTAAAAAAAAAAAAAAAAAAAAAAAAAAAAA<br>AAAAAAAAAAAAAAAAAAAA | 7 |
| ssODN-TTR_3-30% | AAAAAAAAAAAAAAAAAAAAAAAAAAAAAAAAAAAAAAAAAAAAAAAAAA<br>AAAAAAATGACGTGAAAAATTAAAAAAAAAAAAAAAAAAAAAAAAAAAAA<br>AAAAAAAAAAAAAAAAAAAA | 8 |
| ssODN-TTR_3-0% | AAAAAAAAAAAAAAAAAAAAAAAAAAAAAAAAAAAAAAAAAAAAAAAAAA<br>AAAAAAATATAAAAAAAAATTAAAAAAAAAAAAAAAAAAAAAAAAAAAAA<br>AAAAAAAAAAAAAAAAAAAA | 9 |
| ssODN-TTR_4-80% | AAAAAAAAAAAAAAAAAAAAAAAAAAAAAAAAAAAAAAAAAAAAAAAAAA<br>AAAGTGCTGTAGGAGTATGAAAAAAAAAAAAAAAAAAAAAAAAAAAAAAA<br>AAAAAAAAAAAAAAAAAAAA | 10 |
| ssODN-TTR_4-60% | AAAAAAAAAAAAAAAAAAAAAAAAAAAAAAAAAAAAAAAAAAAAAAAAAA<br>AAAAAGCTGTAGGAGTAAAAAAAAAAAAAAAAAAAAAAAAAAAAAAAAAA<br>AAAAAAAAAAAAAAAAAAAA | 11 |
| ssODN-TTR4_50% | AAAAAAAAAAAAAAAAAAAAAAAAAAAAAAAAAAAAAAAAAAAAAAAAAA<br>ATGCTGTAGACGTGGCTGTAAAAAAAAAAAAAAAAAAAAAAAAAAAAAAA<br>AAAAAAAAAAAAAAAAAAAA | 12 |
| ssODN-TTR_4-40% | AAAAAAAAAAAAAAAAAAAAAAAAAAAAAAAAAAAAAAAAAAAAAAAAAA<br>AAAAAAATGTAGGAGATAAAAAAAAAAAAAAAAAAAAAAAAAAAAAAAAA<br>AAAAAAAAAAAAAAAAAAAA | 13 |

(continued)

| ssODN | Base sequence | SEQ ID NO |
|---|---|---|
| ssODN-TTR_4-30% | AAAAAAAAAAAAAAAAAAAAAAAAAAAAAAAAAAAAAAAAAAAAAAAAAAAAAAA<br>AAAAAAAAGTAGGAAATAAAAAAAAAAAAAAAAAAAAAAAAAAAAAAAAAAAAAA<br>AAAAAAAAAAAAAAAAAAAAAA | 14 |
| ssODN-polyA | AAAAAAAAAAAAAAAAAAAAAAAAAAAAAAAAAAAAAAAAAAAAAAAAAAAAAAA<br>AAAAAAAAAAAAAAAAAAAAAAAAAAAAAAAAAAAAAAAAAAAAAAAAAAAAAAA<br>AAAAAAAAAAAAAAAAAAAAAA | 15 |

As ssODNs that pair with sgTTR-G211, the six types described in Table 3 (ssODN-TTR_3-10°C-3', ssODN-TTR_3-10°C-5', ssODN-TTR_3-24°C-3', ssODN-TTR_3-24°C-5', ssODN-TTR_3-50°C-3', ssODN-TTR_3-50°C-5') in which a mismatch sequence in the sgRNA with respect to the ssON-pairing region exists on either the 5' or 3' side were also used.

When measuring the Tm value of sgRNA and ssODN in the RNP complex using the fluorescence quenching method, sgRNA modified at the 5' end with the fluorescent substance FAM and ssODN modified at the 3' end with the quenching substance IBFQ (Iowa Black® FQ) were used. As the FAM-modified sgRNA, an RNA in which FAM was linked to the 5' end of sgTTR-G211 (sgTTR-G211-5FAM) was used. The IBFQ-modified ssODN used was an IBFQ-modified RNA (ssODN-80%-3IBFQ) in which IBFQ was bound to the 3' end of a sequence that is 80% matched with the ssON-paired region in the sgRNA (ssODN-80%) , an IBFQ-modified RNA (ssODN-40%-3IBFQ) in which IBFQ was bound to the 3' end of a sequence that is 40% matched with the ssON-paired region (ssODN-40%), or an IBFQ-modified RNA (ssODN-0%-3IBFQ) in which IBFQ was bound to the 3' end of an RNA that is a sequence that is 0% matched with the ssON-paired region (ssODN-0%).

[Table 3]

| | | SEQ ID NO |
|---|---|---|
| ssODN-TTR_3-10°C-3' | AAAAAAAAAAAAAAAAAAAAAAAAAAAAAAAAAAAAAAAAAAAA<br>AAAAAAAAAAAAAAAAAATATAAAAAAAGTAAAAAAAAAAAAAA<br>AAAAAAAAAAAAAAAAAAAAAAAAAAAAAAAAAAAAAAAAAAAA | 16 |
| ssODN-TTR_3-10°C-5' | AAAAAAAAAAAAAAAAAAAAAAAAAAAAAAAAAAAAAAAAAAAA<br>AAAAAAAAAAAATGCAAATATAAAAAAAAATTAAAAAAAAAAAA<br>AAAAAAAAAAAAAAAAAAAAAAAAAAAAAAAAAAAAAAAAAAAA | 17 |
| ssODN-TTR_3-24°C-3' | AAAAAAAAAAAAAAAAAAAAAAAAAAAAAAAAAAAAAAAAAAAA<br>AAAAAAAAAAAAAAAAAATATAAAGGCTGTAAAAAAAAAAAAAA<br>AAAAAAAAAAAAAAAAAAAAAAAAAAAAAAAAAAAAAAAAAAAA | 18 |
| ssODN-TTR_3-24°C-5' | AAAAAAAAAAAAAAAAAAAAAAAAAAAAAAAAAAAAAAAAAAAA<br>AAAAAAAAAAAATGCTGTAGTAAAAAAAAATTAAAAAAAAAAAA<br>AAAAAAAAAAAAAAAAAAAAAAAAAAAAAAAAAAAAAAAAAAAA | 19 |
| ssODN-TTR_3-50°C-3' | AAAAAAAAAAAAAAAAAAAAAAAAAAAAAAAAAAAAAAAAAAAA<br>AAAAAAAAAAAATGCTGTAGACGTGGCTAATTAAAAAAAAAAAA<br>AAAAAAAAAAAAAAAAAAAAAAAAAAAAAAAAAAAAAAAAAAAA | 20 |
| ssODN-TTR_3-50°C-5' | AAAAAAAAAAAAAAAAAAAAAAAAAAAAAAAAAAAAAAAAAAAA<br>AAAAAAAAAAAAAAATGTAGACGTGGCTGTAAAAAAAAAAAAAA<br>AAAAAAAAAAAAAAAAAAAAAAAAAAAAAAAAAAAAAAAAAAAA | 21 |
| ssODN-80% | AACTGTAGACGTGGCTGTTT | 22 |
| ssODM-40% | AAAAAAAGACGTGGAAAATT | 23 |

(continued)

| | | SEQ ID NO |
|---|---|---|
| ssODN-O% | AAAAAATATAAAAAAAAATT | 24 |

<Preparation of Lipid Nanoparticles>

In the following experiments, unless otherwise specified, lipid nanoparticles were prepared by the alcohol dilution method using a flow path. As the flow path, a microfluidic device with a built-in mixer, "iLiNP" (manufactured by Lilac Pharma), was used. Flow rate in the device was controlled using a syringe pump (Harvard Apparatus).

Specifically, lipid nanoparticles containing only nucleic acid were prepared as follows. First, an ethanol solution containing each lipid component at a predetermined molar ratio and adjusted to have a lipid concentration of 8 mM, and an acetate buffer solution (25 mM, pH 4.0) adjusted to have a nucleic acid concentration of 48.9 ng/mL were sent into the micro-flow path at rates of 0.125 mL/min and 0.375 mL/min (total flow rate 0.5 mL/min), respectively, and the lipid nanoparticle solution discharged from the flow path was collected. The lipid nanoparticle solution was applied to a dialysis membrane (MWCO 12,000-14,000) and dialyzed at 4 °C for 2 hours or longer with PBS(-) (pH 7.4) as the external aqueous phase, and then the lipid nanoparticle solution was recovered from the dialysis membrane.

RNP-containing lipid nanoparticles were prepared as follows. First, a solution of Cas protein (10 μM) was added dropwise to an equivalent amount of a sgRNA solution (10 μM) with vigorously mixing to prepare an RNP solution (5 μM). The RNP solution was mixed with an equivalent amount of a ssODN solution (5 μM) to obtain an RNP-ssODN complex (2.5 μM).

Next, an ethanol solution containing each lipid component at a predetermined molar ratio and adjusted to have a lipid concentration of 8 mM, and an RNP-ssODN solution in which the RNP-ssODN complex was diluted to 160 nM with 20 mM MES buffer (pH 6.0, 50 mM NaCl) were sent into the micro-flow path at 0.050 mL/min and 0.450 mL/min (total flow rate 0.5 mL/min), respectively, and the lipid nanoparticle solution discharged from the flow path was collected. The lipid nanoparticle solution was applied to a dialysis membrane (MWCO 12,000-14,000) and dialyzed at 4 °C for 2 hours or longer with PBS(-) (pH 7.4) as the external aqueous phase, and then the lipid nanoparticle solution was recovered from the dialysis membrane. The recovered lipid nanoparticle solution was concentrated by ultrafiltration using a centrifugal filter unit ("Amicon Ultra-15", MWCO 100 kDa, Millipore).

To prepare a fluorescently labeled RNP-containing lipid nanoparticles, an appropriate amount of fluorescent molecules, for example, 0.5 mol % in the case of DiR of the near-infrared fluorescent cyanine dye, was added to the above-described ethanol solution adjusted to have a lipid concentration of 8 mM.

<Measurement of Average Particle Diameter and Zeta Potential of Lipid Nanoparticles>

The mean particle diameter (number average value), ζ-mean particle diameter, and polydispersity index (PdI) of the lipid nanoparticles in PBS(-) (pH 7.4), as well as the zeta potential in 10 mM HEPES buffer (pH 7.4), were measured using an analyzing device, Zetasizer Nano ZS ZEN3600 (Malvern), which utilizes dynamic light scattering.

<Measurement of pKa of Lipid Nanoparticles>

The pKa of the lipid nanoparticles was measured using p-Toluenesulfonic acid (TNS). First, TNS (final concentration: 0.75 μM) and lipid nanoparticles (final concentration: 30 μM) were mixed in buffer solutions (200 mL) adjusted to various pH values (pH 3.5 to 9.5). The buffer solutions used were: 20 mM citrate buffer solution, 20 mM sodium phosphate buffer solution, or 20 mM Tris-HCl buffer solution containing 130 mM NaCl. The fluorescence intensity of the prepared mixture was measured using a spectrofluorometer (VarioskanLux, Thermo Fisher Scientific) at the setting of λex = 321 nm and Aem = 447 nm. The highest and lowest measured values were taken as 100% and 0% charge rate, respectively, and the pH showing a 50% charge rate was calculated as the apparent pKa. The percentage of the contained cationically charged pH-sensitive cationic lipid was calculated using the Henderson-Hasselbalch equation.

<Encapsulation Rate (Encapsulation Efficiency) of Lipid Nanoparticles>

The encapsulation rate of the lipid nanoparticles was calculated by measuring the amount of encapsulated nucleic acid (the amount of ssODN in the case of RNP-ssODN) using an RNA intercalator Ribogreen (manufactured by Life Technologies).

<Metabolic Evaluation of Lipid Nanoparticles (Measurement of Lipid Amount in the Liver)>

For liquid chromatography-mass spectrometry (LC/MS) for lipid quantification, liver samples stored at -80 °C prior to analysis were used. Mice were intravenously injected with lipid nanoparticles (0.5 mg RNA/kg body weight) and then liver tissue was harvested.

The tissue was homogenized in 200 μL of water, and the resulting tissue homogenate (50 μL) was subjected to an extraction with 400 μL of acetonitrile/isopropanol (50:50 (v/v)). The extract was centrifuged (4000 rpm, 10 min), the supernatant was filtered, and the filtrate was diluted 50-fold with water/acetonitrile/isopropanol (1:8:8 (v/v)) and transferred to a vial for LC/MS analysis using an Accucore. LC/MS was performed under the following conditions. The parent lipids and their predicted metabolites were detected under optimized conditions. Each lipid molecule was monitored using the following masses: $[M+H]^+$ -> 916 (CL4H6), 864 (CL4F6), 982 (CL4F10-b-7), 752 (CL4F10-e-2).

(LC/MS Analysis Conditions)
LC/MS device: LTQ-Orbitrap XL (Thermo Scientific)
Column: RP-MS LC column (2.6 μm, 2.1 mm × 50 mm, Thermo Scientific)
Temperature: 40 °C
Mobile phase: Gradient using Solvent A (acetonitrile/water (60:40 (v/v)) containing 10 mM ammonium formate and 0.1% formic acid) and Solvent B (isopropanol/acetonitrile (90:10 (v/v)) containing 10 mM ammonium formate and 0.1% formic acid).
Gradient conditions (concentration of Solvent B): 0 min (50%) -> 2 min (90%) -> 4 min (90%) -> 4.5 min (50%) -> 8 min (50%)
Mode: Positive ion mode
Injection volume: 1 μL
Flow rate: 150 μL/min

<Measurement of Fluc Activity>

First, lipid nanoparticles encapsulating Fluc mRNA (Flue mRNA-loaded lipid nanoparticles) were intravenously injected into BALB/c mice (female, 4 weeks old) at a dose of 0.01 mg mRNA/kg body weight. Six hours after intravenous injection, the mice were euthanized, and each tissue was collected, frozen in liquid nitrogen, and stored at -80 °C. The tissues were homogenized in passive lysis buffer (Promega) using a bead cell disrupter (Micro Smash MS-100R, TOMY Seiko). Tissue debris was removed by centrifugation and the supernatant was collected. Fluc activity in the supernatant was measured using a luciferase assay system (E1500, Promega) according to the manufacturer's protocol. Luminescence was measured using a luminometer (Luminescencer-PSN, ATTO). The protein concentration was determined using a commercially available measurement kit (BCA Protein Assay Kit, Pierce). Fluc activity was expressed as relative light units (RLU) per mg of protein.

<Measurement of TTR-KO Activity>

Lipid nanoparticles encapsulating Cas9/sgTTR-RNP (Cas9/sgTTR-RNP-loaded lipid nanoparticles) were intravenously injected to BALB/c mice (female, 4 weeks old) at the indicated doses. One week after intravenous injection, the mice were euthanized, blood was collected, and serum was isolated by centrifugation. The TTR protein concentration in the serum was determined using a commercially available ELISA kit (Prealbumin ELISA Kit (mouse), Aviva Systems Biology) according to the manufacturer's protocol. The serum TTR protein concentration of untreated mice was taken as 100%, and the relative value (%) of the serum TTR protein concentration of mice administered with each lipid nanoparticle was taken as the knockout activity (TTR-KO activity) of the lipid nanoparticle.

<Measurement of In vitro DNA Cleavage Activity>

Genomic DNA was collected and purified from BALB/c mouse liver tissue using a commercially available nucleic acid extraction kit (NucleoSpin Tissue, MACHEREY-NAGEL) according to the manufacturer's protocol.

The dsDNA from the TTR locus was amplified by PCR (using KAPA HiFi HotStart DNA polymerase (KAPA Biosystems)) using the genomic DNA as a template and the primer set shown in Table 4, and purified using a commercially available nucleic acid purification kit (ISOSPIN PCR Product, Nippon Gene).

[Table 4]

| | | SEQ ID NO |
|---|---|---|
| TTR forward primer | 5' -ACC TTT CAG TGG CTC CAT TC-3' | 25 |
| TTR reverse primer | 5' -TAT GAG CTG CCA TTC TGG TG-3' | 26 |

The ssODN (5 equivalents relative to lipid nanoparticles) was added to Cas9/sgTTR-RNP to form a TTR-RNP-ssODN complex. The TTR-RNP-ssODN complex and the purified PCR product (0.2 equivalent relative to RNP) were mixed in a reaction buffer (50 mM Tris-HCl, 100 mM NaCl, 10 mM $MgCl_2$, 1 mM DTT, pH 7.9) and incubated at 37 °C for 30 min. The reaction solution was then heated at 65 °C for 5 minutes to inactivate Cas9-RNP, and the dsDNA was then subjected to electrophoresis on a 3% agarose gel at 100 V for 40 minutes. After electrophoresis, the gel was stained (GelRed, Biotium) and visualized using a fluorescent/visible light gel imaging system (Printgraph CMOS I, ATTO). Bands were quantified using image analysis software (ImageJ).

DNA cleavage was calculated using the following formula: in the formula, Int_full and Int_cleaved indicate the intensities derived from full length (uncleaved) and cleaved DNA, respectively.

$$[\text{DNA cleavage}] = [\text{Int}_{\text{cleaved}}] / [\text{Int}_{\text{full}}]$$

<Measurement of Tm Value between sgRNA and ssODN by Fluorescence Quenching Method>

To measure the Tm value between sgRNA and ssODN in the RNP complex using the fluorescence quenching method, a FAM-modified sgRNA whose 5' end was modified with the fluorescent substance FAM and a 20-base-long IBFQ-modified ssODN whose 3' end was modified with the quenching substance IBFQ were used.

Specifically, IBFQ-modified ssODN (5 equivalents relative to RNP) was added to Cas9 RNP containing FAM-modified sgRNA (final RNP concentration: 2.5 μM) to form an RNP-ssODN complex. The resulting solution (10 μL) was incubated at 10 °C, after which the temperature was increased by 1 °C at 10 second intervals. The fluorescence intensity was measured at each temperature from 11 to 70 °C using a thermal cycler ("Dice® Real Time System III", TaKaRa Bio Inc.). The temperature at which the fluorescence intensity was maximized was taken as the measured Tm value (°C).

<T7E1 Assay>

Lipid nanoparticles encapsulating TTR-RNP-ssODN complexes (TTR-RNP-ssODN-loaded lipid nanoparticles) were intravenously injected to BALB/c mice (female, 4 weeks old) at 2 mg RNP/kg body weight for two consecutive days. One week after the last injection, the mice were euthanized and liver tissues were harvested, flash frozen in liquid nitrogen, and stored at - 80 °C.

Genomic DNA was extracted and purified from the collected liver tissue using a commercially available nucleic acid extraction kit (NucleoSpin Tissue, MACHEREY-NAGEL) according to the manufacturer's protocol.

The T7E1 assay was performed using a commercially available genome editing detection kit (Alt-R®, Integrated DNA Technologies). The dsDNA treated with T7E1 was subjected to agarose gel electrophoresis in the same manner as in the above described <Measurement of In vitro DNA Cleavage Activity>, and the gel after electrophoresis was stained and the bands were quantified.

<Evaluation of Biodistribution>

For fluorescent labeling of lipid nanoparticles, the RNP-ssODN solution described above was mixed with a three-fold molar amount of a fluorescent molecule (DyLight 650 NHS ester) in DMSO and incubated for 30 min at ambient temperature in the dark. Unreacted fluorescent molecules were removed by ultrafiltration (Amicon Ultra-0.5, MWCO 3 kDa, Millipore).

Lipid nanoparticles encapsulating the obtained fluorescently labeled RNP-ssODN (fluorescently labeled RNP-ssODN-loaded lipid nanoparticles) were intravenously injected into BALB/c mice (female, 4 weeks old) at 2 mg RNP/kg body weight. One hour after intravenous injection, the mice were euthanized and various tissues, including blood, were collected. The collected blood was immediately diluted with 1% sodium dodecyl sulfate (SDS) solution. The collected tissues were immediately weighed and then homogenized in 1% sodium dodecyl sulfate (SDS) solution. The resulting homogenates were transferred to a black 96-well plate, and fluorescence was measured using a microplate reader (VarioskanLux, Thermo Fisher Scientific) at settings of Aex=652 nm and λem=672 nm. Calibration curves were generated

using fluorescently labeled RNP-ssODN-loaded lipid nanoparticles and blood or tissue homogenates similarly prepared from untreated mice. Biodistribution was expressed as the percentage of injected dose per tissue or blood (% injected dose).

<Toxicity Assessment>

TTR-RNP-ssODN-loaded lipid nanoparticles were intravenously injected to BALB/c mice (female, 4 weeks old) at 2 mg RNP/kg body weight for two consecutive days. One week after the last injection, mice were anesthetized, weighed, and blood was collected. Blood was collected from the inferior vena cava and processed to serum. Hematological parameters in serum were measured by Oriental Yeast Co., Ltd.

[Synthesis Example 1] Synthesis of CL4F6[7-(4-(diisopropylamino)butyl)-13-((2-hexylnonanoyl)oxy)-7-hydroxytridecyl 2-hexyldecanoate]

[Chem. 11]

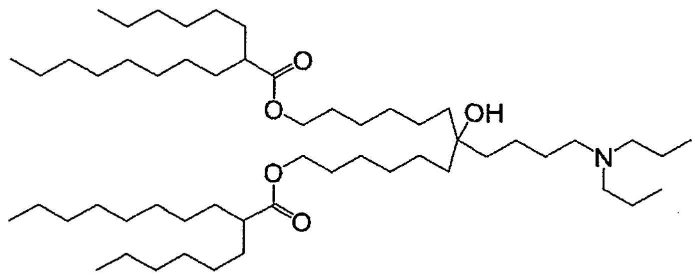

7-(4-(diisopropylamino)butyl)tridecane-1,7,13-triol (1.0 mmol) synthesized by the method described in Patent Literature 1 was dissolved in 5 mL of dichloromethane, and then 2-hexyldecanoic acid (2.20 mmol), DMAP (N,N-dimethyl-4-aminopyridine) (0.20 mmol) and EDCI (1-ethyl-3-(3-dimethylaminopropyl)carbodiimide) (3.0 mmol) were added thereto, and the mixture was reacted at room temperature overnight. The solvent was removed using a rotary evaporator, and the residue was suspended in ethyl acetate and filtered to remove insoluble matter. The filtrate was washed with 0.5N aqueous sodium oxide solution and saturated saline solution. The organic layer was dehydrated by adding anhydrous sodium sulfate. After filtering, the solvent was removed using a rotary evaporator to give a crude product. The crude product was purified by being subjected to silica gel chromatography (eluent: dichloromethane:methanol (continuous gradient)) to give CL4F6.

[Synthesis Example 2] Synthesis of CL4F6-b-3 [7-(4-(dipropylamino)butyl)-7-hydroxy-13-((3-propylhexanoyl)oxy)tridecyl 3-ethylheptanoic acid]

[Chem. 12]

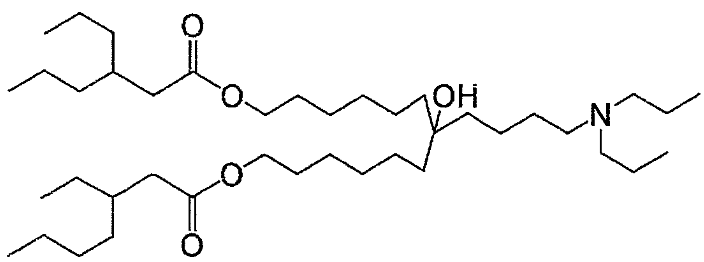

(1) Synthesis of ethyl 3-propylhex-2-enoate

To triethyl phosphonoacetate (2.1 mL, 10.5 mmol) dissolved in THF (tetrahydrofuran, 20 mL) was added NaH

(60% in mineral oil, 0.40 g, 10.0 mmol), and the resulted reaction mixture was stirred on ice for 30 min. 4-Heptanone (0.57 g, 5.0 mmol) was added to the reaction mixture, and the mixture was stirred at 65 °C overnight. The reaction mixture was cooled to room temperature, diluted with EtOAc, then washed with water and brine, and dried over anhydrous $Na_2SO_4$. The dried reaction mixture was filtered and the filtrate was evaporated. This gave ethyl 3-propylhex-2-enoate (1.76 g, 191% yield) as a yellow oil. The crude product was used in the next reaction without further purification.

(2) Synthesis of ethyl 3-propylhexanoate

Ethyl 3-propylhex-2-enoate (1.76 g) was dissolved in a mixed solvent of MeOH (10 mL) and DCM (dichloromethane, 10 mL) under an Ar atmosphere, and then Pd/C (176 mg, 10 wt%) was added. The resulting mixture was hydrogenated under balloon pressure at ambient temperature overnight. The reaction mixture was filtered through a small pad of celite, washed with DCM and the filtrate was evaporated. The resulting residue was loaded onto a normal phase column (Sfaer Silica HC D, Biotage) and purified by flash chromatography (Selekt, Biotage) using a gradient mobile phase of hexane and EtOAc. This gave ethyl 3-propylhexanoate (0.59 g, 63% yield) as a colorless oil.

(3) Synthesis of 3-propylhexanoic acid

Ethyl 3-propylhexanoate (0.59 g) was dissolved in a mixed solvent of 2-propanol (10.7 mL) and water (5.3 mL), and then KOH (1.77 g, 31.5 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 3-propylhexanoic acid (0.46 g, 92% yield) as a colorless oil. The crude product was used in the next reaction without further purification.

(4) Synthesis of CL4F6-b-3

3-Propylhexanoic acid (348 mg, 2.2 mmol) was dissolved in anhydrous DCM (10 mL) and 7-(4-dipropylamino)butyl)tridecane-1,7,13-triol (388 mg, 1.0 mmol), and to the resulting mixture was added DMAP (12.2 mg, 0.10 mmol) and EDCI-HCl (1-(3-dimethylaminopropyl)-3-ethylcarbodiimide hydrochloride) (479 mg, 2.5 mmol), then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F6-b-3 (340 mg, 52% yield) as a colorless oil.

[Synthesis Example 3] Synthesis of CL4F7-b-4 [7-(4-(dipropylamino)butyl)-7-hydroxytridecane-1,13-diylbis(3-butylheptanoate)]

[Chem. 13]

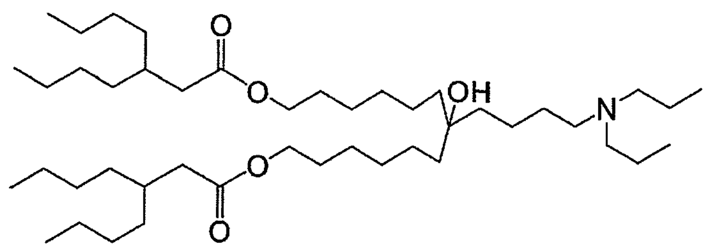

(1) Synthesis of ethyl 3-butylhept-2-enoate

The reaction was carried out in the same manner as in Synthesis example 2 (1) except that 5-nonanone (0.71 g, 5.0 mmol) was used instead of 4-heptanone, to give ethyl 3-butylhept-2-enoate (1.65 g, yield 155%) as a yellow oil. The crude product was used in the next reaction without further purification.

(2) Synthesis of ethyl 3-butylheptanoate

The reaction was carried out in the same manner as in Synthesis example 2 (2) except that ethyl 3-butyllhept-2-enoate (1.65 g) was used instead of ethyl 3-propylhex-2-enoate and that 165 mg (10 wt%) of Pd/C was used, to give ethyl 3-butylheptanoate (0.50 g, yield 47%) as a colorless oil.

(3) Synthesis of 3-butylheptanoic acid

Ethyl 3-butylheptanoate (0.54 g) was dissolved in a mixed solvent of 2-propanol (7.8 mL) and water (3.9 mL), and then KOH (1.29 g, 23 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 3-butylheptanoic acid (0.41 g, 95% yield) as a colorless oil. The crude product was used in the next reaction without further purification.

(4) Synthesis of CL4F7-b-4

In anhydrous DCM (10 mL) and 7-(4-dipropylamino)butyl)tridecane-1,7,13-triol (247 mg, 0.64 mmol), 3-butylheptanoic acid (261 mg, 1.4 mmol) was dissolved, and to the resulting mixture was added DMAP (7.8 mg, 0.064 mmol) and EDCl-HCl (307 mg, 1.6 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F7-b-4 (150 mg, 32% yield) as a colorless oil.

[Synthesis Example 4] Synthesis of CL4F8-b-5 [7-(4-(dipropylamino)butyl)-7-hydroxytridecane-1,13-diylbis(3-pentyloctanoate)]

[Chem. 14]

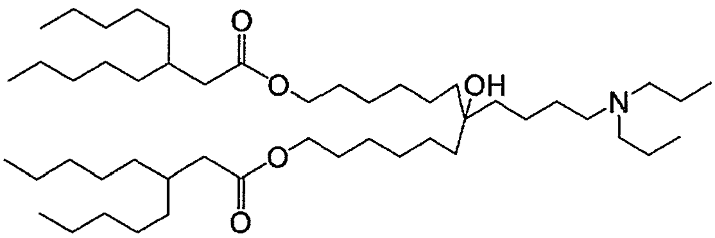

(1) Synthesis of ethyl 3-pentyloct-2-enoate

The reaction was carried out in the same manner as in Synthesis Example 2 (1) except that 6-undecane (0.85 g, 5.0 mmol) was used instead of 4-heptanone, to give ethyl 3-pentyloct-2-enoate (3.94 g, yield 164%) as a yellow oil. The crude product was used in the next reaction without further purification.

(2) Synthesis of ethyl 3-pentyl octanoate

The reaction was carried out in the same manner as in Synthesis Example 2 (2) except that ethyl 3-pentyloct-2-enoate (1.65 g) was used instead of ethyl 3-propylhex-2-enoate, and that 394 mg (10 wt%) of Pd/C was used, to give ethyl 3-pentyloctanoate (1.11 g, yield 46%) as a colorless oil.

(3) Synthesis of 3-pentyloctanoic acid

Ethyl 3-pentyl octanoate (1.11 g) was dissolved in a mixed solvent of 2-propanol (15.6 mL) and water (7.8 mL), and then KOH (2.58 g, 46 mmol) was added, and the reaction mixture was stirred at 70 °C overnight. The reaction mixture was cooled to room temperature and hexane was added, then washed with 1N aqueous HCl, water, and brine, and dried

over anhydrous $Na_2SO_4$. The dried reaction mixture was filtered and the filtrate was evaporated. This gave 3-pentyloctanoic acid (0.94 g, 97% yield) as a colorless oil. The crude product was used in the next reaction without further purification.

(4) Synthesis of CL4F8-b-5

In anhydrous DCM (10 mL) and 7-(4-dipropylamino)butyl)tridecane-1,7,13-triol (379 mg, 0.98 mmol),3-pentyloctanoic acid (461 mg, 2.15 mmol) was dissolved, and to the resulting mixture was added DMAP (11.9 mg, 0.10 mmol) and EDCI-HCl (469 mg, 2.45 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F8-b-5 (320 mg, 42% yield) as a colorless oil.

[Synthesis Example 5] Synthesis of CL4F9-b-6 [7-(4-(dipropylamino)butyl)-7-hydroxytridecane-1,13-diylbis(3-hexylnonanoate)]

[Chem. 15]

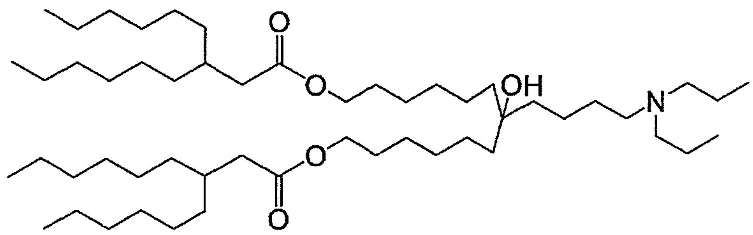

(1) Synthesis of ethyl 3-hexylnon-2-enoate

The reaction was carried out in the same manner as in Synthesis example 2 (1) except that 9-tridecanone (0.99 g, 5.0 mmol) was used instead of 4-heptanone, to give ethyl 3-hexylnon-2-enoate (1.63 g, yield 121%) as a colorless oil. The crude product was used in the next reaction without further purification.

(2) Synthesis of ethyl 3-hexylnonanoate

The reaction was carried out in the same manner as in Synthesis example 2 (2) except that ethyl 3-hexylnon-2-enoate (1.63 g) was used instead of ethyl 3-propylhex-2-enoate, and that 163 mg (10 wt %) of Pd/C was used, to give 3-hexylnonanoate ethyl (0.54 g, yield 40%) as a colorless oil.

(3) Synthesis of 3-hexylnonanoic acid

Ethyl 3-hexylnonanoate (0.54 g) was dissolved in a mixed solvent of 2-propanol (6.8 mL) and water (3.4 mL), and then KOH (1.12 g, 20 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 3-hexylnonanoic acid (0.44 g, 92% yield) as a colorless oil. The crude product was used in the next reaction without further purification.

(4) Synthesis of CL4F9-b-6

In anhydrous DCM (10 mL) and 7-(4-dipropylamino)butyl)tridecane-1,7,13-triol (247 mg, 0.64 mmol), 3-hexylnonanoic acid (339 mg, 1.4 mmol) was dissolved and to the resulting mixture was added DMAP (7.8 mg, 0.064 mmol) and EDCI-HCl (307 mg, 1.6 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded

onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F9-b-6 (250 mg, 46% yield) as a colorless oil.

[Synthesis Example 6] Synthesis of CL4F10-b-7 [7-(4-(dipropylamino)butyl)-7-hydroxytridecane-1,13-diylbis(3-heptyldecanoate)]

[Chem. 16]

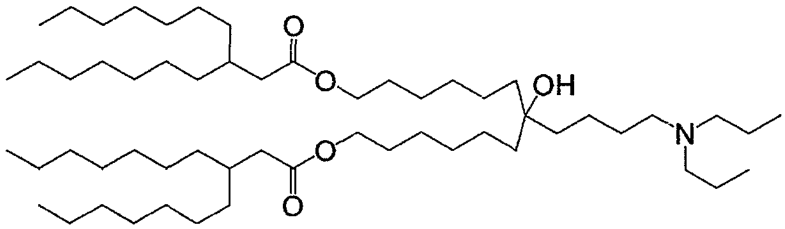

(1) Synthesis of ethyl 3-heptyldec-2-enoate

To triethyl phosphonoacetate (1.20 mL, 6.0 mmol) dissolved in THF (20 mL) was added NaH (60% in mineral oil, 0.22 g, 5.5 mmol), and the resulted reaction mixture was stirred on ice for 3 h. To the reaction mixture, 8-pentadecanone (1.13 g, 5.0 mmol) was added, and then the mixture was refluxed overnight. The reaction mixture was cooled to room temperature, diluted with EtOAc, then washed with water and brine, and dried over anhydrous $Na_2SO_4$. The dried reaction mixture was filtered and the filtrate was evaporated. This gave a mixture of 8-pentadecanone and ethyl 3-heptyldec-2-enoate (1.63 g, 110% yield) as a yellow oil. The crude product was used in the next reaction without further purification.

(2) Synthesis of ethyl 3-heptyldecanoate

The reaction was carried out in the same manner as in Synthesis example 2 (2) except that ethyl 3-heptyldec-2-enoate (1.63 g) was used instead of ethyl 3-propylhex-2-enoate, and that 163 mg (10 wt%) of Pd/C was used, to give a mixture of 8-pentadecanone and ethyl 3-heptyldecanoate (1.16 g, yield 45%) as a colorless oil.

(3) Synthesis of 3-heptyldecanoic acid

A mixture of 8-pentadecanone and ethyl 3-heptyldecanoate (1.16 g) was dissolved in a mixed solvent of 2-propanol (15.3 mL) and water (7.7 mL), and then KOH (1.54 g, 27.4 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave a mixture of 8-pentadecanone and 3-heptyldecanoic acid (1.05 g, 97% yield) as a colorless oil. The crude product was used in the next reaction without further purification.

(4) Synthesis of CL4F10-b-7

A mixture of 8-pentadecanone and 3-heptyldecanoic acid (1.05 g, 2.20 mmol of 3-heptyldecanoic acid) was dissolved in anhydrous DCM (10 mL) and 7-(4-dipropylamino)butyl)tridecane-1,7,13-triol (388 mg, 1.0 mmol), then to the resulting mixture DMAP (12.2 mg, 0.1 mmol) and EDCI-HCl (49 mg, 2.5 mmol) were added, and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F10-b-7 (360 mg, 40% yield) as a colorless oil.

[Synthesis Example 7] Synthesis of CL4F11-b-8 [7-(4-(dipropylamino)butyl)-7-hydroxytridecane-1,13-diylbis(3-octylundecanoate)]

[Chem. 17]

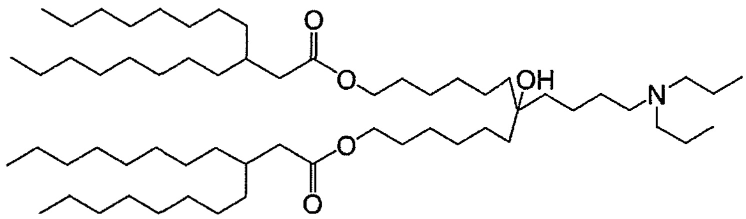

(1) Synthesis of ethyl 3-octylundec-2-enoate

The reaction was carried out in the same manner as in Synthesis Example 2(1) except that 9-heptadecanone (1.27 g, 5.0 mmol) was used instead of 4-heptanone, to give ethyl 3-octylundecan-2-enoate (2.12 g, yield 131%) as a reddish brown oil. The crude product was used in the next reaction without further purification.

(2) Synthesis of ethyl 3-octylundecanoate

The reaction was carried out in the same manner as in Synthesis example 2 (2) except that ethyl 3-octylundec-2-enoate (2.12 g) was used instead of ethyl 3-propylhex-2-enoate, and that 212 mg (10 wt%) of Pd/C was used, to give ethyl 3-octylundecanoate (1.05 g, yield 64%) as a colorless oil.

(3) Synthesis of 3-octylundecanoic acid

Ethyl 3-octylundecanoate (1.05 g) was dissolved in a mixed solvent of 2-propanol (10.8 mL) and water (5.4 mL), and then KOH (1.79 g, 32 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 3-octylundecanoic acid (0.84 g, 88% yield) as a yellow oil. The crude product was used in the next reaction without further purification.

(4) Synthesis of CL4F11-b-8

In anhydrous DCM (10 mL) and 7-(4-dipropylamino)butyl)tridecane-1,7,13-triol (247 mg, 0.64 mmol), 3-octylundecanoic acid (418 mg, 1.4 mmol) was dissolved, and to the resulting mixture was added DMAP (7.8 mg, 0.064 mmol) and EDCI-HCl (307 mg, 1.6 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F11-b-8 (384 mg, 63% yield) as a yellow oil.

[Synthesis Example 8] Synthesis of CL4F9-b [7-(4-(dipropylamino)butyl)-7-hydroxytridecane-1,13-diylbis(2-cycloheptyl acetate)]

[Chem. 18]

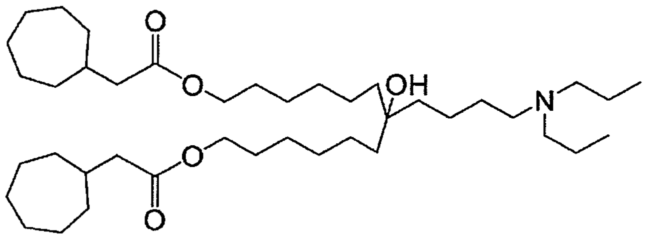

(1) Synthesis of ethyl 2-cycloheptylidene acetate

The reaction was carried out in the same manner as in Synthesis example 2 (1) except that cycloheptanone (0.56 g, 5.0 mmol) was used instead of 4-heptanone, to give ethyl 2-cycloheptylideneacetate (1.72 g, yield 189%) as a colorless oil. The crude product was used in the next reaction without further purification.

(2) Synthesis of ethyl 2-cycloheptyl acetate

The reaction was carried out in the same manner as in Synthesis example 2 (2) except that ethyl 2-cycloheptylidene acetate (1.72 g) was used instead of ethyl 3-propylhex-2-enoate, and that 172 mg (10 wt%) of Pd/C was used, to give 2-cycloheptyl acetate ethyl (0.80 g, yield 87%) as a colorless oil.

(3) Synthesis of 2-cycloheptylacetic acid

Ethyl 2-cycloheptyl acetate (0.80 g) was dissolved in a mixed solvent of 2-propanol (14.8 mL) and water (7.4 mL), and then KOH (2.45 g, 43.7 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 2-cycloheptylacetic acid (0.60 g, 77% yield) as a colorless oil. The crude product was used in the next reaction without further purification.

(4) Synthesis of CL4F9-b

In anhydrous DCM (10 mL) and 7-(4-dipropylamino)butyl)tridecane-1,7,13-triol (670 mg, 1.73 mmol), 2-cycloheptylacetic acid (600 mg, 3.8 mmol) was dissolved, and to the resulting mixture was added DMAP (21 mg, 0.10 mmol) and EDCI-HCl (830 mg, 2.5 mmol) and then stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F9-b (430 mg, 27% yield) as a colorless oil.

[Synthesis Example 9] Synthesis of CL4F14-b [7-(4-(dipropylamino)butyl)-7-hydroxytridecane-1,13-diylbis(2-cyclododecyl acetate)]

[Chem. 19]

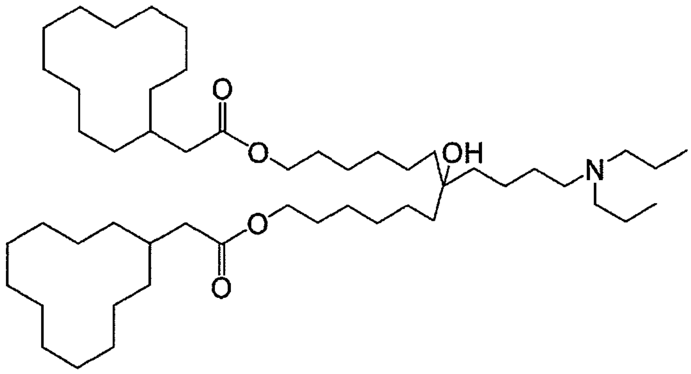

(1) Synthesis of ethyl 2-cyclododecylidene acetate

The reaction was carried out in the same manner as in Synthesis example 2 (1) except that cyclododecanone (0.91 g, 5.0 mmol) was used instead of 4-heptanone, to give ethyl 2-cyclododecylideneacetate (2.0 g, yield 162%) as a yellow oil. The crude product was used in the next reaction without further purification.

(2) Synthesis of ethyl 2-cyclododecyl acetate

The reaction was carried out in the same manner as in Synthesis example 2 (2) except that ethyl 2-cyclododecylidene acetate (2.0 g) was used instead of ethyl 3-propylhex-2-enoate, and that 200 mg (10 wt %) of Pd/C was used, to give 2-cyclododecyl acetate ethyl (1.06 g, yield 84%) as a colorless oil.

(3) Synthesis of 2-cyclododecylacetic acid

Ethyl 2-cyclododecyl acetate (1.06 g) was dissolved in a mixed solvent of 2-propanol (14.1 mL) and water (7.1 mL), and then KOH (2.35 g, 41.8 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 2-cyclododecylacetic acid (0.92 g, 82% yield) as a white solid. The crude product was used in the next reaction without further purification.

(4) Synthesis of CL4F14-b

2-Cyclododecylacetic acid (920 mg, 4.1 mmol) was dissolved in anhydrous DCM (10 mL) and 7-(4-dipropylamino)butyl)tridecane-1,7,13-triol (720 mg, 1.86 mmol), and to the resulting mixture was added DMAP (23 mg, 0.18 mmol) and EDCl-HCl (890 mg, 4.66 mmol) and then stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F14-b (770 mg, 52% yield) as a colorless oil.

[Synthesis Example 10] Synthesis of CL4F17-b [7-(4-(dipropylamino)butyl)-7-hydroxytridecane-1,13-diylbis(2-cyclopentadecyl acetate)]

[Chem. 20]

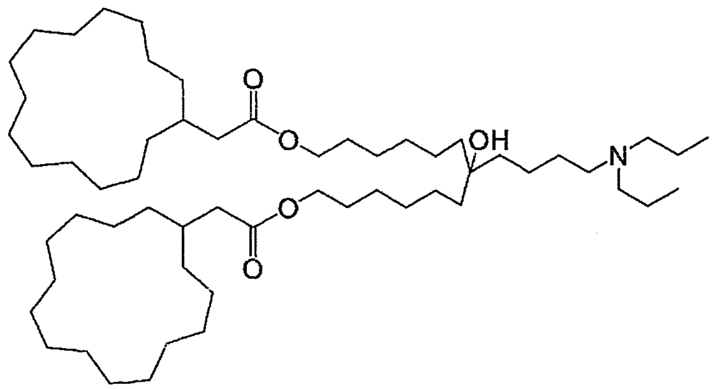

(1) Synthesis of ethyl 2-cyclopentadecylidene acetate

The reaction was carried out in the same manner as in Synthesis example 2 (1) except that cyclopentadecanone (1.12 g, 5.0 mmol) was used instead of 4-heptanone,
to give ethyl 2-cyclopentadecylidene acetate (2.3 g, yield 156%) was obtained as a yellow oil. The crude product was used in the next reaction without further purification.

(2) Synthesis of ethyl 2-cyclopentadecyl acetate

The reaction was carried out in the same manner as in Synthesis example 2 (2) except that ethyl 2-cyclopentadecylidene acetate (2.3 g) was used instead of ethyl 3-propylhex-2-enoate, and that 230 mg (10 wt %) of Pd/C was used, to give ethyl 2-cyclopentadecyl acetate (0.84 g, yield 57%) as a colorless oil.

(3) Synthesis of 2-cyclopentadecylacetic acid

Ethyl 2-cyclopentadecyl acetate (0.84 g) was dissolved in a mixed solvent of 2-propanol (7.5 mL) and water (4.7 mL), and then KOH (1.57 g, 28 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 2-cyclopentadecylacetic acid (0.69 g, 91% yield) as a white solid. The crude product was used in the next reaction without further purification.

(4) Synthesis of CL4F17-b

In anhydrous DCM (10 mL) and 7-(4-dipropylamino)butyl)tridecane-1,7,13-triol (450 mg, 1.16 mmol), 2-cyclopentadecylacetic acid (690 mg, 2.56 mmol) was dissolved, and to the resulting mixture was added DMAP (14.2 mg, 0.12 mmol) and EDCI-HCl (560 mg, 2.9 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This gave CL4F17-b (280 mg, 27% yield) as a colorless oil.

[Synthesis Example 11] Synthesis of CL4F10-e-2 [7-(4-(dipropylamino)butyl)-7-hydroxytridecane-1,13-diylbis(6-ethyldecanoate)]

[Chem. 21]

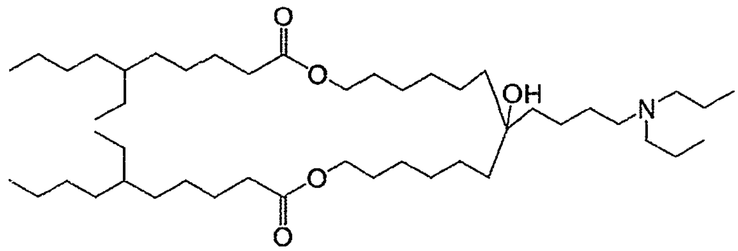

(1) Synthesis of ethyl 6-ethyldec-4-enoate

Under an Ar atmosphere cooled to 0 °C, to triphenylphosphonium bromide (2.74 g, 6.0 mmol) suspended in anhydrous THF (6 mL), 1 M potassium tert-butoxide (THF solution) (6.0 mL) was added dropwise, and the resulted reaction mixture was stirred at room temperature for 30 minutes. Next, to the reaction mixture was added 2-ethylhexanal (0.64 g, 5.0 mmol), and the mixture was stirred at room temperature overnight. The reaction mixture was quenched with saturated $NH_4Cl$ aqueous solution, then diluted with EtOAc, further washed with brine, and dried over anhydrous $Na_2SO_4$. The dried reaction mixture was filtered and the filtrate was evaporated. The residue was dissolved in hexane and passed through silica gel, and the solvent was evaporated. The residue was loaded onto a normal phase column (Sfaer Silica HC D, Biotage) and purified by flash chromatography (Selekt, Biotage) using a gradient mobile phase of hexane and EtOAc. This gave ethyl 6-ethyldec-4-enoate (0.38 g, 34%) as a colorless oil.

(2) Synthesis of ethyl 6-ethyldecanoate

The reaction was carried out in the same manner as in Synthesis example 2 (2) except that ethyl 6-ethyldec-4-enoate (0.38 g, 1.69 mmol) was used instead of ethyl 3-propylhex-2-enoate, and that 38 mg (10 wt%) of Pd/C was used, to give ethyl 6-ethyldecanoate (0.38 g, 99% yield) as a colorless oil. The crude product was used in the next reaction without further purification.

(3) Synthesis of 6-ethyldecanoic acid

Ethyl 6-ethyldecanoate (0.38 g, 1.67 mmol) was dissolved in a mixed solvent of 2-propanol (5.6 mL) and water (2.8 mL), and then KOH (0.94 g, 16.7 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 6-ethyldecanoic acid (0.31 g, 90% yield) as a colorless oil. The crude product was used in the next reaction without further purification.

(4) Synthesis of CL4F10-e-2

In anhydrous DCM (10 mL) and 7-(4-dipropylamino)butyl)tridecane-1,7,13-triol (264 mg, 0.68 mmol), 6-ethyldecanoic acid (305 mg, 1.5 mmol) was dissolved, and to the resulting mixture was added DMAP (8.3 mg, 0.064 mmol) and EDCI-HCl (326 mg, 1.7 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. This residue was applied to a normal phase column (Sfaer Silica HC D, Biotage) and purified by flash chromatography (Slekt, Biotage) using a gradient mobile phase of DCM and MeOH. This gave CL4F10-e-2 (270 mg, 52% yield) as a colorless oil.

[Synthesis Example 12] Synthesis of CL4F1 1-e-3 [7-(4-(dipropylamino)butyl)-7-hydroxytridecane-1,13-diylbis(6-propylundecanoate)]

[Chem. 22]

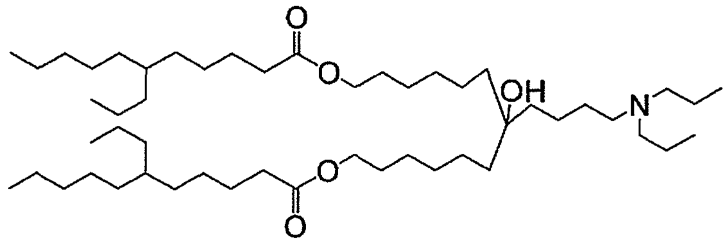

(1) Synthesis of 2-propylheptanal

In anhydrous DCM (30 mL), 2-propylheptanol-1-ol (1.58 g, 10 mmol) was dissolved, and TEMPO (2,2,6,6-tetramethylpiperidine 1-oxyl) (15.5 mg, 0.10 mmol) and $Bu_4NHSO_4$ (0.17 g, 0.50 mmol) were added and the resulting mixture was cooled on ice. Then, to the mixture was added NaOCl $5H_2O$ (1.98 g, 12.0 mmol), and the reaction mixture was stirred on ice for 3 hours. The reaction mixture was quenched with saturated $Na_2S_2$ Os aqueous solution, then diluted with EtOAc, further washed with brine, and dried over anhydrous $Na_2SO_4$. The dried reaction mixture was filtered and the filtrate was evaporated. This gave 2-propylheptanal (1.46 g, 93%) as a colorless oil. The crude product was used in the next reaction without further purification.

(2) Synthesis of 6-butyldodec-4-enoate ethyl

Under an Ar atmosphere cooled to -78 °C, to (4-ethoxy-4-oxobutyl)triphenylphosphonium bromide (5.12 g, 11.2 mmol) suspended in anhydrous THF (8 mL), 1 M potassium tert-butoxide (THF solution) (11.2 mL) was added dropwise, and the resulted reaction mixture was stirred at -78 °C for 30 minutes. Then, to the reaction mixture was added 2-propylheptanal (1.46 g), and the mixture was allowed to warm gradually to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was dissolved in hexane and passed through silica gel, and the solvent was evaporated. The residue was loaded onto a normal phase column (Sfaer Silica HC D, Biotage) and purified by flash chromatography (Selekt, Biotage) using a gradient mobile phase of hexane and EtOAc. This gave ethyl 6-butyldodec-4-enoate (0.98 g, 41%) as a yellow oil.

(3) Synthesis of ethyl 6-butyl dodecanoate

The reaction was carried out in the same manner as in Synthesis example 2 (2) except that ethyl 6-butyldodec-4-enoate (0.98 g, 3.85 mmol) was used instead of ethyl 3-propylhex-2-enoate, and that 98 mg (10 wt%) of Pd/C was used, to give ethyl 6-butyldodecanoate (0.91 g, yield 92%) as a cloudy white oil. The crude product was used in the next reaction without further purification.

(4) Synthesis of 6-butyldodecanoic acid

Ethyl 6-butyldodecanoate (0.91 g, 3.5 mmol) was dissolved in a mixed solvent of 2-propanol (11.8 mL) and water (5.9 mL), and then KOH (1.96 g, 35 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 6-butyldodecanoic acid (0.78 g, 97% yield) as a yellow oil. The crude product was used in the next reaction without further purification.

(5) Synthesis of CL4F11-e-3

In anhydrous DCM (10 mL) and 7-(4-dipropylamino)butyl)tridecane-1,7,13-triol (460 mg, 1.2 mmol), 6-butyldodecanoic acid (692 mg, 2.7 mmol) was dissolved, and to the resulting mixture was added DMAP (14.7 mg, 0.12 mmol) and EDCI-HCl (575 mg, 3.0 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume

ratio)) containing 0.1% TFA. The resulting residue was loaded onto a normal phase column (Sfaer Silica HC D, Biotage) and purified by flash chromatography (Selekt, Biotage) using a gradient mobile phase of DCM and MeOH. This gave CL4F11-e-3 (445 mg, 46% yield) as a colorless oil.

[Synthesis Example 13] Synthesis of CL4F12-e-4 [7-(4-(dipropylamino)butyl)-7-hydroxytridecane-1,13-diylbis(6-butyldodecanoate)]

[Chem. 23]

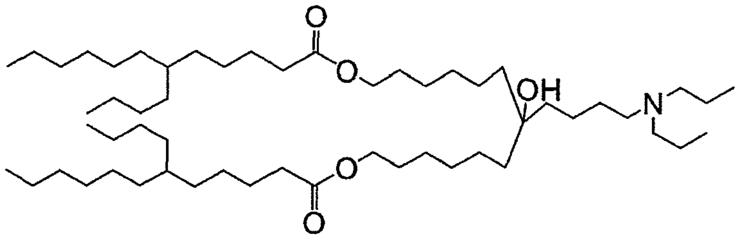

(1) Synthesis of 2-butyloctanal

In anhydrous DCM (30 mL), 2-butyl-1-n-octanol (1.86 g, 10 mmol) was dissolved, and TEMPO (15.5 mg, 0.10 mmol) and $Bu_4NHSO_4$ (0.17 g, 0.50 mmol) were added, and the resulting mixture was cooled on ice. Then, to the mixture was added NaOCl $5H_2O$ (1.98 g, 12.0 mmol), and the reaction mixture was stirred on ice for 3 hours. The reaction mixture was quenched with saturated aqueous $Na_2S_2O_3$ solution, then diluted with EtOAc, further washed with water and brine, and dried over anhydrous $Na_2SO_4$. The dried reaction mixture was filtered and the filtrate was evaporated. The residue was loaded onto a normal phase column (Sfaer Silica HC D, Biotage) and purified by flash chromatography (Selekt, Biotage) using a gradient mobile phase of hexane and EtOAc. This gave 2-butyloctanal (1.86 g, 101%) as a colorless oil.

(2) Synthesis of ethyl 6-hexyldodec-4-enoate

Under an Ar atmosphere cooled to 0 °C, to (4-ethoxy-4-oxobutyl)triphenylphosphonium bromide (5.49 g, 12.0 mmol) suspended in anhydrous THF (12 mL), 1 M potassium tert-butoxide (THF solution) (12.0 mL) was added dropwise, and the resulted reaction mixture was stirred at room temperature for 30 minutes. Then, to the reaction mixture was added 2-butyloctanal (1.86 g, 10.0 mmol) and the reaction mixture was stirred at room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was dissolved in hexane and passed through silica gel, and the solvent was evaporated. The residue was loaded onto a normal phase column (Sfaer Silica HC D, Biotage) and purified by flash chromatography (Selekt, Biotage) using a gradient mobile phase of hexane and EtOAc. This gave ethyl 6-butyldodec-4-enoate (1.75 g) as a colorless oil.

(3) Synthesis of ethyl 6-butyl dodecanoate

The reaction was carried out in the same manner as in Synthesis example 2 (2) except that ethyl 6-butyldodec-4-enoate (1.75 g) was used instead of ethyl 3-propylhex-2-enoate, and that 175 mg (10 wt%) of Pd/C was used, to give 6-butyldodecanoate ethyl (1.75 g) as a cloudy white oil. The crude product was used in the next reaction without further purification.

(4) Synthesis of 6-butyldodecanoic acid

Ethyl 6-butyldodecanoate (1.75 g) was dissolved in a mixed solvent of 2-propanol (4.0 mL) and water (2.0 mL), and then KOH (0.67 g, 12 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 6-butyldodecanoic acid (1.59 g) as a yellow oil. The crude product was used in the next reaction without further purification.

(5) Synthesis of CL4F12-e-4

In anhydrous DCM (10 mL) and 7-(4-dipropylamino)butyl)tridecane-1,7,13-triol (210 mg, 0.55 mmol), 6-butyldodecanoic acid (1.59 g) was dissolved, and to the resulting mixture was added DMAP (6.7 mg, 0.055 mmol) and EDCI-HCl (260 mg, 1.38 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. The resulting residue was loaded onto a normal phase column (Sfaer Silica HC D, Biotage) and purified by flash chromatography (Selekt, Biotage) using a gradient mobile phase of DCM and MeOH. This gave CL4F12-e-4 (85 mg, 18% yield) as a colorless oil.

[Synthesis Example 14] Synthesis of CL4F12-e-6 [7-(4-(dipropylamino)butyl)-7-hydroxytridecane-1,13-diylbis(6-hexyldodecanoate)]

[Chem. 24]

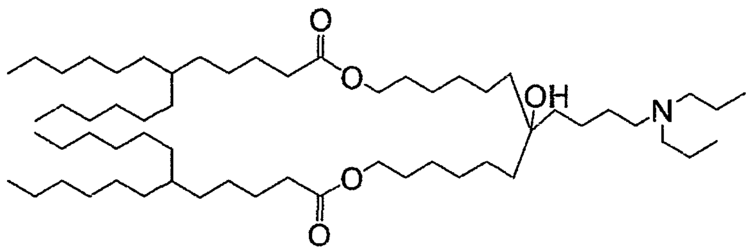

(1) Synthesis of 2-hexyloctanal

The reaction was carried out in the same manner as in Synthesis example 13 (1) except that 2-hexyl-1-n-octanol (2.14 g, 10 mmol) was used instead of 2-butyl-1-n-octanol, to give 2-hexyloctanal (1.81 g, 85%) as a colorless oil.

(2) Synthesis of ethyl 6-hexyldodec-4-enoate

Under an Ar atmosphere cooled to -78 °C, to (4-ethoxy-4-oxobutyl)triphenylphosphonium bromide (4.67 g, 10.2 mmol) suspended in anhydrous THF (8 mL), 1 M potassium tert-butoxide (THF solution) (10.2 mL) was added dropwise, and the resulted reaction mixture was stirred at -78 °C for 30 minutes. Then, to the reaction mixture was added 2-hexyloctanal (1.81 g), and the reaction mixture was allowed to warm gradually to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was dissolved in hexane and passed through silica gel, and the solvent was evaporated. The residue was loaded onto a normal phase column (Sfaer Silica HC D, Biotage) and purified by flash chromatography (Selekt, Biotage) using a gradient mobile phase of hexane and EtOAc. This gave ethyl 6-hexyldodec-4-enoate (1.93 g, 73% yield) as a colorless oil.

(3) Synthesis of ethyl 6-hexyldodecanoate

The reaction was carried out in the same manner as in Synthesis example 2 (2) except that ethyl 6-hexyldodec-4-enoate (1.93 g, 6.2 mmol) was used instead of ethyl 3-propylhex-2-enoate, and that 193 mg (10 wt%) of Pd/C was used, to

give 6-hexyldodecanoate ethyl (1.75 g) as a cloudy white oil. The crude product was used in the next reaction without further purification.

(4) Synthesis of 6-hexyldodecanoic acid

Ethyl 6-hexyldodecanoate (1.95 g, 6.2 mmol) was dissolved in a mixed solvent of 2-propanol (21 mL) and water (10.5 mL), and then KOH (3.48 g, 62 mmol) was added, and the reaction mixture was stirred at 70 °C overnight. The reaction mixture was cooled to room temperature and hexane was added, then washed with 1N aqueous HCl, water, and brine, and dried over anhydrous $Na_2SO_4$. The dried reaction mixture was filtered and the filtrate was evaporated. This gave 6-hexyldodecanoic acid (1.73 g, 98% yield) as a yellow oil. The crude product was used in the next reaction without further purification.

(5) Synthesis of CL4F12-e-6

In anhydrous DCM (10 mL) and 7-(4-dipropylamino)butyl)tridecane-1,7,13-triol (757 mg, 1.95 mmol), 6-hexyldodecanoic acid (1.2 g, 4.2 mmol) was dissolved, and to the resulting mixture was added DMAP (24.4 mg, 0.2 mmol) and EDCl-HCl (930 mg, 4.9 mmol), and then the mixture was stirred at ambient temperature overnight. The reaction mixture was then evaporated and the residue was suspended in EtOAc, washed with 0.5 N aqueous NaOH and brine, and dried over anhydrous $Na_2SO_4$. The residue was loaded onto a reversed-phase column (HP C18Aq, Teledyne ISCO) and purified by flash chromatography (CombiFlash Rf, Teledyne ISCO) using a gradient mobile phase of 0.1% TFA aqueous solution and acetonitrile/2-propanol (1:1 (volume ratio)) containing 0.1% TFA. The resulting residue was loaded onto a normal phase column (Sfaer Silica HC D, Biotage) and purified by flash chromatography (Selekt, Biotage) using a gradient mobile phase of DCM and MeOH. This gave CL4F12-e-6 (320 mg, 18% yield) as a yellow oil.

[Synthesis Example 15] Synthesis of CL4F1 1-ζ-2 [7-(4-(dipropylamino)butyl)-7-hydroxytridecane-1,13-diylbis(7-ethylundecanoate)]

[Chem. 25]

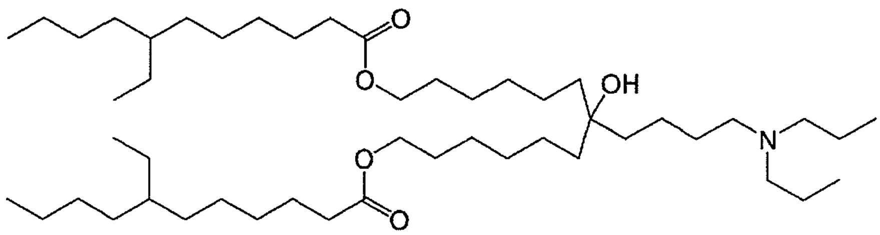

(1) Synthesis of 7-ethylundec-5-enoic acid

Under an Ar atmosphere cooled to -78 °C, 1M potassium tert-butoxide (THF solution) (12 mL) was added dropwise to 4-(carboxybutyl)triphenylphosphonium bromide (2.66 g, 6.0 mmol) suspended in anhydrous THF (6 mL), and the resulted reaction mixture was stirred at -78 °C for 30 minutes. To the reaction mixture was then added 2-ethylhexanal (0.64 g, 5.0 mmol), and the reaction mixture was allowed to gradually warm to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. This gave ethyl undec-5-enoate (0.69 g, 65% yield) as a colorless oil. The crude product was used in the next reaction without further purification.

(2) Synthesis of 7-ethylundecanoic acid

Under an Ar atmosphere, ethyl undec-5-enoate (0.69 g, 3.2 mmol) was dissolved in a mixed solvent of MeOH (10 mL) and DCM (10 mL), and then Pd/C (69 mg, 10 wt%) was added. The resulting mixture was hydrogenated under balloon pressure at ambient temperature overnight. The reaction mixture was filtered through a small pad of celite, washed with DCM and the filtrate was evaporated. This gave 7-ethylundecanoic acid (0.69 g, 100% yield) as a cloudy white oil. The crude product was used in the next reaction without further purification.

(3) Synthesis of CL4F11-ζ-2

7-Ethylundecanoic acid (0.69 g, 3.2 mmol) was dissolved in a mixture of anhydrous DCM (10 mL), 7-(4-dipropylamino)butyl)tridecane-1,7,13-triol (562 mg, 1.45 mmol) and EDCl-HCl (690 mg, 3.6 mmol), and the reaction mixture was stirred at ambient temperature overnight. The reaction mixture was then evaporated and the residue was suspended in EtOAc, washed with 0.5 N aqueous NaOH and brine, and dried over anhydrous $Na_2SO_4$. The dried reaction mixture was filtered and the filtrate was evaporated. The residue was loaded onto a reversed-phase column (Sfaer C18, Biotage) and purified by flash chromatography (Slekt, Biotage) using a gradient mobile phase of 0.1% TFA aqueous solution and a mixed solvent of acetonitrile/2-propanol (1:1 (volume ratio)) containing 0.1% TFA. The residue was applied to a normal phase column (Sfaer Silica HC D, Biotage) and purified by flash chromatography (Slekt, Biotage) using a gradient mobile phase of DCM and MeOH. This gave CL4F 11-ζ-2 (472 mg, 42% yield) as a colorless oil.

[Synthesis Example 16] Synthesis of CL4F12-η-2 [7-(4-(dipropylamino)butyl)-7-hydroxytridecane-1,13-diylbis(8-ethyldodecanoate)]

[Chem. 26]

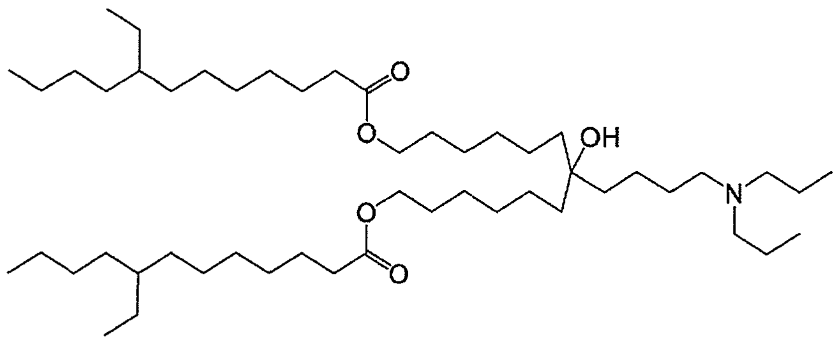

(1) Synthesis of 8-ethyldodec-6-enoic acid

The reaction was carried out in the same manner as in Synthesis example 15 (1) except that 5-(carboxypentyl) triphenylphosphonium bromide (2.74 g, 6.0 mmol) was used instead of 4-(carboxybutyl)triphenylphosphonium bromide (2.66 g, 6.0 mmol), to give 8-ethyldodec-6-enoic acid (0.85 g, yield 75%) as a colorless oil. The crude product was used in the next reaction without further purification.

(2) Synthesis of 8-ethyldodecanoic acid

The reaction was carried out in the same manner as in Synthesis example 15 (2) except that ethyldodec-6-enoic acid (0.85 g, 3.7 mmol) was used instead of ethylundec-5-enoic acid (0.69 g, 3.2 mmol), and that Pd/C (85 mg, 10 wt%) was used instead of Pd/C (69 mg, 10 wt%), to give 8-ethyldodecanoic acid (0.86 g, yield 102%) as a cloudy white oil. The crude product was used in the next reaction without further purification.

(3) Synthesis of CL4F12-η-2

The reaction was carried out in the same manner as in Synthesis example 15 (3) except that 8-ethyldodecanoic acid (0.55 g, 2.4 mmol) was used instead of 7-ethylundecanoic acid (0.69 g, 3.2 mmol), and that a mixture of anhydrous DCM (10 mL), 7-(4-dipropylamino)butyl)tridecane-1,7,13-triol (430 mg, 1.1 mmol), DMAP (13 mg, 0.11 mmol) and EDCl-HCl (527 mg, 2.75 mmol) was used instead of a mixture of anhydrous DCM (10 mL), 7-(4-dipropylamino)butyl) tridecane-1,7,13-triol (562 mg, 1.45 mmol) and EDCl-HCl (690 mg, 3.6 mmol), to give CL4F12-η-2 (202 mg, yield 23%) as a yellow oil.

[Synthesis Example 17] Synthesis of CL4F11-η-3(3) [7-(4-(dipropylamino)butyl)-7-hydroxytridecane-1,13-d iy l bis(8,10,10-trimethylundecanoate)]

[Chem. 27]

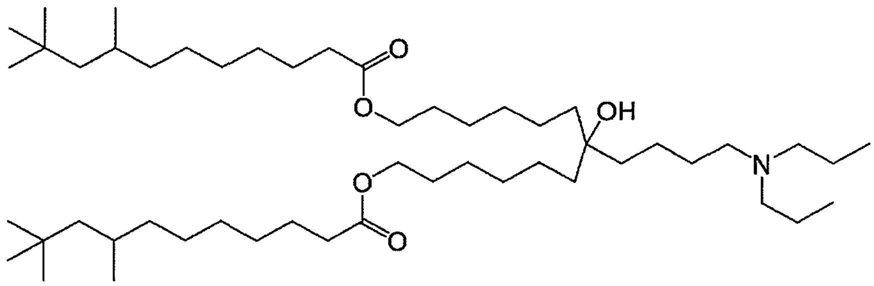

(1) Synthesis of 3,5,5-trimethylhexanal

3,5,5-Trimethylhexan-1-ol (1.44 g, 10 mmol) was dissolved in anhydrous DCM (30 mL), TEMPO (15.5 mg, 0.10 mmol), and $Bu_4NHSO_4$ (0.17 g, 0.50 mmol) were added, and the resulting mixture was cooled on ice. Then, to the mixture was added NaOCl $5H_2O$ (1.98 g, 12.0 mmol), and the reaction mixture was stirred on ice for 3 hours. The reaction mixture was quenched with saturated $Na_2S_2$ $O_3$ aqueous solution, then diluted with EtOAc, further washed with brine, and dried over anhydrous $Na_2SO_4$. The dried reaction mixture was filtered and the filtrate was evaporated. This gave 3,5,5-trimethylhexanal (0.91 g, 64% yield) as a yellow oil. The crude product was used in the next reaction without further purification.

(2) Synthesis of 8,10,10-trimethylundec-5-enoic acid

Under an Ar atmosphere cooled to -78 °C, 1M potassium tert-butoxide (THF solution) (12 mL) was added dropwise to 5-(carboxybutyl)triphenylphosphonium bromide (2.74 g, 6.0 mmol) suspended in anhydrous THF (6 mL), and the resulted reaction mixture was stirred at -78 °C for 30 minutes. 3,5,5-trimethylhexanal (0.91 g) was then added to the reaction mixture and the reaction mixture was allowed to warm gradually to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was loaded onto a reversed-phase column (Sfaer C18, Biotage) and purified by flash chromatography (Slekt, Biotage) using a gradient mobile phase of 0.1% TFA aqueous solution and acetonitrile containing 0.1% TFA. This gave 8,10,10-trimethylundec-5-enoic acid (0.42 g, 37% yield) as a colorless oil.

(3) Synthesis of 8,10,10-trimethylundecanoic acid

The reaction was carried out in the same manner as in Synthesis example 15 (2) except that 8,10,10-trimethylundec-5-enoic acid (0.42 g, 1.9 mmol) was used instead of ethylundec-5-enoic acid (0.69 g, 3.2 mmol), and that Pd/C (42 mg, 10 wt%) was used instead of Pd/C (69 mg, 10 wt%), to give 8,10,10-trimethylundecanoic acid (0.42 g, yield 99%) as a cloudy white oil. The crude product was used in the next reaction without further purification.

(4) Synthesis of CL4F11-η-3(3)

The reaction was carried out in the same manner as in Synthesis example 15 (3) except that 8,10,10-trimethylundecanoic acid (0.42 g, 1,8 mmol) was used instead of 7-ethylundecanoic acid (0.69 g, 3.2 mmol), and that a mixture of anhydrous DCM (10 mL), 7-(4-dipropylamino)butyl)tridecane-1,7,13-triol (318 mg, 0.8 mmol), DMAP (10 mg, 0.1 mmol) and EDCl-HCl (383 mg, 2.0 mmol) was used instead of a mixture of anhydrous DCM (10 mL), 7-(4-dipropylamino)butyl)tridecane-1,7,13-triol (562 mg, 1.45 mmol) and EDCl-HCl (690 mg, 3.6 mmol), to give CL4F11-η-3(3) (251 mg, yield 38%) as a colorless oil.

[Synthesis Example 18] Synthesis of CL4F10-ζ-3(3) [7-(4-(dipropylamino)butyl)-7-hydroxytridecane-1,13-diyl-bis(7,9,9-trimethyldecanoate)]

[Chem. 28]

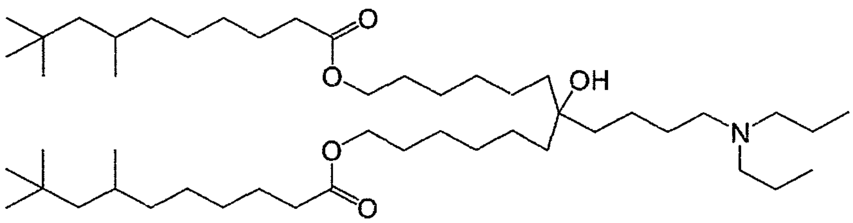

(1) Synthesis of 3,5,5-trimethylhexanal

The reaction was carried out in the same manner as in Synthesis Example 17(1) to obtain 3,5,5-trimethylhexanal (1.18 g, yield 83%) as a yellow oil. The crude product was used in the next reaction without further purification.

(2) Synthesis of 7,9,9-trimethyldec-4-enoic acid

Under an Ar atmosphere cooled to -78 °C, 1M potassium tert-butoxide (THF solution) (20 mL) was added dropwise to 3-(carboxypropyl)triphenylphosphonium (4.3 g, 10.0 mmol) suspended in anhydrous THF (6 mL), and the resulted reaction mixture was stirred at -78 °C for 30 minutes. 3,5,5-trimethylhexanal (1.18 g) was then added to the reaction mixture and the reaction mixture was allowed to warm gradually to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was loaded onto a reversed-phase column (Sfaer C18, Biotage) and purified by flash chromatography (Slekt, Biotage) using a gradient mobile phase of 0.1% TFA aqueous solution and acetonitrile containing 0.1% TFA. This gave 7,9,9-trimethyldec-4-enoic acid (0.51 g, 29% yield) as a yellow oil.

(3) Synthesis of 7,9,9-trimethyldecanoic acid

The reaction was carried out in the same manner as in Synthesis example 15 (2) except that 7,9,9-trimethyldec-4-enoic acid (0.51 g, 2.4 mmol) was used instead of ethylundec-5-enoic acid (0.69 g, 3.2 mmol), and that Pd/C (51 mg, 10 wt%) was used instead of Pd/C (69 mg, 10 wt%), to give 7,9,9-trimethyldecanoic acid (0.51 g, yield 99%) as a cloudy white oil. The crude product was used in the next reaction without further purification.

(4) Synthesis of CL4F10-ζ-3(3)

The reaction was carried out in the same manner as in Synthesis example 15 (3) except that 7,9,9-trimethyldecanoic acid (0.51 g, 2.4 mmol) was used instead of 7-ethylundecanoic acid (0.69 g, 3.2 mmol), and that a mixture of anhydrous DCM (10 mL), 7-(4-dipropylamino)butyl)tridecane-1,7,13-triol (426 mg, 1.1 mmol), DMAP (13 mg, 0.1 mmol) and EDCl-HCl (518 mg, 2.7 mmol) was used instead of a mixture of anhydrous DCM (10 mL), 7-(4-dipropylamino)butyl) tridecane-1,7,13-triol (562 mg, 1.45 mmol) and EDCl-HCl (690 mg, 3.6 mmol), to give CL4F10-ζ-3(3) (75 mg, yield 9%) as a yellow oil.

[Synthesis Example 19] Synthesis of CL4F12-θ-3(3) [7-(4-(dipropylamino)butyl)-7-hydroxytridecane-1,13-diyl-bis(9,11,11-trimethyldodecanoate)]

[Chem. 29]

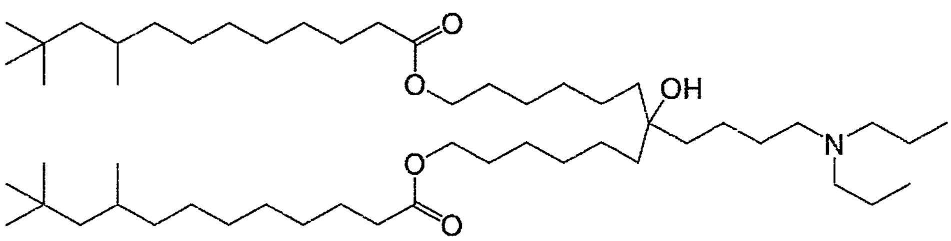

(1) Synthesis of 3,5,5-trimethylhexanal

The reaction was carried out in the same manner as in Synthesis Example 17(1) to give 3,5,5-trimethylhexanal (1.37 g, yield 94%) as a yellow oil. The crude product was used in the next reaction without further purification.

(2) Synthesis of 9,11,11-trimethyldodec-6-enoic acid

Under an Ar atmosphere cooled to -78 °C, 1M potassium tert-butoxide (THF solution) (23 mL) was added dropwise to 5-(carboxypentyl)triphenylphosphonium bromide (5.2 g, 11.3 mmol) suspended in anhydrous THF (6 mL), and the resulted reaction mixture was stirred at -78 °C for 30 minutes. Then to the reaction mixture was added 3,5,5-trimethylhexanal (1.37 g), and the reaction mixture was allowed to warm gradually to room temperature overnight. The reaction mixture was quenched with saturated aqueous $NH_4Cl$, then diluted with $Et_2O$, further washed with brine, and dried over anhydrous $Na_2SO_4$. The dried reaction mixture was filtered and the filtrate was evaporated. The residue was loaded onto a reversed-phase column (Sfaer C18, Biotage) and purified by flash chromatography (Slekt, Biotage) using a gradient mobile phase of 0.1% TFA aqueous solution and acetonitrile containing 0.1% TFA. This gave 9,11,11-trimethyldodec-6-enoic acid (0.47 g, 21% yield) as a colorless oil.

(3) Synthesis of 9,11,11-trimethyldodecanoic acid

The reaction was carried out in the same manner as in Synthesis example 15 (2) except that 9,11,11-trimethyldodec-6-enoic acid (0.47 g, 2.0 mmol) was used instead of ethylundec-5-enoic acid (0.69 g, 3.2 mmol), and that Pd/C (47 mg, 10 wt%) was used instead of Pd/C (69 mg, 10 wt%), to give 9,11,11-trimethyldodecanoic acid (0.47 g, yield 96%) as a cloudy white oil. The crude product was used in the next reaction without further purification.

(4) Synthesis of CL4F12-θ-3(3)

The reaction was carried out in the same manner as in Synthesis example 15 (3) except that 9,11,11-trimethyldodecanoic acid (0.47 g, 1.9 mmol) was used instead of 7-ethylundecanoic acid (0.69 g, 3.2 mmol), and that a mixture of anhydrous DCM (10 mL), 7-(4-dipropylamino)butyl)tridecane-1,7,13-triol (333 mg, 0.9 mmol), DMAP (11 mg, 0.1 mmol) and EDCI-HCl (414 mg, 2.2 mmol) was used instead of a mixture of anhydrous DCM (10 mL), 7-(4-dipropylamino)butyl)tridecane-1,7,13-triol (562 mg, 1.45 mmoi), and EDCI-HCl (690 mg, 3.6 mmol), to give CL4F12-8-3 (3) (233 mg, 32% yield) as a yellow oil.

[Synthesis Example 20] Synthesis of CL4F12-ζ-2(2) [7-(4-(dipropylamino)butyl)-7-hydroxytridecane-1,13-diylbis(7,11-dimethyldodecanoate)]

[Chem. 30]

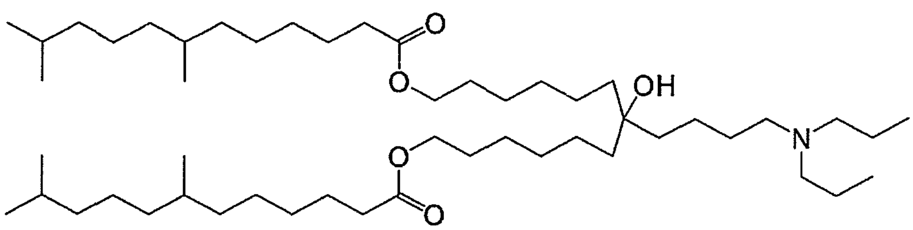

(1) Synthesis of 3,7-dimethyloctanal

The reaction was carried out in the same manner as in Synthesis example 17 (1) except that 3,7-dimethyloctan-1-ol (1.58 g, 10.0 mmol) was used instead of 3,5,5-trimethylhexan-1-ol (1.44 g, 10 mmol), to give 3,7-dimethyloctanal (1.59 g, yield 102%) as a colorless oil. The crude product was used in the next reaction without further purification.

(2) Synthesis of 7,11-dimethyldodec-4-enoic acid

The reaction was carried out in the same manner as in Synthesis example 17 (2) except that 3-(carboxypropyl)triphenylphosphonium bromide (5.2 g, 12.0 mmol) was used instead of 5-(carboxypentyl)triphenylphosphonium bromide (5.2 g, 11.3 mmol) and that 3,7-dimethyloctanal (1.59 g) was used instead of 3,5,5-trimethylhexanal (1.37 g), to give 7,1 1-dimethyldodec-4-enoic acid (0.90 g, yield 40%) as a colorless oil.

(3) Synthesis of 7,1 1-dimethyldodecanoic acid

The reaction was carried out in the same manner as in Synthesis example 15 (2) except that 7,11-dimethyldodec-4-enoic acid (0.90 g, 4.0 mmol) was used instead of ethylundec-5-enoic acid (0.69 g, 3.2 mmol), and that Pd/C (90 mg, 10 wt%) was used instead of Pd/C (69 mg, 10 wt%), to give 7,11-dimethyldodecanoic acid (0.90 g, yield 99%) as a cloudy white oil. The crude product was used in the next reaction without further purification.

(4) Synthesis of CL4F12-ζ-2(2)

The reaction was carried out in the same manner as in Synthesis example 15 (3) except that 7,11-dimethyldodecanoic acid (0.90 g, 4.0 mmol) was used instead of 7-ethylundecanoic acid (0.69 g, 3.2 mmol), and a mixture of anhydrous DCM (10 mL), 7-(4-dipropylamino)butyl)tridecane-1,7,13-triol (698 mg, 1.8 mmol), DMAP (22 mg, 0.2 mmol) and EDCI-HCl (861 mg, 4.5 mmol) was used instead of a mixture of anhydrous DCM (10 mL), 7-(4-dipropylamino)butyl) tridecane-1,7,13-triol (562 mg, 1.45 mmol) and EDCI-HCl (690 mg, 3.6 mmol), to give CL4F12-ζ-2(2) (306 mg, 21% yield) as a yellow oil.

[Synthesis Example 21] Synthesis of CL4F13-η-2(2) [7-(4-(dipropylamino)butyl)-7-hydroxytridecane-1,13-diylbis(8,12-dimethyltridecanoate)]

[Chem. 31]

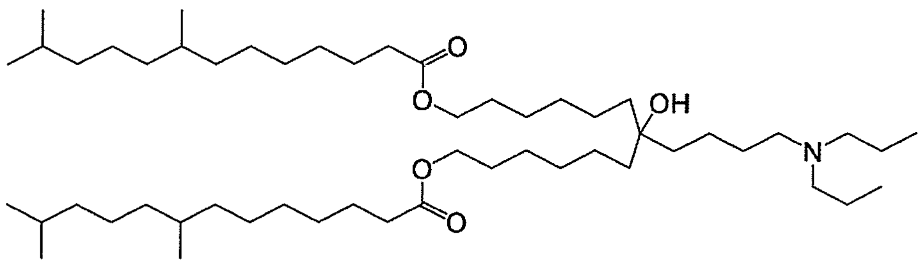

(1) Synthesis of 3,7-dimethyloctanal

The reaction was carried out in the same manner as in Synthesis Example 19(1) to give 3,7-dimethyloctanal (1.63 g, yield 104%) as a colorless oil. The crude product was used in the next reaction without further purification.

(2) Synthesis of 8,12-dimethyltridecanoic acid

The reaction was carried out in the same manner as in Synthesis example 17 (2) except that 4-(carboxybutyl) triphenylphosphonium bromide (5.3 g, 12.0 mmol) was used instead of 5-(carboxypentyl)triphenylphosphonium bromide (5.2 g, 11.3 mmol), and that 3,7-dimethyloctanal (1.63 g) was used instead of 3,5,5-trimethylhexanal (1.37 g), to give 8,12-dimethyltridecanoic acid (0.77 g, yield 32%) as a colorless oil.

(3) Synthesis of 8,12-dimethyltridecanoic acid

The reaction was carried out in the same manner as in Synthesis example 15 (3) except that 7,11-dimethyldodec-4-enoic acid (0.90 g, 4.0 mmol) was used instead of ethylundec-5-enoic acid (0.69 g, 3.2 mmol), and that Pd/C (90 mg, 10 wt%) was used instead of Pd/C (69 mg, 10 wt%), to give 8,12-dimethyltridecanoic acid (0.76 g, yield 98%) as a cloudy white oil. The crude product was used in the next reaction without further purification.

(4) Synthesis of CL4F13-η-2(2)

The reaction was carried out in the same manner as in Synthesis example 15 (3) except that 8,12-dimethyltridecanoic acid (0.76 g, 3.1 mmol) was used instead of 7-ethylundecanoic acid (0.69 g, 3.2 mmol), and that a mixture of anhydrous DCM (10 mL), 7-(4-dipropylamino)butyl)tridecane-1,7,13-triol (553 mg, 1.4 mmol), DMAP (17 mg, 0.1 mmol) and EDCl-HCl (684 mg, 3.1 mmol) was used instead of a mixture of anhydrous DCM (10 mL), 7-(4-dipropylamino)butyl) tridecane-1,7,13-triol (562 mg, 1.45 mmol) and EDCl-HCl (690 mg, 3.6 mmol), to give CL4F13-η-2(2) (558 mg, 47% yield) as a yellow oil.

[Example 1]

Lipid nanoparticles were prepared using the lipids synthesized in Synthesis Examples 1 to 14 as constituent lipids.

In addition, 7-(4-(diisopropylamino)butyl)-7-hydroxytridecane-1,13-diyl dioleate (CL4H6) was also used as a constituent lipid. CL4H6 used was synthesized by the method described in Patent Literature 1.

As neutral lipids, DSPC, cholesterol (chol), and PEG-DMG were used.

Specifically, lipid nanoparticles loaded with Fluc mRNA (Flue mRNA-loaded lipid nanoparticles) were prepared by the alcohol dilution method using a pH-sensitive cationic lipid, DSPC, cholesterol, and PEG-DMG in a molar ratio of 50/10/40/1.5 (mol%). The type of pH-sensitive cationic lipid used in the preparation, the encapsulation rate (%), ζ-average particle diameter (nm), number-average particle diameter (nm), PdI, ζ-potential (mV), and pKa of the prepared lipid nanoparticles were measured.

Table 5 shows the results of lipid nanoparticles containing pH-sensitive cationic lipids in which, in the general formula (A), c is 1 (pH-sensitive cationic lipids branched at the β position) among the pH-sensitive cationic lipids of the present invention.

[Table 5]

| pH-sensitive cationic lipid | Encapsulation rate (%) | ζ-average (nm) | PdI | Number-average particle diameter (nm) | ζ-potential (mV) | pKa |
|---|---|---|---|---|---|---|
| CL4F6 | 88.4 | 131.2 | 0.202 | 76.5 | -3.40 | 6.20 |
| CL4F6-b-3 | 88.9 | 141.4 | 0. 144 | 89.3 | -3. 44 | 6.55 |
| CL4F7-b-4 | 77.5 | 237.6 | 0.267 | 94.4 | -3.06 | 6.40 |
| CL4F8-b-5 | 91.0 | 106.3 | 0.185 | 68.7 | -5.16 | 6.25 |
| CL4F9-b-6 | 91.4 | 81.5 | 0.267 | 45.1 | -4.32 | 6.25 |
| CL4F10-b-7 | 94.6 | 65.3 | 0.196 | 35.3 | -3.30 | 6.25 |
| CL4F11-b-8 | 95.5 | 84.5 | 0.235 | 50.5 | -2.95 | 6.20 |
| CL4F9-b | 31.5 | 243.3 | 0.150 | 135.0 | -0.67 | 6.75 |
| CL4F14-b | 91.5 | 98.6 | 0.112 | 70.1 | -2.43 | 6.45 |
| CL4F17-b | 87.5 | 90.5 | 0.259 | 55.2 | -2.40 | 6.50 |

With any of the pH-sensitive cationic lipids was used, lipid nanoparticles could be prepared in which nucleic acid has been encapsulated. It was observed that the greater the carbon number of the pH-sensitive cationic lipid, the higher the encapsulation rate (%), the smaller the particle diameter, and the smaller the pKa. Moreover, when linear pH-sensitive cationic lipids (CL4F6-b-3, CL4F7-b-4, CL4F8-b-5, CL4F9-b-6, CL4F10-b-7, CL4F11-b-8) were compared with cyclic pH-sensitive cationic lipids (CL4F9-b, CL4F14-b, CL4F17-b), it was observed that the cyclic types tended to have a higher pKa than the linear types.

Next, each of the prepared Fluc mRNA-loaded lipid nanoparticles was administered to Balb/c mice, and the Fluc activity (RLU/mg protein) was measured. The results are shown in FIG. 1. Fluc activity was confirmed in the liver and spleen of mice administered with either type of lipid nanoparticle. These results confirmed that all of the pH-sensitive cationic lipids used in this study are suitable as constituent lipids for lipid nanoparticles useful as gene carriers. Among them, CL4F8-B-5, CL4F9-B-6, and CL4F10-B-7 showed gene transfer ability equal to or greater than that of CL4F6.

Moreover, Table 6 shows the results of lipid nanoparticles containing pH-sensitive cationic lipids in which, in the general formula (A), c is 4 (pH-sensitive cationic lipids branched at the ε position) among the pH-sensitive cationic lipids of the present invention.

[Table 6]

| pH-sensitive cationic lipid | Encapsulation rate (%) | ζ-average (nm) | PdI | Number-average particle diameter (nm) | ζ-potential (mV) | pKa |
|---|---|---|---|---|---|---|
| CL4F10-e-2 | 67.5 | 129.9 | 0. 177 | 72.4 | -2.17 | 6.55 |
| CL4F10-e-3 | 91.3 | 84.0 | 0.273 | 39.6 | -3.94 | 6.60 |
| GL4F10-e-4 | 87.8 | 107.5 | 0.208 | 64.2 | -2.46 | 6.35 |
| CL4F10-e-6 | 90.5 | 92.1 | 0. 261 | 46.0 | -6.38 | 6.30 |

With any of the pH-sensitive cationic lipids was used, lipid nanoparticles could be prepared in which nucleic acid has been encapsulated. It was observed that, even in the case of pH-sensitive cationic lipids branched at the ε position, the greater the number of carbon atoms, the higher the encapsulation rate (%), the smaller the particle diameter, and the smaller the pKa.

Next, each of the prepared Fluc mRNA-loaded lipid nanoparticles was administered to Balb/c mice, and the Fluc activity (RLU/mg protein) was measured. The results are shown in FIG. 2. Fluc activity was confirmed in the liver and spleen of mice administered with either type of lipid nanoparticle. These results confirmed that all of the pH-sensitive cationic lipids used in this study are suitable as constituent lipids for lipid nanoparticles useful as gene carriers. Notably, all of the lipid nanoparticles containing pH-sensitive cationic lipids also showed high gene transfer activity in the spleen compared to lipid nanoparticles containing CL4F6. These results suggested that lipid nanoparticles comprising a pH-sensitive cationic lipid branched at the ε position, among the pH-sensitive cationic lipids of the present invention, are useful as gene carriers for targeting the spleen.

[Example 2]

For lipid nanoparticles containing CL4F11-e-3 as a pH-sensitive cationic lipid, the effects of the type and content ratio of neutral lipids were examined.

Specifically, Fluc mRNA-loaded lipid nanoparticles were prepared by the alcohol dilution method using CL4F11-e-3, DSPC or DOPE, cholesterol, and PEG-DMG in a molar ratio of 50/10/40/0.75 (mol %). The type of pH-sensitive cationic lipid used in the preparation, the encapsulation rate (%), ζ-average particle diameter (nm), number-average particle diameter (nm), PdI, and ζ-potential (mV) of the prepared lipid nanoparticles were measured. The results are shown in Table 7.

[Table 7]

| Neutral lipid | Encapsulation rate (%) | ζ-average (nm) | PdI | Number-average particle diameter (nm) | ζ-potential (mV) |
|---|---|---|---|---|---|
| DSPC | 85.3 | 104.7 | 0.275 | 54.7 | -2.41 |
| DOPE | 89.1 | 82.1 | 0.196 | 55.7 | -2.35 |

As a result, lipid nanoparticles in which a nucleic acid has been encapsulated could be prepared with both the lipid nanoparticles containing DSPC and lipid nanoparticles containing DOPE, regardless of the ratio of the neutral lipid content therein. The lipid nanoparticles containing DOPE showed more uniform and higher encapsulation rate.

Next, each of the prepared Fluc mRNA-loaded lipid nanoparticles was administered to Balb/c mice, and the Fluc activity (RLU/mg protein) was measured. The results are shown in FIG. 3. In mice administered with either of the lipid nanoparticles, a high Fluc activity was confirmed in the liver and spleen, confirming their usefulness as gene carriers for targeting the liver or spleen. In particular, the lipid nanoparticles containing DOPE showed stable activity over a wide range of DOPE contents.

[Example 3]

Among the Fluc mRNA-loaded lipid nanoparticles prepared in Example 1, the lipid nanoparticles containing CL4H6, CL4F6, or CL4F10-e-2 were examined for their metabolism in the liver.

Specifically, lipid nanoparticles encapsulating Fluc mRNA (Fluc mRNA-loaded lipid nanoparticles) were intravenously injected into BALB/c mice (female, 4 weeks old) at a dose of 0.5 mg mRNA/kg body weight. The mice were euthanized 0.5 hours, 4 hours, or 24 hours after the intravenous injection, and the amount of lipid derived from each lipid nanoparticle in the liver was measured. The measurement results are shown in FIG. 4. As a result, with regard to the

content in the liver, CL4H6 was metabolized and almost completely disappeared from the liver 24 hours after administration, whereas CL4F6 was hardly metabolized and the content in the liver did not change 24 hours after administration. On the other hand, CL4F10-e-2 was metabolized in vivo in the same manner as CL4H6, and most of it was degraded within 24 hours after administration. From these results, it was found that, among the pH-sensitive cationic lipids of the present invention, those branched at the ε-position have good biodegradability.

[Example 4]

Lipid nanoparticles encapsulating TTR-RNP-ssODN complexes (TTR-RNP-ssODN-loaded lipid nanoparticles) were prepared using CL4H6, CL4F6, CL4F8-b-5, CL4F9-b-6, CL4F10-b-7, CL4F11-e-3, or CL4F12-e-6 as the pH-sensitive cationic lipid. As the ssODN in the TTR-RNP-ssODN-loaded lipid nanoparticles, ssODN-TTR_3-40% was used.
Specifically, TTR-RNP-ssODN-loaded lipid nanoparticles were prepared by the alcohol dilution method using a pH-sensitive cationic lipid, DSPC, cholesterol, and PEG-DMG in a molar ratio of 50/10/40/3.5 (mol %). The type of pH-sensitive cationic lipid used in the preparation, the encapsulation rate (%), ζ-average particle diameter (nm), number-average particle diameter (nm), PdI, and ζ-potential (mV) of the prepared lipid nanoparticles were measured. The measurement results are shown in Table 8.

[Table 8]

| pH-sensitive cationic lipid | Encapsulation rate (%) | ζ-average (nm) | PdI | Number-average particle diameter (nm) | ζ-potential (mV) |
|---|---|---|---|---|---|
| CL4H6 | 83.5 | 136.5 | 0 0.254 | 80.1 | 2.83 |
| CL4F6 | 87.3 | 122.8 | 0 0.084 | 90.4 | -0.75 |
| CL4F8-b-5 | 60.2 | 178.0 | 0 0.128 | 127.5 | -0. 95 |
| CL4F9-b-6 | 84.9 | 141.5 | 0 0.145 | 99.6 | -0. 10 |
| CL4F10-b-7 | 86.0 | 127.1 | 0 0.104 | 82.5 | -0. 48 |
| CL4F11-e-3 | 76. 1 | 162.5 | 0 0.104 | 116.0 | 1. 40 |
| CL4F12-e-6 | 81. 4 | 145.6 | 0 0.121 | 106.2 | 0.34 |

Next, each TTR-RNP-ssODN-loaded lipid nanoparticle was administered to mice to measure TTR-KO activity. FIG. 5 shows the results of measuring TTR activity (TTR protein amount) (μg/mL) in mouse serum 7 days after administration of each lipid nanoparticle. In the figure, "NT" indicates the results for mice that were not administered lipid nanoparticles. As a result, TTR-RNP-ssODN-loaded lipid nanoparticles containing CL4F8-b-5, CL4F9-b-6, CL4F10-b-7, CL4F11-e-3, or CL4F12-e-6 induced lower serum TTR activity and higher gene knockout than TTR-RNP-ssODN-loaded lipid nanoparticles containing CL4F6, whose hydrocarbon side chain is branched at the α-position. In particular, the pH-sensitive cationic lipids with fewer carbon atoms had higher knockout activity, and, in particular, CL4F11-e-3 achieved 90% or higher knockout.
Similarly, TTR-RNP-ssODN-loaded lipid nanoparticles were prepared in the same manner using CL4F11-e-3 or CL4F12-e-4 as the pH-sensitive cationic lipid, and the encapsulation rate (%), ζ-average particle diameter (nm), number-average particle diameter (nm), PdI, and ζ-potential (mV) of the prepared lipid nanoparticles were measured. The measurement results are shown in Table 9.

[Table 9]

| pH-sensitive cationic lipid | Encapsulation rate (%) | ζ-average (nm) | PdI | Number-average particle diameter (nm) | ζ-potential (mV) |
|---|---|---|---|---|---|
| CL4F11-e-3 | 60.6 | 163.4 | 0.056 | 132.1 | 0.35 |
| CL4F12-e-4 | 66.8 | 150.0 | 0.074 | 114.3 | -0.94 |

Each TTR-RNP-ssODN-loaded lipid nanoparticle was administered to mice, and TTR activity (μg/mL) in the mouse serum was measured 7 days after administration. The measurement results are shown in FIG. 6. it was revealed that, although the lipid nanoparticles containing CL4F12-e-4 also exhibited excellent knockout activity, the lipid nanoparticles containing CL4F11-e-3 exhibited the highest knockout activity. These results suggested that, in terms of delivery efficiency in the delivery of lipid nanoparticles, in a pH-sensitive cationic lipid, in the general formula (A), $Nc_S$, which is the carbon number or either $R^{11}$ or $R^{12}$ that has the smaller carbon number, is preferably 3 to 6, more preferably 3 to 5, and that c is preferably 4 or more. It was also suggested that, in the general formula (A), the sum of $R^{11}$,

$R^{12}$ and c is preferably about 9 to 16, and most preferably about 11 to 14.

[Example 5]

The effect of the match rate of the ssODN used with the sgRNA on the knockout activity of TTR-RNP-ssODN-loaded lipid nanoparticles was examined.

Specifically, TTR-RNP-ssODN complexes were prepared using sgTTR-G211 or sgTTR-G269 as gRNA and the 11 types of ssODN listed in Table 2 above. The lipid nanoparticles were prepared by the alcohol dilution method using CL4H6, DSPC, cholesterol, and PEG-DMG in a molar ratio of 50/10/40/3.5 (mol %).

Next, each TTR-RNP-ssODN-loaded lipid nanoparticle was administered to mice, and the TTR-KO activity was measured to determine the TTR-KO activity. The obtained TTR-KO activity, the GC content (%) and the Tm value of the ssODN used are shown in Table 10 (n=2). Note that the GC content (%) of the ssODN refers to the GC content (%) of bases in the ssODN that match the sgRNA.

[Table 10]

| sgRNA | ssODN | K0 (%) | | GC (%) | Tm (°C) |
|---|---|---|---|---|---|
| | | 1st test | 2nd test | | |
| G211 | ssODN-TTR_3-30% | 17.4 | 31.6 | 67 | 20 |
| | ssODN-TTR_3-40% | 47.8 | 56.5 | 62 | 26 |
| | ssODN-TTR_3-50% | 34.4 | 46.1 | 60 | 32 |
| | ssODN-TTR_3-60% | 26.4 | 35.4 | 58 | 38 |
| | ssODN-TTR_3-80% | 0.3 | 5.6 | 56 | 50 |
| | ssODN-TTR_3-100% | 20.0 | 28.6 | 50 | 62 |
| G269 | ssODN-TTR_4-80% | | 21.0 | 50 | 48 |
| | ssODN-TTR_4-60% | -0.9 | 30. 7 | 50 | 36 |
| | ssODN-TTR_4-50% | 15.4 | 33.8 | 50 | 30 |
| | ssODN-TTR_4-40% | 19.9 | 29.6 | 50 | 24 |
| | ssODN-TTR_4-30% | 16.4 | 14.4 | 50 | 18 |

As shown in Table 10, among the sgRNAs that paired with sgTTR-G211, ssODN-TTR_3-40% had the highest TTR-KO activity with Tm value =26 °C. Among the sgRNA that paired with sgTTR-G269, ssODN-TTR_4-40% had the highest TTR-KO activity with Tm value = 24 °C. These results demonstrated that the TTR-KO activity was affected by the Tm value of the ssODN, and was improved by using ssODN with a Tm value in the range of 20 to 40 °C.

[Example 6]

TTR-RNP-ssODN-loaded lipid nanoparticles were prepared using sgTTR-G211 and ssODN-TTR_3-0%, ssODN-TTR_3-40%, or ssODN-TTR_3-80%. As a control, lipid nanoparticles encapsulating TTR-RNP without using ssODN (TTR-RNP-loaded lipid nanoparticles) were prepared. The lipid nanoparticles were prepared with a composition of CL4H6, DSPC, cholesterol, and PEG-DMG in a molar ratio of 50/10/40/3.5 (mol %).

The encapsulation rate (%), ζ-average particle diameter (nm), number-average particle diameter (nm), PdI, and ζ-potential (mV) of the produced lipid nanoparticles were measured. The measurement results are shown in Table 11.

[Table 11]

| ssODN | Encapsulation rate (%) | ζ-average (nm) | PdI | Number-average particle diameter (nm) | ζ-potential (mV) |
|---|---|---|---|---|---|
| ssODN-TTR_3-0% | 89.4 | 128.4 | 0.192 | 67.2 | 3.49 |
| ssODN-TTR_3-40% | 86.0 | 120.1 | 0.094 | 85.5 | 2.54 |
| ssODN-TTR_3-80% | 69.4 | 141.3 | 0.112 | 96.8 | 2.88 |

For each of the produced TTR-RNPs, the in vitro DNA cleavage activity (%) was measured (n=3). Statistical analysis used non-repeated ANOVA followed by SNK test. The measurement results (%) (mean value ±SD) are shown in FIG.7. In the figure, "**" means p<0.01. In the figure, "ssODN(-)" indicates the result for TTR-RNP produced without using ssODN, and "0%" indicates the result for TTR-RNP-ssODN containing ssODN-TTR_3-0%. "40%" and "80%" indicate the results for TTR-RNP-ssODN containing ssODN-TTR_3-40% and ssODN-TTR_3-80%, respectively.

As shown in FIG. 7, TTR-RNP-ssODN containing ssODN had reduced DNA cleavage activity in vitro compared to TTR-RNP not containing ssODN (ssODN(-)). TTR-RNP-ssODN containing ssODN-TTR_3-80% had extremely low DNA cleavage activity, whereas TTR-RNP-ssODN containing ssODN-TTR_3-40% had DNA cleavage activity that was about the same as that of TTR-RNP-ssODN containing ssODN-TTR_3-0%. These results demonstrated that the DNA cleavage activity can be improved by controlling the Tm value of ssODN.

Each of the prepared lipid nanoparticles (TTR-RNP-ssODN-loaded lipid nanoparticles containing ssODN-TTR_3-0%, TTR-RNP-ssODN-loaded lipid nanoparticles containing ssODN-TTR_3-40%, and TTR-RNP-ssODN-loaded lipid nanoparticles containing ssODN-TTR_3-80%) was administered to mice to evaluate biodistribution. As a control, TTR-RNP that did not contain ssODN was administered in the same manner to mice as is, without being encapsulated in lipid nanoparticles (free RNP), and the biodistribution was evaluated. The evaluation results are shown in Table 12. The amount of TTR-RNP transferred to the liver and spleen was greater in the case when the lipid nanoparticles encapsulating TTR-RNP-ssODN containing ssODN were administered than that in the case when TTR-RNP not containing ssODN (free RNP) was administered as is (in the table, "ssODN(-)(free)"). It became clear that the presence or absence of encapsulation in lipid nanoparticles and the presence or absence of ssODN in RNPs affect the transfer to the liver and spleen.

[Table 12]

| | ID % / tissue | | | | |
|---|---|---|---|---|---|
| ssODN | liver | spleen | kidney | lung | blood |
| ssODN-TTR_3-0% | 31.62 | 4.18 | 0.17 | 0.18 | 8.48 |
| ssODN-TTR_3-40% | 42.28 | 3.49 | 0.14 | 0.26 | 7.57 |
| ssODN-TTR_3-80% | 43.14 | 3.21 | 0.16 | 0.33 | 11.71 |
| ssODN (-) (free) | 27.78 | 0.89 | 0.22 | 0.32 | 3.07 |

The T7E1 assay was performed on TTR-RNP-ssODN-loaded lipid nanoparticles containing ssODN-TTR_3-40%. Specifically, 2 mg RNP/kg body weight was intravenously injected for two consecutive days, and one week after the final injection, the mice were euthanized and their blood and livers were collected.

Genomic DNA was extracted from the harvested liver and subjected to a T7E1 assay (n=3). As a control, the T7E1 assay was performed in the same manner on livers collected from mice that had not been administered lipid nanoparticles. As a result, administration of TTR-RNP-ssODN-loaded lipid nanoparticles containing ssODN-TTR_3-40% achieved 27% KO activity at the DNA level.

Serum was prepared from the collected blood, and the TTR protein concentration in the serum was measured to determine TTR-KO activity. As a control, TTR protein concentrations in serum of mice that had not been administered lipid nanoparticles were also measured. As a result, the serum TTR protein concentration of mice administered with TTR-RNP-ssODN-loaded lipid nanoparticles containing ssODN-TTR_3-40% was reduced by 81% compared to untreated mice, confirming a high TTR-KO activity.

We also investigated the effect of differences in mismatch sites in the ssON-pairing region in the sgRNA on gene knockout activity. Specifically, TTR-RNP-ssODN-loaded lipid nanoparticles (CL4H6/DSPC/cholesterol/PEG-DMG=50/10/40/3.5 (mol%)) were produced in the same manner as above using sgTTR-G211 and ssODN-TTR_3-10°C-3', ssODN-TTR_3-10°C-5', ssODN-TTR_3-24°C-3', ssODN-TTR_3-24°C-5', ssODN-TTR_3-50°C-3', or ssODN-TTR_3-50°C-5'. The encapsulation rate (%), ζ-average particle diameter (nm), number-average particle diameter (nm), PdI, and ζ-potential (mV) of the produced lipid nanoparticles were measured. The measurement results are shown in Table 13.

[Table 13]

| ssODN | Encapsulation rate (%) | ζ-average (nm) | PdI | Number-average particle diameter (nm) | ζ-potential (mV) |
|---|---|---|---|---|---|
| ssODN-TTR_3-10°C-3' | 62.9 | 172.6 | 0.269 | 95.3 | 5.20 |
| ssODN-TTR_3-10°C-5' | 59.0 | 141.0 | 0.146 | 82.4 | 3. 98 |

(continued)

| ssODN | Encapsulation rate (%) | ζ-average (nm) | PdI | Number-average particle diameter (nm) | ζ-potential (mV) |
|---|---|---|---|---|---|
| ssODN-TTR_3-24°C-3' | 65.6 | 140.1 | 0.200 | 73.1 | 5.73 |
| ssODN-TTR_3-24°C-5' | 61.5 | 123.5 | 0.147 | 76.7 | 2.71 |
| ssODN-TTR_3-50°C-3' | 58.7 | 139.9 | 0.166 | 79.0 | 4.02 |
| ssODN-TTR_3-50°C-5' | 56.6 | 124.9 | 0.094 | 86.8 | 3.46 |

Each TTR-RNP-ssODN-loaded lipid nanoparticle was administered to mice, and the amount of TTR protein in the mouse serum (μg/mL) 7 days after administration was measured. The measurement results are shown in FIG. 8. In the figure, "NT" indicates the results for mice that were not administered lipid nanoparticles. In addition, "10°C-3'", "10°C-5'", "24°C-3'", "24°C-5'", "50°C-3'", and "50°C-5'" respectively show the results for mice administered with TTR-RNP-ssODN-loaded lipid nanoparticles containing ssODN-TTR_3-10°C-3', ssODN-TTR_3-10°C-5', ssODN-TTR_3-24°C-3', ssODN-TTR_3-24°C-5', ssODN-TTR_3-50°C-3', or ssODN-TTR_3-50°C-5'. The serum TTR levels in the case of the TTR-RNP-ssODN-loaded lipid nanoparticles containing ssODN-TTR_3-10°C-3' and in the case of the TTR-RNP-ssODN-loaded lipid nanoparticles containing ssODN-TTR_3-10°C-5' were about the same. Similarly, serum TTR was about the same for TTR-RNP-ssODN-loaded lipid nanoparticles containing ssODN-TTR_3-24°C-3', TTR-RNP-ssODN-loaded lipid nanoparticles containing ssODN-TTR_3-24°C-5', TTR-RNP-ssODN-loaded lipid nanoparticles containing ssODN-TTR_3-50°C-3', and TTR-RNP-ssODN-loaded lipid nanoparticles containing ssODN-TTR_3-50°C-5'. These results suggested that the difference in the mismatch site has almost no effect on the gene knockout activity, and that the Tm value is important.

[Example 7]

Lipid nanoparticles were prepared using CL4F6 synthesized in Synthesis Example 1, CL4F9-b-6 synthesized in Synthesis Example 5, CL4F14-b synthesized in Synthesis Example 9, CL4F12-e-4 synthesized in Synthesis Example 13, CL4F12-e-6 synthesized in Synthesis Example 14, or MC3 as a constituent lipid.
As neutral lipids, DSPC, cholesterol (chol), and PEG-DMG were used.
Specifically, lipid nanoparticles loaded with Fluc mRNA (Fluc mRNA-loaded lipid nanoparticles) were prepared by the alcohol dilution method using a pH-sensitive cationic lipid, DSPC, cholesterol, and PEG-DMG in a molar ratio of 50/10/40/1.5 (mol%). Note that, the composition of the lipid nanoparticles containing MC3 as the pH-sensitive cationic lipid comprises MC3, DSPC, cholesterol, and PEG-DMG in a molar ratio of 50/10/38.5/1.5 (mol %).
Next, among the prepared Fluc mRNA-loaded lipid nanoparticles, lipid nanoparticles containing CL4F6, CL4F9-b-6, CL4F14-b, or CL4F12-e-4 were administered to Balb/c mice, and the Flue activity (RLU/mg protein) was measured (n=2). The results are shown in FIG. 9. In mice administered with either type of lipid nanoparticle, Flue activity was confirmed in the liver, spleen, lungs, brain, heart, kidneys, and muscles. In particular, Fluc activity was high in the liver and spleen. These results confirmed that all of the pH-sensitive cationic lipids used in this study are suitable as constituent lipids for lipid nanoparticles that are useful as gene carriers for various tissues, and are particularly useful as constituent lipids for gene carriers for the liver and spleen.
In addition, for mice administered with Fluc mRNA-loaded lipid nanoparticles containing CL4F12-e-4, blood was collected 24 hours after administration and tested for each component. As a control, a blood test was also performed in a similar manner on mice that received PBS instead of the Fluc mRNA-loaded lipid nanoparticles. The results are shown in Table 14 (n=3, unpaired t-test). In the table, TP stands for total protein, AST for aspartate transaminase, ALB for albumin, LDH for lactose dehydrogenase, BUN for blood urea nitrogen, γ-GT for γ-glutamyl transpeptidase, CRE for creatinine, T-CHO for total cholesterol, IP for inorganic phosphate, TG for triglyceride, HDL-C for high-density lipoprotein cholesterol, T-BIL for total bilirubin, and GLU for glucose, respectively. Additionally, ** indicates P<0.01, and * indicates P<0.05. As a result, no particular toxicity was observed in mice administered with Fluc mRNA-loaded lipid nanoparticles containing CL4F12-e-4.

[Table 14]

| Parameters | PBS | LNP | Parameters | PBS | LNP |
|---|---|---|---|---|---|
| TP (g/dL) | 5 ± 0.3 | 4.9 ± 0.1 | ALT (IU/L) | 25.3 ± 6.6 | 39.3 ± 3.4 |
| ALB (g/dL) | 3.5 ± 0.2 | 3.3 ± 0.1 | LDH (IU/L) | 618.7 ± 108.7 | 732 ± 39.6 |
| BUN (mg/dL) | 20.1 ± 2.8 | 25.3 ± 2.8 | AMY (IU/L) | 2590 ± 154.5 | 1879.3 ± 136.2** |
| CRE (mg/dL) | 0.1 ± 0.02 | 0.1 ± 0.01 | γ-GT (IU/L) | 6> | 6> |

(continued)

| Parameters | PBS | LNP | Parameters | PBS | LNP |
|---|---|---|---|---|---|
| Na (mEq/L) | 146.7 ± 7.7 | 150.7 ± 1.9 | T-CHO (mg/dL) | 90 ± 4.3 | 154.7 ± 8.4** |
| K (mEq/L) | 8.1 ± 1.4 | 6.9 ± 0.2 | TG (mg/dL) | 140 ± 31.3 | 134 ± 52.8 |
| Cl (mEq/L) | 94 ± 4.3 | 98 ± 0 | HDL-C (mg/dL) | 53.3 ± 2.5 | 78.7 ± 4.7** |
| Ca (mg/dL) | 9.3 ± 0.5 | 11.9 ± 0.4** | T-BIL (mg/dL) | 0.1 ± 0 | 0.03 ± 0.01 |
| IP (mg/dL) | 11.9 ± 0.1 | 11.1 ± 0.5 | GLU (mg/dL) | 235.3 ± 50.5 | 214 ± 8.2 |
| AST (IU/L) | 52.7 ± 9.3 | 95.3 ± 3.4** | | | |

In addition, among the prepared Flue mRNA-loaded lipid nanoparticles, for the lipid nanoparticles containing CL4F12-e-6 or MC3, the encapsulation rate (%), ζ-average particle diameter (nm), number-average particle diameter (nm), PdI, ζ-potential (mV), and pKa of the prepared lipid nanoparticles were measured. The results are shown in Table 15.

[Table 15]

| Cationic lipid | Encapsulation rate (%) | ζ-average (nm) | PdI | Number-average particle diameter (nm) | ζ-potential (mV) |
|---|---|---|---|---|---|
| MC3 | 79.3 | 68.6 | 0.198 | 48.0 | 2.19 |
| CL4F12-e-6 | 91.0 | 128.2 | 0.141 | 74.9 | 2.99 |

Fluc mRNA-loaded lipid nanoparticles containing CL4F12-e-6 or MC3 were each administered to Balb/c mice, and the Flue activity (RLU/mg protein) was measured (n=2). The results are shown in FIG. 10. It was confirmed that, in the liver, spleen, lung, and kidney, the lipid nanoparticles containing CL4F12-e-6 had higher Fluc activity than the lipid nanoparticles containing MC3. These results indicate that lipid nanoparticles containing the pH-sensitive cationic lipid of the present invention, such as CL4F12-e-6, as a constituent lipid are good gene carriers with higher gene expression activity than lipid nanoparticles containing MC3 that are currently in clinical application.

Furthermore, the lipid nanoparticles loaded with Fluc mRNA containing CL4F12-e-6 were administered to mice after either freeze-thaw treatment or without freeze-thaw treatment, and the gene expression activity in each tissue was examined.

Specifically, Fluc mRNA-loaded lipid nanoparticles were prepared as follows. First, a 1 mg/mL mRNA solution was diluted with citrate buffer (25 mM, pH 4.0) to 73.3 ng/μL, and this was used as an mRNA solution. A lipid solution was prepared by dissolving each lipid in ethanol so that the total lipid concentration was 8 mM and the molar ratio composition of each lipid was CL4F12-e-6/DSPC/cholesterol/PEG-DMG=50/10/40/1.5 (mol%) (N/P=6). A 10 mL syringe (HAMILTON) was attached to a micro-flow path with a built-in baffle mixer, and the solutions were run at the total flow rate of 2.0 mL/min, with a flow rate ratio of mRNA solution: lipid solution = 3:1 (1.5 mL/min:0.5 mL/min). The solution flowing through the flow path was collected, and an appropriate amount was transferred to a dialysis membrane ("Spectra/Por 4 dialysis membrane", MWCO: 12000-15000), dialyzed twice for 2 hours at 4 °C in sucrose-containing Tris buffer (20 mM Tris-HCl, 9% by mass sucrose, pH 7.4), and collected to give Fluc mRNA-loaded lipid nanoparticles.

A portion of the obtained Fluc mRNA-loaded lipid nanoparticles was frozen at -80 °C and then dissolved at room temperature to prepare a "frozen sample." On the other hand, samples that were not subjected to the freeze-thaw treatment were designated as "non-frozen samples." The encapsulation rate (%), ζ-average particle diameter (nm), number-average particle diameter (nm), PdI, ζ-potential (mV), and pKa of the prepared lipid nanoparticles were measured for the frozen and non-frozen samples. The results are shown in Table 16. In the "Freeze" column, "-" indicates the results for non-frozen samples, and "+" indicates the results for frozen samples.

[Table 16]

| Freeze | Encapsulation rate (%) | ζ-average (nm) | PdI | Number-average particle diameter (nm) | ζ-potential (mV) |
|---|---|---|---|---|---|
| - | 89.6 | 104.9 | 0.115 | 73.8 | 0.84 |
| + | 90.1 | 108.8 | 0.124 | 74.8 | 4.17 |

Furthermore, the frozen sample and the non-frozen sample were each administered to Balb/c mice, and the Fluc activity (RLU/mg protein) was measured (n=3, unpaired t-test). The results are shown in FIG. 11. As shown in Figure 11, no significant difference was observed in gene expression activity in each tissue between the frozen and non-frozen samples,

confirming that lipid nanoparticles containing CL4F12-e-6 were hardly affected by the freeze-thaw treatment.

[Example 8]

Lipid nanoparticles encapsulating the TTR-RNP-ssODN complex (TTR-RNP-ssODN-loaded lipid nanoparticles) were prepared in the same manner as in Example 4 using CL4F11-e-3 synthesized in Synthesis Example 12, CL4F11-ζ-2 synthesized in Synthesis Example 15, CL4F10-ζ-3(3) synthesized in Synthesis Example 18, CL4F12-η-2 synthesized in Synthesis Example 16, CL4F11-η-3(3) synthesized in Synthesis Example 17, CL4F12-ζ-2(2) synthesized in Synthesis Example 20, CL4F12-θ-3(3) synthesized in Synthesis Example 19, and CL4F13-η-2(2) synthesized in Synthesis Example 21 as constituent lipids. As the ssODN in the TTR-RNP-ssODN-loaded lipid nanoparticles, ssODN-TTR_3-40% was used.

As neutral lipids, DSPC, cholesterol (chol), and PEG-DMG were used.

Specifically, TTR-RNP-ssODN-loaded lipid nanoparticles were prepared by the alcohol dilution method using a pH-sensitive cationic lipid, DSPC, cholesterol, and PEG-DMG in a molar ratio of 50/10/40/3.5 (mol %). The encapsulation rate (%), ζ-average particle diameter (nm), number-average particle diameter (nm), PdI, ζ-potential (mV), and pKa of each of the prepared lipid nanoparticles were measured. The results are shown in Table 17. In the table, "Side-chain carbon number" refers to the total number of carbon atoms constituting the side chain of the contained pH-sensitive cationic lipid (the total number of carbon atoms constituting the molecule minus the number of carbon atoms from the end of the main chain to the branching position).

[Table 17]

| Side-chain carbon number | pH-sensitive cationic lipid | Encapsulation rate (%) | ζ-average (nm) | PdI | Number-average particle diameter (nm) | ζ-potential (mV) |
|---|---|---|---|---|---|---|
| 14 | CL4F11-e-3 | 80.1 | 140.2 | 0. 076 | 105.9 | -1.01 |
| 13 | CL4F11-ζ-2 | 71. 4 | 192.4 | 0.230 | 89.9 | -1.34 |
| | CL4F10-ζ-3(3) | 58.4 | 160.0 | 0.092 | 119.2 | 3.05 |
| 14 | CL4F12-η-2 | 74.9 | 118.2 | 0.138 | 77.2 | 3.16 |
| | CL4F11-η-3(3) | 80.4 | 150.7 | 0. 249 | 34.9 | 6.45 |
| | CL4F12-ζ-2(2) | 67. 1 | 125.3 | 0.116 | 82.2 | 2.44 |
| 15 | CL4F12-e-3(3) | 80. 6 | 103.0 | 0.104 | 71.8 | 4.37 |
| | CL4F13-η-2(2 | 79.6 | 97.6 | 0. 132 | 55. 3 | 0.28 |

Next, each TTR-RNP-ssODN-loaded lipid nanoparticle was administered to mice to measure TTR-KO activity. FIG. 12 shows the results of the measurement of the amount of TTR protein (μg/mL) in the serum of mice 7 days after administration of each lipid nanoparticle. In the figure, "NT" indicates the results for mice that were not administered lipid nanoparticles. The same applies to "NT" in the following figures. As a result, lipids with fewer carbon atoms in the scaffold showed lower serum TTR activity and higher gene expression knockout activity. The TTR-RNP-ssODN-loaded lipid nanoparticles containing CL4F11-ζ-2 (in the figure, "CL4F11-ζ-2") showed the lowest serum TTR protein amount and the highest knockout activity. For this reason, TTR-RNP-ssODN-loaded lipid nanoparticles containing CL4F11-ζ-2 were used in subsequent experiments.

TTR-RNP-ssODN-loaded lipid nanoparticles containing CL4F11-ζ-2 were prepared in the same manner, except that the ratio of the PEG-DMG content was made 1.5 to 3.5 mol%, and that the weight ratio of RNP to be contained relative to the total weight of lipids constituting the lipid nanoparticles ([RNP weight]/[total lipid weight]) was made 2, 4, or 5.6 mass%. For each of the prepared TTR-RNP-ssODN-loaded lipid nanoparticles, the encapsulation rate (%), ζ-average particle diameter (nm), number-average particle diameter (nm), PdI, ζ-potential (mV), and pKa were measured. The results are shown in Table 18. In the table, "RNP/lipid" represents [RNP weight]/[total lipid weight], and "PEG" represents the ratio of PEG-DMG content (mol %) relative to the total lipid amount.

[Table 18]

| RNP/lipid (%) | PEG (mol%) | Encapsulation rate (%) | ζ-average (nm) | PdI | Number-average particle diameter (nm) | ζ-potential (mV) |
|---|---|---|---|---|---|---|
| 5.6 | 3.5 | 79.0 | 155.1 | 0.198 | 96.2 | 2.73 |
| | 2.5 | 67.8 | 164.7 | 0.129 | 109.8 | 2.81 |
| | 1.5 | 59.0 | 187.2 | 0.156 | 140.9 | -0.14 |
| 4.0 | 3.5 | 82.4 | 132.1 | 0.128 | 93.5 | 4.25 |
| | 2.5 | 73.9 | 152.3 | 0.077 | 121.2 | 1.59 |
| | 1.5 | 56.7 | 202.1 | 0.239 | 127.3 | -0.51 |
| 2.0 | 3.5 | 93.8 | 136.0 | 0.182 | 93.5 | 3.84 |
| | 2.5 | 86.7 | 179.2 | 0.213 | 107.4 | 4.97 |
| | 1.5 | 68.6 | 174.6 | 0.104 | 137.0 | 2.83 |

As shown in Table 18, it was observed that the encapsulation rate tends to decrease with decreasing PEG-DMG content ratio. On the other hand, although it was observed that a smaller weight ratio of [RNP weight]/[total lipid weight] tended to result in a smaller particle diameter of the lipid nanoparticles, no dramatic effect was observed.

Each TTR-RNP-ssODN-loaded lipid nanoparticle was administered to mice (0.2 mg RNP/kg body weight), and the amount of TTR protein in the mouse serum (μg/mL) 7 days after administration was measured. The measurement results are shown in FIG. 13. As shown in Figure 13, serum TTR activity was about the same in mice administered with any of the TTR-RNP-ssODN-loaded lipid nanoparticles, confirming that the PEG-DMG content ratio and the weight ratio of [RNP weight]/[total lipid weight] did not significantly affect gene knockout activity.

TTR-RNP-ssODN-loaded lipid nanoparticles containing CL4F11-ζ-2 were prepared in the same manner except that the CL4F11-ζ-2 content was 40, 50, or 60 mol%, and the DSPC content was 5 or 10 mol%. That is, the lipid composition ratio of the prepared TR-RNP-ssODN-loaded lipid nanoparticles is CL4F11-ζ-2/DSPC/cholesterol/PEG-DMG = x/y/100-x-y/3.5 (mol%), where the CL4F11-ζ-2 content is x% (x = 40, 50, or 60) and the DSPC content is y% (y = 5 or 10). For each of the prepared TTR-RNP-ssODN-loaded lipid nanoparticles, the encapsulation rate (%), ζ-average particle diameter (nm), number-average particle diameter (nm), PdI, ζ-potential (mV), and pKa were measured. The results are shown in Table 19. In the table, "CL (mol %)" indicates the content ratio (mol %) of CL4F11-ζ-2, and "PC (mol %)" indicates the content ratio (mol %) of DSPC. As shown in Table 19, even if the content ratios of CL4F11-ζ-2 and DSPC were changed, there was little effect on physical properties such as the encapsulation rate (%).

[Table 19]

| CL (mol%) | PC (mol%) | Encapsulation rate(%) | ζ-average (nm) | PdI | Number-average particle diameter (nm) | ζ-potential (mV) |
|---|---|---|---|---|---|---|
| 40 | 5 | 64.1 | 165.3 | 0.090 | 129.3 | 2.49 |
| | 10 | 63.1 | 243.3 | 0.214 | 145.2 | 3.98 |
| 50 | 5 | 10.5 | 163.2 | 0.091 | 128.0 | 3.01 |
| | 10 | 61.5 | 186.7 | 0.118 | 150.9 | 1.90 |
| 60 | 5 | 84.1 | 153.6 | 0.078 | 117.4 | 1.59 |
| | 10 | 83.1 | 132.7 | 0.074 | 106.7 | 2.25 |

Each TTR-RNP-ssODN-loaded lipid nanoparticle was administered to mice (0.2 mg RNP/kg body weight), and the amount of TTR protein in the mouse serum (μg/mL) 7 days after administration was measured. The measurement results are shown in FIG. 14. In the figure, "CL (mol %)" and "PC (mol %)" are the same as in Table 19. As shown in Figure 14, serum TTR activity was about the same in mice administered with any of the TTR-RNP-ssODN-loaded lipid nanoparticles, confirming that the ratios of pH-sensitive cationic lipid and the neutral lipid in the lipid nanoparticles have little effect on gene knockout activity.

The TTR-RNP-ssODN-loaded lipid nanoparticles containing CL4F11-ζ-2 (CL4F11-ζ-2/DSPC/cholesterol/PEG-DMG=50/10/40/3.5 (mol%)) were administered to mice (0.2, 0.75, or 2.0 mg RNP/kg body weight), and the amount of TTR protein (μg/mL) in the serum of the mice 7 days after administration was measured. The results of measuring the serum TTR protein amount are shown in FIG. 15(A). Furthermore, the knockout efficiency (%) calculated from the serum TTR protein amount is shown in FIG. 15(B). As shown in FIG. 15(A), when TTR-RNP-ssODN-loaded lipid nanoparticles

containing CL4F11-ζ-2 were administered at 2.0 mg RNP/kg body weight, 98% suppression of TTR gene expression was achieved even with a single administration. It was also confirmed that the knockout activity increased in a dose-dependent manner (FIG. 15(B)).

TTR-RNP-ssODN-loaded lipid nanoparticles containing CL4F11-ζ-2 (CL4F11-ζ-2/DSPC/cholesterol/PEG-DMG = 50/10/40/3.5 (mol%)) were administered to mice (2.0 mg RNP/kg body weight), and the amount of TTR protein (μg/mL) in the serum of the mice was measured 1, 4, or 12 weeks after administration. As a control, Cas9/sgGFP-RNP prepared using sgRNA targeting the GFP gene (sgGFP) instead of sgTTR was loaded in a similar manner onto a lipid nanoparticle containing CL4F11-ζ-2 (GFP-RNP-ssODN-loaded lipid nanoparticle), which is administered to mice in a similar manner, and the amount of TTR protein in serum (μg/mL) was measured. The measurement results are shown in FIG. 16. In the figure, "TTR" indicates the results for mice administered with TTR-RNP-ssODN-loaded lipid nanoparticles, and "GFP" indicates the results for mice administered with GFP-RNP-ssODN-loaded lipid nanoparticles.

The gene knockout activity of TTR-RNP-ssODN-loaded lipid nanoparticles was compared between the case of lipid nanoparticles containing CL4F11-ζ-2 as a constituent lipid and the case of gene carriers containing SM, ALC, or MC3 as a constituent lipid. ALC and SM are also known as constituent lipids of gene carriers.

For each of the prepared TTR-RNP-ssODN-loaded lipid nanoparticles, the encapsulation rate (%), ζ-average particle diameter (nm), number-average particle diameter (nm), PdI, ζ-potential (mV), and pKa were measured. The results are shown in Table 20. As shown in Table 20, there was no significant difference in physical properties such as the encapsulation rate (%) among all the TTR-RNP-ssODN-loaded lipid nanoparticles.

[Table 20]

| Cationic lipid | Encapsulation rate (%) | ζ-average (nm) | PdI | Number-average particle diameter (nm) | ζ-potential (mV) |
|---|---|---|---|---|---|
| CL4F11-ζ-2 | 60.0 | 140.2 | 0.113 | 98.4 | 3.61 |
| SM | 78.3 | 93.6 | 0.137 | 64.6 | 1.43 |
| ALC | 73.9 | 78.8 | 0.138 | 50.7 | 0.51 |
| MC3 | 78.7 | 155.9 | 0.213 | 82.7 | 0.52 |

Each TTR-RNP-ssODN-loaded lipid nanoparticle was administered to mice (2.0 mg RNP/kg body weight), and the amount of TTR protein in the mouse serum (μg/mL) 7 days after administration was measured (n=3). The measurement results are shown in FIG. 17. As shown in FIG. 17, TTR-RNP-ssODN-loaded lipid nanoparticles containing CL4F11-ζ-2 exhibited significantly superior gene knockout activity than other TTR-RNP-ssODN-loaded lipid nanoparticles.

The gene expression activity of lipid nanoparticles loaded with Fluc mRNA was also compared between the case of lipid nanoparticles containing CL4F11-ζ-2 as a constituent lipid and the case of gene carriers containing SM, ALC, or MC3 as a constituent lipid.

The encapsulation rate (%), ζ-average particle diameter (nm), number-average particle diameter (nm), PdI, ζ-potential (mV), and pKa of each of the prepared Fluc mRNA-loaded lipid nanoparticles were measured. The results are shown in Table 21. As shown in Table 21, there was no significant difference in physical properties such as the encapsulation rate (%) among all the Fluc mRNA-loaded lipid nanoparticles.

[Table 21]

| Cationic lipid | Encapsulation rate (%) | ζ-average (nm) | Pd! | Number-average particle diameter (nm) | ζ-potential (mV) |
|---|---|---|---|---|---|
| CL4F11-ζ-2 | 68.7 | 150.5 | 0.151 | 80.5 | -3.87 |
| SM | 88.4 | 76.7 | 0.131 | 52.4 | 0.24 |
| ALC | 87.4 | 61.9 | 0.085 | 44.7 | -1.75 |
| MC3 | 87.1 | 112.4 | 0.190 | 71.0 | -2.17 |

Each Fluc mRNA-loaded lipid nanoparticle was administered to Balb/c mice (0.01 mg mRNA/kg body weight), and the Fluc activity (RLU/mg protein) of the mice was measured 6 hours after administration (n=3, unpaired t-test). The measurement results are shown in FIG. 18. As shown in FIG. 18, Fluc mRNA-loaded lipid nanoparticles containing CL4F11-ζ-2 showed gene expression activity that was about the same as or greater than that of other Fluc mRNA-loaded lipid nanoparticles. In particular, the gene expression activity of the lipid nanoparticles containing CL4F11-ζ-2 in the spleen was superior to that of any of the Fluc mRNA-loaded lipids containing SM, ALC, or MC3, confirming that lipid nanoparticles containing CL4F11-ζ-2 are suitable as gene carriers to target the spleen.

Furthermore, for TTR-RNP-ssODN-loaded lipid nanoparticles containing CL4F11-ζ-2, CL4F11-e-3, or CL4F12-e-4 as pH-sensitive cationic lipids, the effects of freeze-thaw treatment on the physical properties of the lipid nanoparticles themselves and on gene knockout activity when being administered to mice were investigated.

Specifically, TTR-RNP-ssODN-loaded lipid nanoparticles were prepared as follows. First, a 10 μM gRNA solution was stirred using a vortex mixer while a 10 μM Cas9 solution was gradually added thereto to prepare a 5 μM RNP solution. An equivalent amount of 5 μM RNP solution was mixed with 5 μM ssODN solution to give a 2.5 μM RNP-ssODN solution. A 2.5 μM RNP-ssODN solution was diluted with 50 mM NaCl-containing MES buffer (pH 6.0) to 160 nM, and this was used as an RNP solution. A lipid solution was prepared by dissolving each lipid in ethanol to give a desired molar ratio composition with a total lipid concentration of 8 mM. A 10 mL syringe (HAMILTON) was attached to a micro-flow path with a built-in baffle mixer, and the solutions were run at the total flow rate of 2.0 mL/min, with a flow rate ratio of RNP solution: lipid solution = 9:1 (1.8 mL/min:0.2 mL/min). The solution flowing through the flow path was collected, and an appropriate amount was transferred to a dialysis membrane ("Spectra/Por 4 dialysis membrane", MWCO: 12000-15000), dialyzed twice for 2 hours at 4 °C in sucrose-containing Tris buffer (20 mM Tris-HCl, 9% by mass sucrose, pH 7.4), and collected to give TTR-RNP-ssODN-loaded lipid nanoparticles.

A portion of the obtained TTR-RNP-ssODN-loaded lipid nanoparticles was frozen at -80 °C and then thawed at room temperature to prepare a "frozen sample." On the other hand, samples that were not subjected to the freeze-thaw treatment were designated as "non-frozen samples." The encapsulation rate (%), ζ-average particle diameter (nm), number-average particle diameter (nm), PdI, ζ-potential (mV), and pKa of the prepared lipid nanoparticles were measured for the frozen and non-frozen samples. The results are shown in Table 22. In the table, in the "Freeze" column, "-" indicates the results for non-frozen samples, and "+" indicates the results for frozen samples. There was no significant difference in the physical properties between frozen and non-frozen samples of any of the TTR-RNP-ssODN-loaded lipid nanoparticles.

[Table 22]

| Cationic lipid | Freeze | Encapsulation rate (%) | ζ-average (nm) | PdI | Number-average particle diameter (nm) | ζ-potential (mV) |
|---|---|---|---|---|---|---|
| CL4F11-ζ-2 | | 57.6 | 144.0 | 0.054 | 116.9 | -0.35 |
| CL4F11-e-3 | | 67.1 | 134.5 | 0.076 | 107.5 | 0.14 |
| CL4F12-e-4 | | 75.3 | 145.1 | 0.126 | 91.2 | -0.78 |
| CL4F11-ζ-2 | | 48.1 | 142.6 | 0.070 | 120.5 | 1.65 |
| CL4F11-e-3 | + | 61.1 | 132.3 | 0.036 | 110.2 | -3.02 |
| CL4F12-e-4 | | 76.8 | 128.8 | 0.017 | 105.5 | 1.37 |

In addition, the frozen sample and the non-frozen sample were each administered to Balb/c mice, and the amount of TTR protein in the mouse serum (μg/mL) 7 days after administration was measured (n=3). The results are shown in FIG. 19. In the figure, "CL" indicates the pH-sensitive cationic lipid contained in the administered TTR-RNP-ssODN-loaded lipid nanoparticles. In the figure, in the "Freeze" column, "Non-frozen" indicates a non-frozen sample, and "Frozen" indicates a frozen sample. As shown in Figure 19, no significant difference was observed in the gene knockout activity between the frozen and non-frozen samples, confirming that lipid nanoparticles containing CL4F11-ζ-2, etc., are hardly affected by the freeze-thaw treatment.

For lipid nanoparticles containing CL4F11-ζ-2, their tissue distribution was examined when TTR-RNP-ssODN was loaded thereon and when sgTTR itself was loaded thereon as siRNA without being converted into RNP.

TTR-RNP-ssODN-loaded lipid nanoparticles containing CL4F1 1-ζ-2 labelled with DiR (DiR-labeled TTR-RNP-ssODN-loaded lipid nanoparticles) were used.

siTTR-loaded lipid nanoparticles onto which sgTTR was loaded directly as siRNA were prepared as follows. First, a 1 mg/mL siRNA solution was diluted with 25 mM acetate buffer (pH 4.0) to 48.9 ng/μL, and this was used as a siRNA solution. A lipid solution was prepared by dissolving each lipid in ethanol to give a desired composition (mol %) with a total lipid concentration of 8 mM (N/P=9). To fluorescently label the lipid nanoparticles, a DiR solution was added to the lipid solution at a concentration of 0.5 mol %. A 10 mL syringe (HAMILTON) was attached to a micro-flow path with a built-in baffle mixer, and the solutions were run at the total flow rate of 0.5 mL/min, with a flow rate ratio of siRNA solution: lipid solution = 3:1 (0.375 mL/min:0.125 mL/min). The solution flowing through the flow path was collected, and an appropriate amount was transferred to a dialysis membrane (Spectra/Por 4 dialysis membrane, MWCO: 12000-15000), dialyzed twice in PBS(-) at 4 °C for 2 hours, and collected to give DiR-labeled siTTR-loaded lipid nanoparticles.

To BALB/c mice (female, 4 weeks old), DiR-labeled TTR-RNP-ssODN-loaded lipid nanoparticles or DiR-labeled siTTR-loaded lipid nanoparticles diluted to the same DiR fluorescence intensity as the former were administered to via the tail vein at 0.75 mg RNP/kg body weight. One hour after administration, blood and organs were collected, and DiR

fluorescence intensity was measured using an imaging system IVIS (in vivo imaging system, PerkinElmer). The measurement results are shown in FIG. 20. In the figure, "RNP-LNP" indicates the results for mice administered with DiR-labeled TTR-RNP-ssODN-loaded lipid nanoparticles, and "siRNA-LNP" indicates the results for mice administered with DiR-labeled siTTR-loaded lipid nanoparticles.

As shown in Figure 20, the DiR-labeled TTR-RNP-ssODN-loaded lipid nanoparticles and the DiR-labeled siTTR-loaded lipid nanoparticles showed roughly similar tissue distribution when administered into the body. In detail, it was revealed that the DiR-labeled TTR-RNP-ssODN-loaded lipid nanoparticles had higher spleen selectivity than the DiR-labeled siTTR-loaded lipid nanoparticles.

[Example 9]

The effect of the Tm value of the ssODN used in TTR-RNP-ssODN-loaded lipid nanoparticles was examined.

First, sgTTR-G211-5FAM was used as the sgRNA, and ssODN-80%-3IBFQ, ssODN-40%-3IBFQ, or ssODN-0%-3!BFQ was used as the ssODN to measure the Tm values of the sgRNA and ssODN using the fluorescence quenching method (n = 3). FIG. 21(A) shows a plot of the fluorescence intensity at each temperature from 11 to 70 °C, and FIG. 21(B) shows the amount of change in the fluorescence intensity at each temperature. In the figure, "80%," "40%," and "0%" indicate the results of measurements using ssODN-80%-3IBFQ, ssODN-40%-3IBFQ, and ssODN-0%-31BFQ, respectively.

Since ssODN-0%-3IBFQ always dissociates from sgTTR-G211-5FAM, in the reaction solution containing ssODN-0%-3IBFQ, the fluorescence intensity was high at all temperatures and showed almost no change with temperature, as shown in FIG. 21(A). In contrast, in the reaction solutions containing ssODN-80%-3IBFQ and ssODN-40%-3IBFQ, the fluorescence intensity increased with increasing temperature, approaching the fluorescence intensity of the reaction solution containing ssODN-0%-3IBFQ. In other words, it was confirmed that the dissociation of the RNP-ssODN complex occurs in a temperature-dependent manner.

The Tm value, which is the temperature at which the change in fluorescence intensity is greatest, was 62 °C for ssODN-80%-3IBFQ and 32 °C for ssODN-40%-3IBFQ (FIG. 21(B)). At around 40 °C, ssODN-40%-3IBFQ was almost entirely dissociated from RNP, whereas ssODN-80%-3IBFQ was almost entirely complexed with RNP. These results suggested that ssODN-40%-3IBFQ rapidly dissociated from RNPs within cells (37 °C).

Next, the DNA cleavage activity of the RNP-ssODN complex was measured in vitro when ssODNs with a matching rate of 0, 40, or 80% with the ssON-pairing region in the sgRNA were used. As the sgRNA, sgTTR-G211 was used. As the ssODN, ssODN-TTR_3-80%, ssODN-TTR_3-40%, or ssODN-TTR_3-0% was used. The Tm value between sgTTR-G211 and ssODN-TTR_3-80% was 50 °C, whereas the Tm value between sgTTR-G211 and ssODN-TTR_3-40% was 26 °C.

The in vitro DNA cleavage activity was measured at a reaction temperature of 37 °C or 15 °C (n=3). As a control, the DNA cleavage activity of RNP that did not contain ssODN in the reaction system was measured in the same manner. The DNA cleavage activity was calculated as a relative activity value, taking the activity in the absence of ssODN (ssODN(-)) as 100%. The measurement results at 37 °C are shown in FIG. 22(A), and the measurement results at 15 °C are shown in FIG. 22(B). In the figure, "80%," "40%," and "0%" indicate the results of measurements using ssODN-TTR_3-80%, ssODN-TTR_3-40%, and ssODN-TTR_3-0%, respectively, and "ssODN(-)" indicates the result of measurements in the absence of ssODN.

At 37 °C, the relative DNA cleavage activity of the RNP containing ssODN-TTR_3-40% was about the same as that of the RNP containing ssODN-TTR_3-0%. The relative DNA cleavage activity of RNP containing ssODN-TTR_3-80% was significantly low, as low as about 20%. On the other hand, at 15 °C, the relative DNA cleavage activity of RNP containing ssODN-TTR_3-40% was higher than that of RNP containing ssODN-TTR_3-80%, but was clearly lower than that of RNP containing ssODN-TTR_3-0%. The DNA cleavage activity was significantly different between 37 °C and 15 °C when using ssODN-TTR_3-40%. It is surmised that, at 37 °C, which was higher than the Tm value (26 °C) of ssODN-TTR_3-40%, most of ssODN-TTR_3-40% was dissociated from RNP at 37 °C, thereby exerting RNP's inherent DNA cleavage activity; on the other hand, at 15 °C, which was lower than the Tm value, most of ssODN-TTR_3-40% was complexed with RNP, which results in a decrease in DNA cleavage activity.

These results indicate that gene knockout activity can be enhanced in an animal's body by designing the ssODN so that the Tm value between the sgRNA and the ssODN is lower than the animal's internal body temperature (approximately 37 °C).

We also investigated the effect of the temperature at the time of preparing TTR-RNP-ssODN-loaded lipid nanoparticles on their gene knockout activity in cells.

Specifically, first, TTR-RNP-ssODN-loaded lipid nanoparticles with a lipid composition of CL4H6/DSPC/cholesterol/PEG-DMG=50/10/40/3.5 (mol%)
were obtained in a similar manner as in Example 8, except that sgTTR-G211 was used as the sgRNA, and ssODN-TTR_3-30% or ssODN-TTR_3-40% was used as the ssODN, and that the temperature during mixing of the RNP solution

and the lipid solution in the micro-flow path with a built-in baffle mixer was set to 4 °C.

The obtained TTR-RNP-ssODN-loaded lipid nanoparticles were administered to mice (2.0 mg RNP/kg body weight), and the amount of TTR protein in the mouse serum (μg/mL) 7 days after administration was measured. The measurement results are shown in FIG. 23. In the figure, "30%" and "40%" indicate the results for mice administered TTR-RNP-ssODN-loaded lipid nanoparticles containing ssODN-TTR_3-30% and ssODN-TTR_3-40%, respectively. As shown in FIG. 23, both the TTR-RNP-ssODN-loaded lipid nanoparticles using ssODN-TTR_3-30% and the TTR-RNP-ssODN-loaded lipid nanoparticles using ssODN-TTR_3-40% showed low serum TTR activity, exhibiting sufficient gene knockout activity. When the preparation was carried out in an environment with no particular temperature control, the gene knockout activity was not very high (Table 10), whereas when the preparation was carried out at 4 °C, the gene knockout activity was high, presumably because the production temperature was below the Tm value, and the ssODN and sgRNA formed a complex and were efficiently loaded onto the lipid nanoparticles in that state. These results show that RNP-ssODN-loaded lipid nanoparticles having a sufficiently high gene knockout activity can efficiently be obtained by designing the ssODN so that the Tm value between the sgRNA and ssODN is lower than the animal's internal body temperature (approximately 37 °C), and by setting the manufacturing temperature at the time of loading them onto the lipid nanoparticles to a value sufficiently lower than the Tm value.

[Sequence Listing]

## Claims

1. A lipid nanoparticle comprising a pH-sensitive cationic lipid represented by the formula (I):
[Chem. 1]

$$(R^1)(R^2)C(OH)\text{-}(CH_2)a\text{-}(O\text{-}CO)b\text{-}X \qquad (I)$$

[in the formula (I), a denotes an integer from 3 to 5; b denotes 0 or 1; $R^1$ and $R^2$ each independently denote a group represented by the following general formula (A):
[Chem. 2]

$$(R^{11})(R^{12})HC\text{-}(CH_2)c\text{-}(CO\text{-}O)\text{-}(CH_2)_e\text{-} \qquad (A)$$

(in the formula (A), $R^{11}$ and $R^{12}$ each independently denote a linear or branched $C_{1-15}$ alkyl group; c denotes an integer from 1 to 7; and e denotes an integer from 4 to 12; provided that $R^{11}$ and $R^{12}$ may be joined to each other to form a ring);
X denotes a group represented by the following general formula (B):
[Chem. 3]

$$\text{-}(CH_2)d\text{-}N(R^3)(R^4) \qquad (B)$$

(in formula (B), d denotes an integer from 0 to 3; $R^3$ and $R^4$ each independently denote a $C_{1-4}$ alkyl group or a $C_{2-4}$ alkenyl group (in the $C_{1-4}$ alkyl group or the $C_{2-4}$ alkenyl group, one or two hydrogen atoms may be substituted by a phenyl group), or $R^3$ and $R^4$ may be joined to each other to form a 5- to 7-membered non-aromatic heterocycle (in the heterocycle, one or two hydrogen atoms may be substituted by a $C_{1-4}$ alkyl group or $C_{2-4}$ alkenyl group), or a 5- to 7-membered non-aromatic heterocyclic group (provided that the group is bound to (O-CO)b- via a carbon atom, and one or two hydrogen atoms of the ring may be substituted by a $C_{1-4}$ alkyl group or a $C_{2-4}$ alkenyl group) ].

2. The lipid nanoparticle according to claim 1, wherein c is an integer from 4 to 7.

3. The lipid nanoparticle according to claim 1 or 2, wherein the sum of the number of carbon atoms of the group having a larger carbon number out of $R^{11}$ and $R^{12}$ plus c is 5 to 17.

4. The lipid nanoparticle according to claim 1 or 2, further comprising a sterol and a polyalkylene glycol-modified lipid.

5. The lipid nanoparticle according to claim 1 or 2, comprising a nucleic acid.

**6.** The lipid nanoparticle according to claim 5, wherein the nucleic acid is an siRNA, mRNA, or plasmid DNA.

**7.** The lipid nanoparticle according to claim 1 or 2, comprising a DNA nuclease, a guide RNA, and a single-stranded oligonucleotide comprising a region capable of paring with a part of the guide RNA.

**8.** The lipid nanoparticle according to claim 7, wherein 30 to 60% of the bases in the region of the single-stranded oligonucleotide that can pair with a part of the guide RNA are complementary to the bases in the guide RNA.

**9.** The lipid nanoparticle according to claim 7, wherein the DNA nuclease is a Cas9 protein, and the guide RNA consists of crRNA and tracrRNA.

**10.** A lipid nanoparticle comprising a lipid ingredient, a DNA nuclease, a guide RNA, and a single-stranded oligonucleotide comprising a region capable of paring with a part of the guide RNA, wherein 30 to 60% of the bases in the region of the single-stranded oligonucleotide that can pair with a part of the guide RNA are complementary to the bases in the guide RNA.

**11.** A pharmaceutical composition, wherein the lipid nanoparticle according to any one of claim 1, 2 and 10 is an active ingredient.

**12.** The pharmaceutical composition according to claim 11, for use in gene therapy.

**13.** A method for expressing an exogenous gene, comprising administering the lipid nanoparticles according to any one of claim 1, 2 and 10 in which an exogenous gene of interest to be expressed in liver cells or spleen cells is encapsulated to a subject animal (excluding human) and expressing the exogenous gene in the liver or spleen of the subject animal.

**14.** A method for genome editing, comprising introducing the lipid nanoparticle according to claim 10 into a cell.

**15.** A pH-sensitive cationic lipid represented by the formula (I):
[Chem. 4]

$$(R^1)\ (R^2)C(OH)\text{-}(CH_2)a\text{-}(O\text{-}CO)b\text{-}X \qquad (I)$$

[in the formula (I), a denotes an integer from 3 to 5; b denotes 0 or 1; $R^1$ and $R^2$ each independently denote a group represented by the following general formula (A):
[Chem. 5]

$$(R^{11})\ (R^{12})HC\text{-}(CH_2)_c\text{-}(CO\text{-}O)\text{-}\ (CH_2)_e\ \text{-} \qquad (A)$$

(in the formula (A), $R^{11}$ and $R^{12}$ each independently denote a linear or branched $C_{1-15}$ alkyl group; c denotes an integer from 1 to 7; and e denotes an integer from 4 to 12; provided that $R^{11}$ and $R^{12}$ may be joined to each other to form a ring);
X denotes a group represented by the following general formula (B):
[Chem. 6]

$$\text{-}\ (CH_2)d\text{-}N^3(R^3)(R^4) \qquad (B)$$

(in formula (B), d denotes an integer from 0 to 3; $R^3$ and $R^4$ each independently denote a $C_{1-4}$ alkyl group or a $C_{2-4}$ alkenyl group (in the $C_{1-4}$ alkyl group or the $C_{2-4}$ alkenyl group, one or two hydrogen atoms may be substituted by a phenyl group), or $R^3$ and $R^4$ may be joined to each other to form a 5- to 7-membered non-aromatic heterocycle (in the heterocycle, one or two hydrogen atoms may be substituted by a $C_{1-4}$ alkyl group or $C_{2-4}$ alkenyl group), or a 5- to 7-membered non-aromatic heterocyclic group (provided that the group is bound to (O-CO)b- via a carbon atom, and one or two hydrogen atoms of the ring may be substituted by a $C_{1-4}$ alkyl group or a $C_{2-4}$ alkenyl group) ].

**16.** The pH-sensitive cationic lipid according to claim 15, that is

7-(4-(dipropylamino)butyl)-7-hydroxy-13-((3-propylhexanoyl)oxy)tridecyl 3-ethylheptanoic acid,
7-(4-(dipropylamino)butyl)-7-hydroxytridecane-1,13-diylbis(3-butylheptanoate),
7-(4-(dipropylamino)butyl)-7-hydroxytridecane-1,13-diylbis(3-pentyloctanoate),
7-(4-(dipropylamino)butyl)-7-hydroxytridecane-1,13-diylbis(3-hexylnonanoate),
7-(4-(dipropylamino)butyl)-7-hydroxytridecane-1,13-diylbis(3-heptyldecanoate),
7-(4-(dipropylamino)butyl)-7-hydroxytridecane-1,13-diylbis(3-octylundecanoate),
7-(4-(dipropylamino)butyl)-7-hydroxytridecane-1,13-diylbis(2-cycloheptyl acetate),
7-(4-(dipropylamino)butyl)-7-hydroxytridecane-1,13-diylbis(2-cyclododecyl acetate),
7-(4-(dipropylamino)butyl)-7-hydroxytridecane-1,13-diylbis(2-cyclopentadecyl acetate),
7-(4-(dipropylamino)butyl)-7-hydroxytridecane-1,13-diylbis(6-ethyldecanoate),
7-(4-(dipropylamino)butyl)-7-hydroxytridecane-1,13-diylbis(6-propylundecanoate),
7-(4-(dipropylamino)butyl)-7-hydroxytridecane-1,13-diylbis(6-butyldodecanoate),
7-(4-(dipropylamino)butyl)-7-hydroxytridecane-1,13-diylbis(6-hexyldodecanoate),
7-(4-(dipropylamino)butyl)-7-hydroxytridecane-1,13-diylbis(7-ethylundecanoate),
7-(4-(dipropylamino)butyl)-7-hydroxytridecane-1,13-diylbis(8-ethyldodecanoate),
7-(4-(dipropylamino)butyl)-7-hydroxytridecane-1,13-diylbis(8,10,10-trimethylundecanoate),
7-(4-(dipropylamino)butyl)-7-hydroxytridecane-1,13-diylbis(7,9,9-trimethyldecanoate),
7-(4-(dipropylamino)butyl)-7-hydroxytridecane-1,13-diylbis(9,11,11-trimethyldodecanoate),
7-(4-(dipropylamino)butyl)-7-hydroxytridecane-1,13-diylbis(7,11-dimethyldodecanoate), or
7-(4-(dipropylamino)butyl)-7-hydroxytridecane-1,13-diylbis(8,12-dimethyltridecanoate).

[Fig. 1]

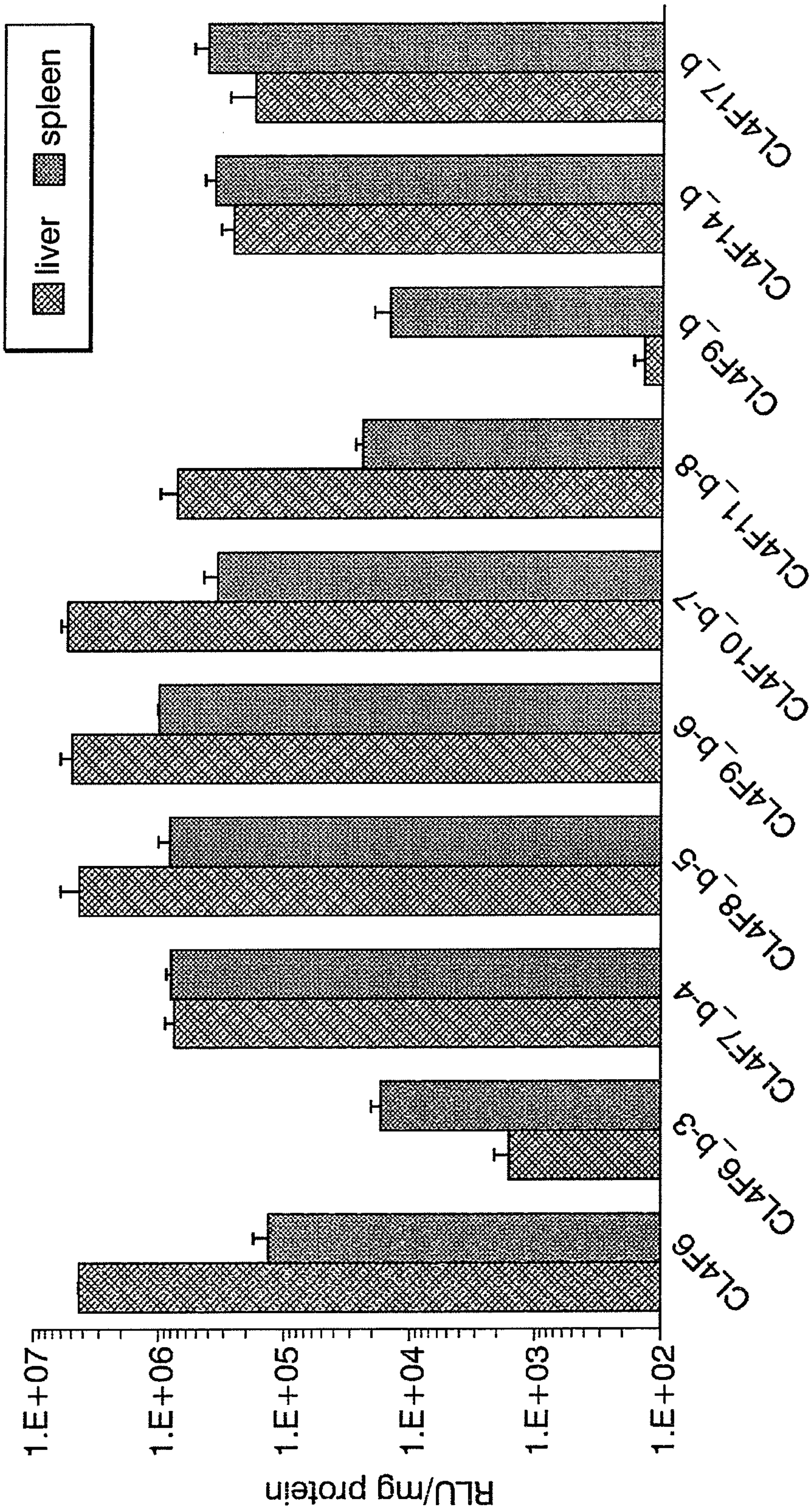
liver
spleen
CL4F6
CL4F6_b-3
CL4F7_b-4
CL4F8_b-5
CL4F9_b-6
CL4F10_b-7
CL4F11_b-8
CL4F9_b
CL4F14_b
CL4F17_b
1.E+07
1.E+06
1.E+05
1.E+04
1.E+03
1.E+02
RLU/mg protein

[Fig. 2]

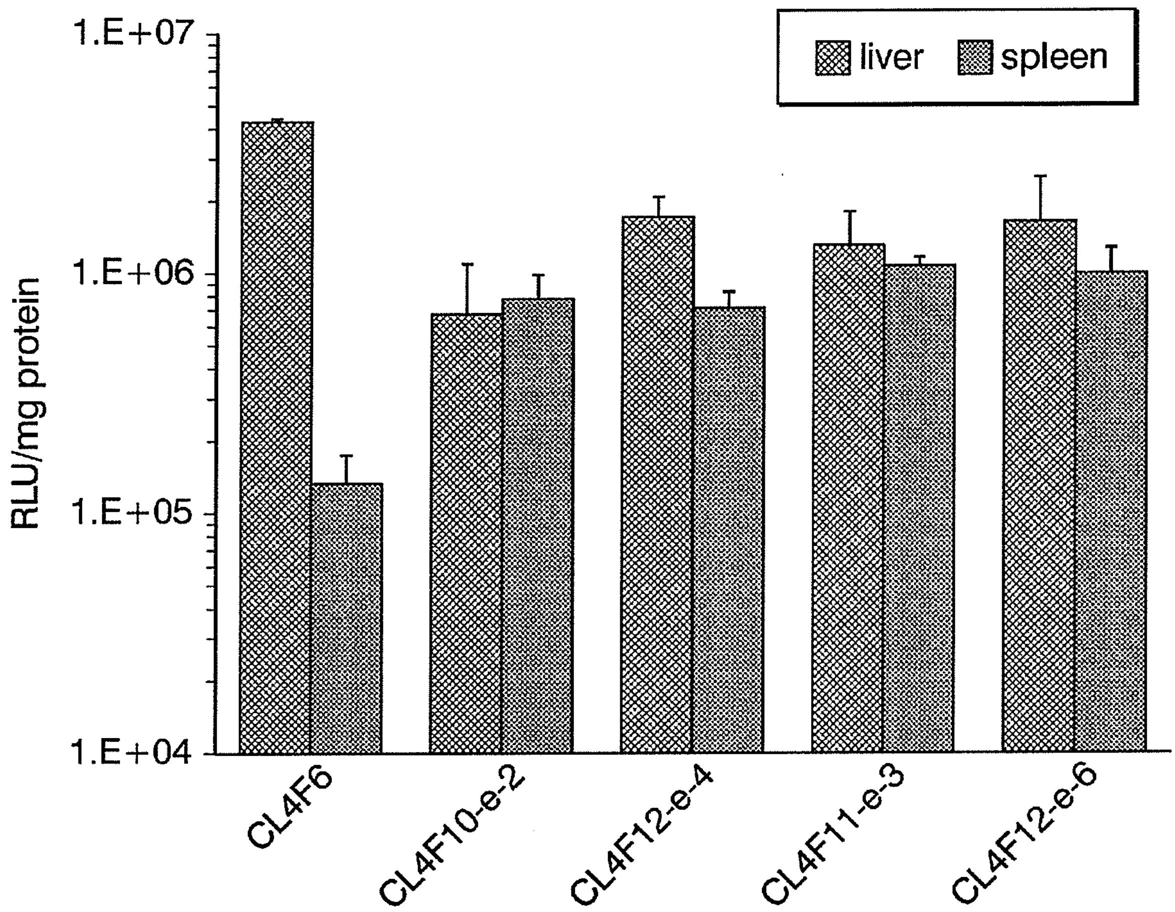
1.E+07
1.E+06
1.E+05
1.E+04
RLU/mg protein
liver
spleen
CL4F6
CL4F10-e-2
CL4F12-e-4
CL4F11-e-3
CL4F12-e-6

[FIG. 3]

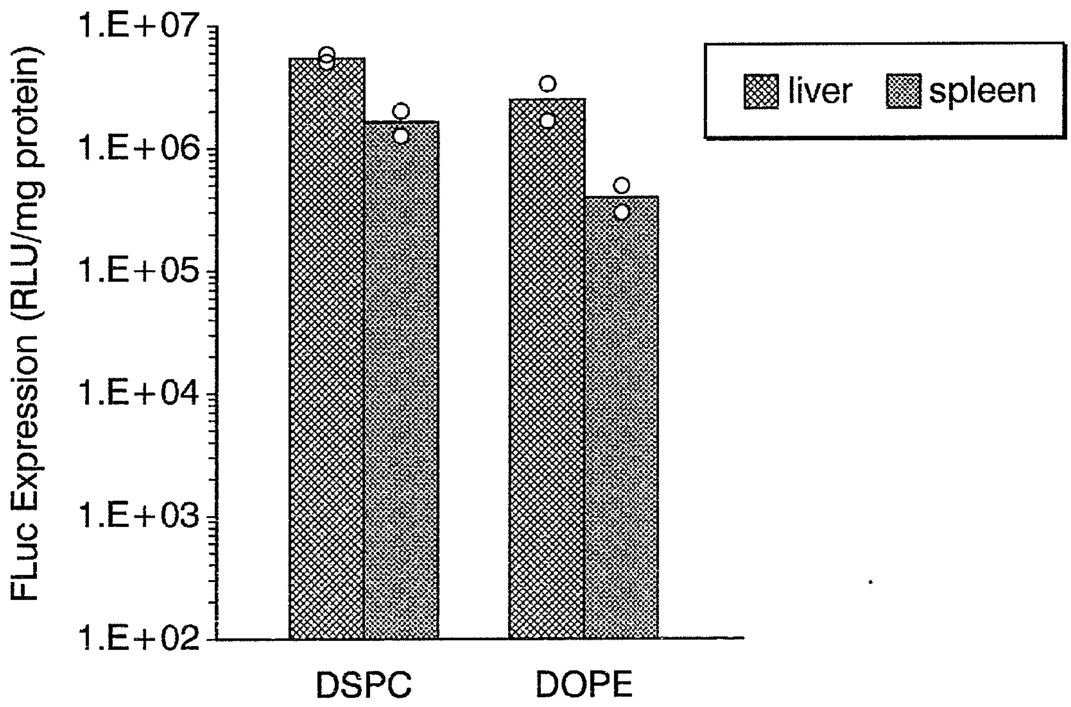
1.E+07
1.E+06
1.E+05
1.E+04
1.E+03
1.E+02
FLuc Expression (RLU/mg protein)
liver
spleen
DSPC
DOPE

[FIG. 4]

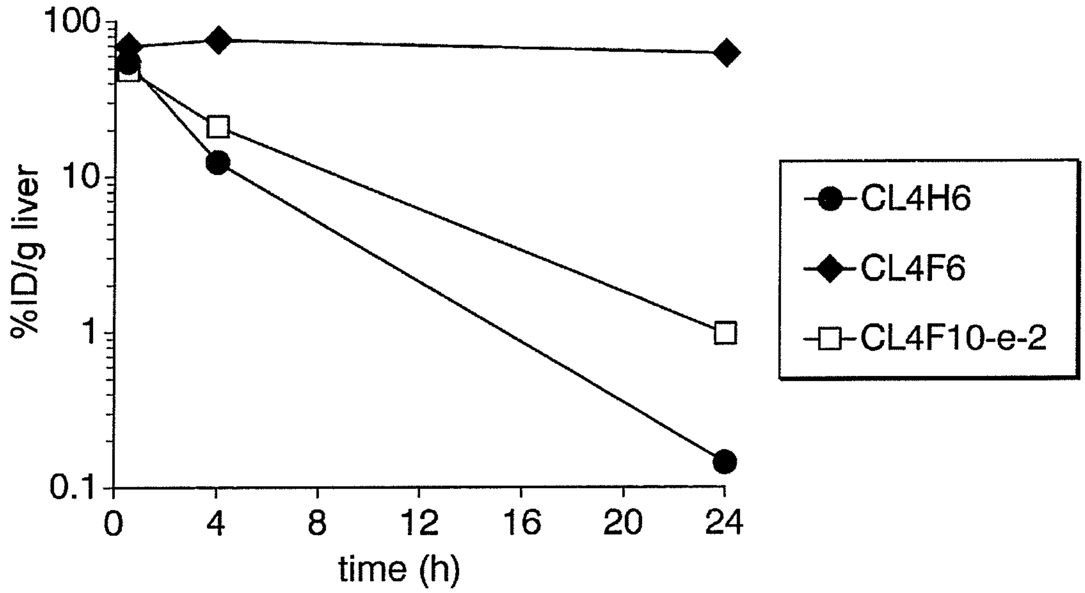
100
10
1
0.1
%ID/g liver
0
4
8
12
16
20
24
time (h)
CL4H6
CL4F6
CL4F10-e-2

[FIG. 5]

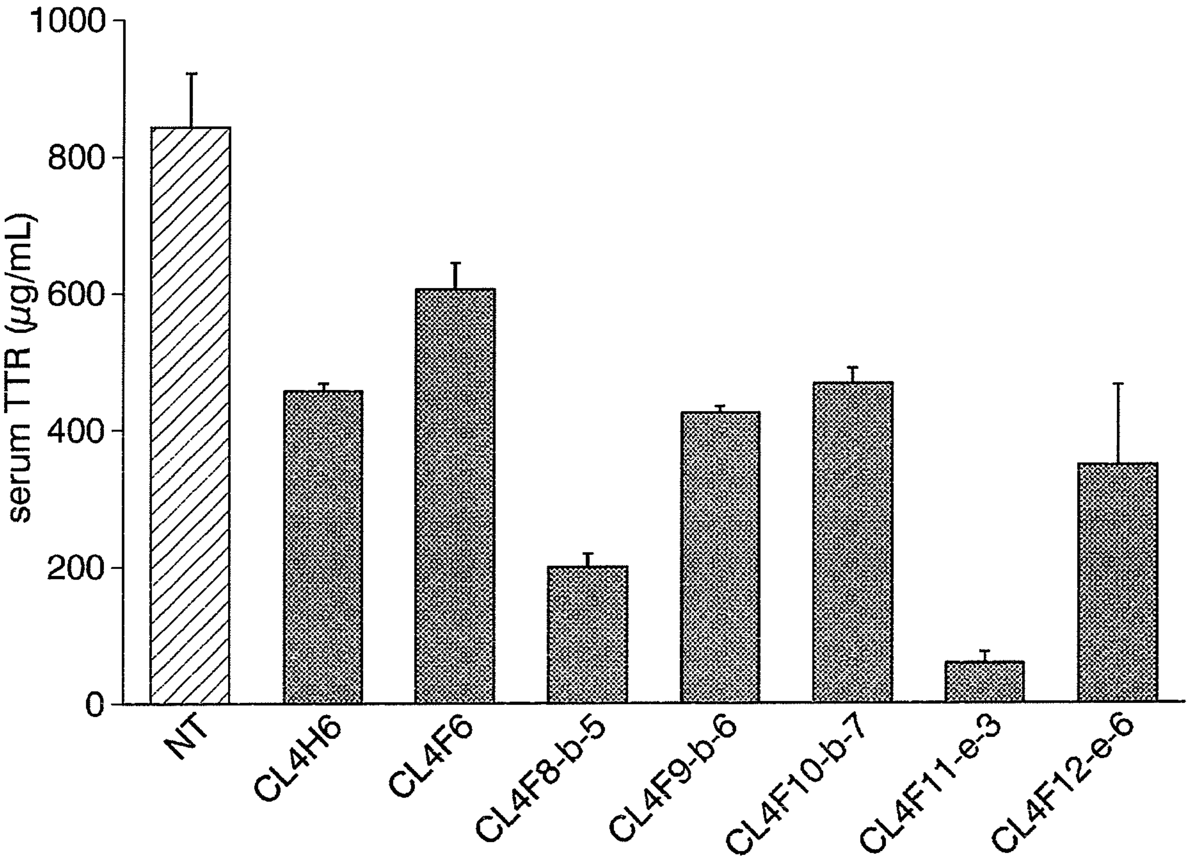
1000
800
600
400
200
0
serum TTR (μg/mL)
NT
CL4H6
CL4F6
CL4F8-b-5
CL4F9-b-6
CL4F10-b-7
CL4F11-e-3
CL4F12-e-6

[FIG. 6]

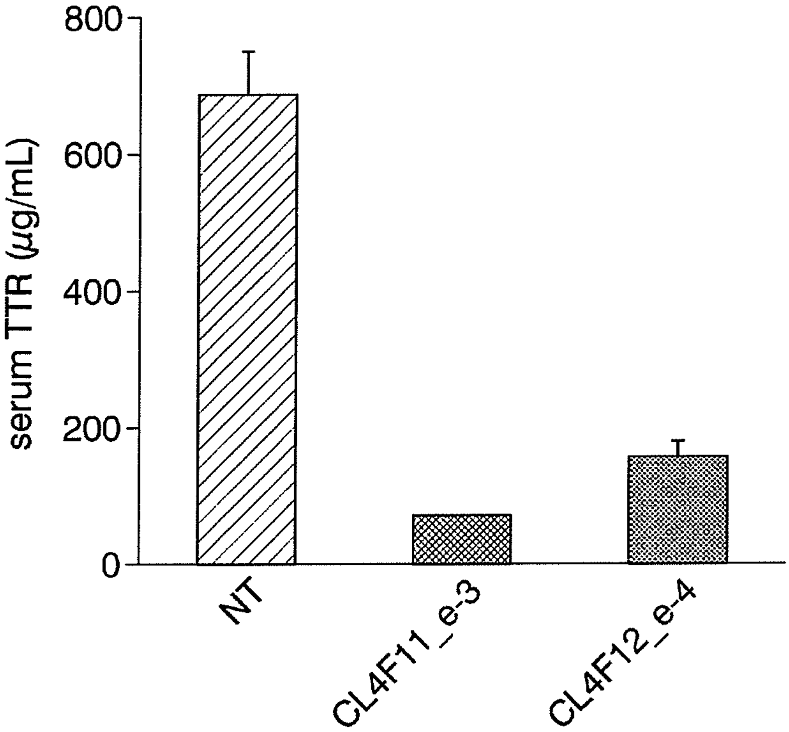
800
600
400
200
0
serum TTR (μg/mL)
NT
CL4F11_e-3
CL4F12_e-4

[FIG. 7]

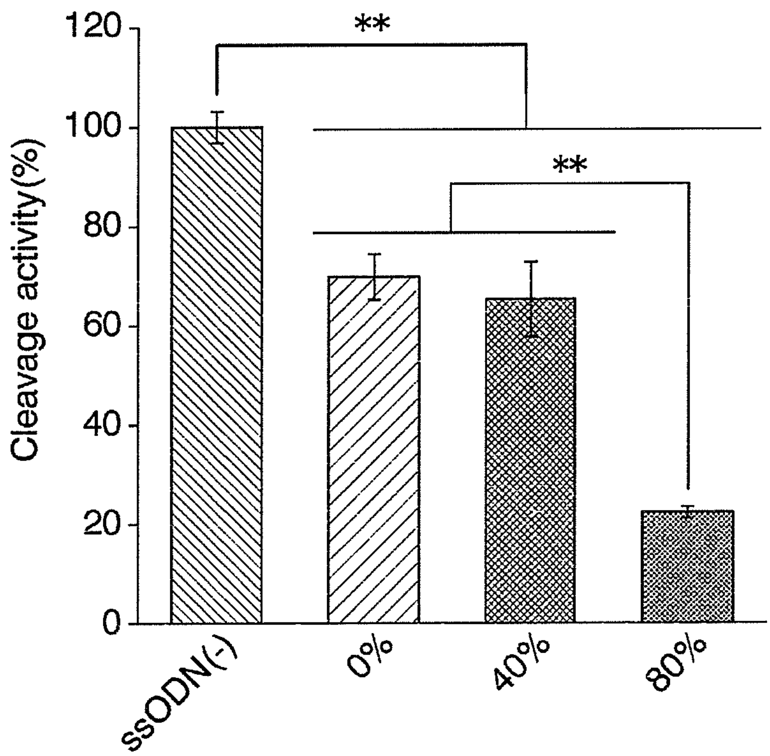
120
100
80
60
40
20
0
Cleavage activity(%)
**
**
ssODN(-)
0%
40%
80%

[FIG. 8]

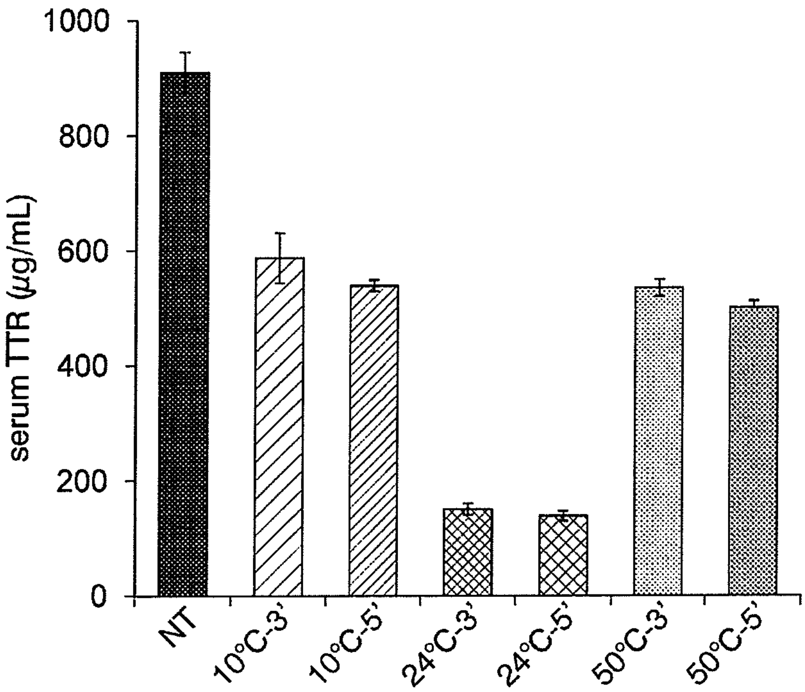

1000
800
600
400
200
0
serum TTR (μg/mL)
NT
10°C-3'
10°C-5'
24°C-3'
24°C-5'
50°C-3'
50°C-5'


[FIG. 9]

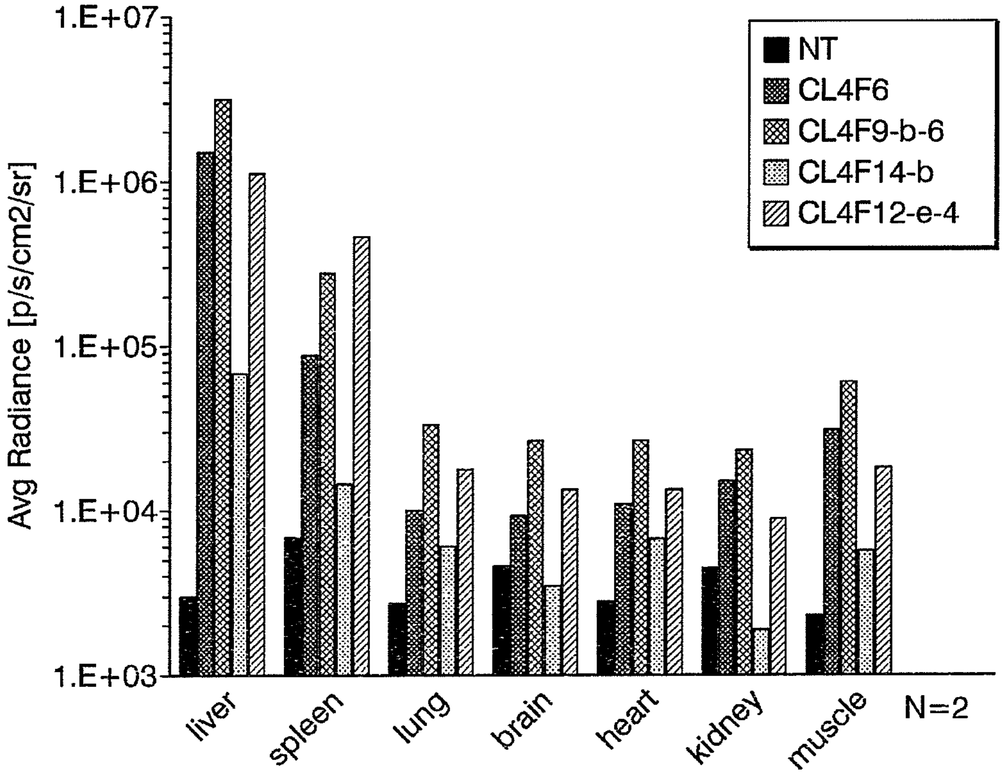

1.E+07
1.E+06
1.E+05
1.E+04
1.E+03
Avg Radiance [p/s/cm2/sr]
NT
CL4F6
CL4F9-b-6
CL4F14-b
CL4F12-e-4
liver
spleen
lung
brain
heart
kidney
muscle
N=2

[FIG. 10]

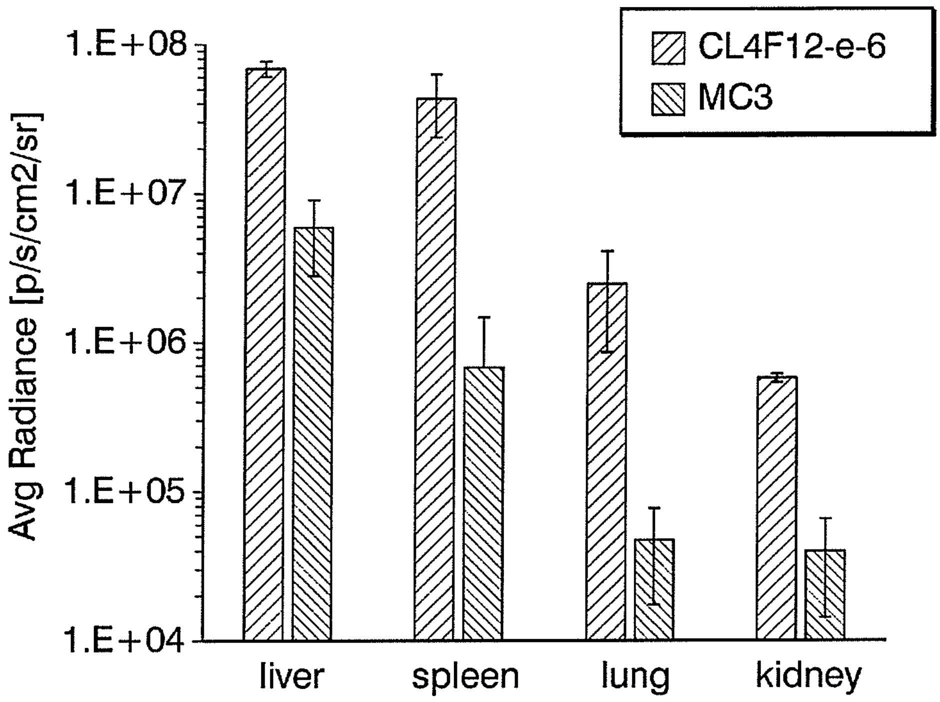


[FIG. 11]

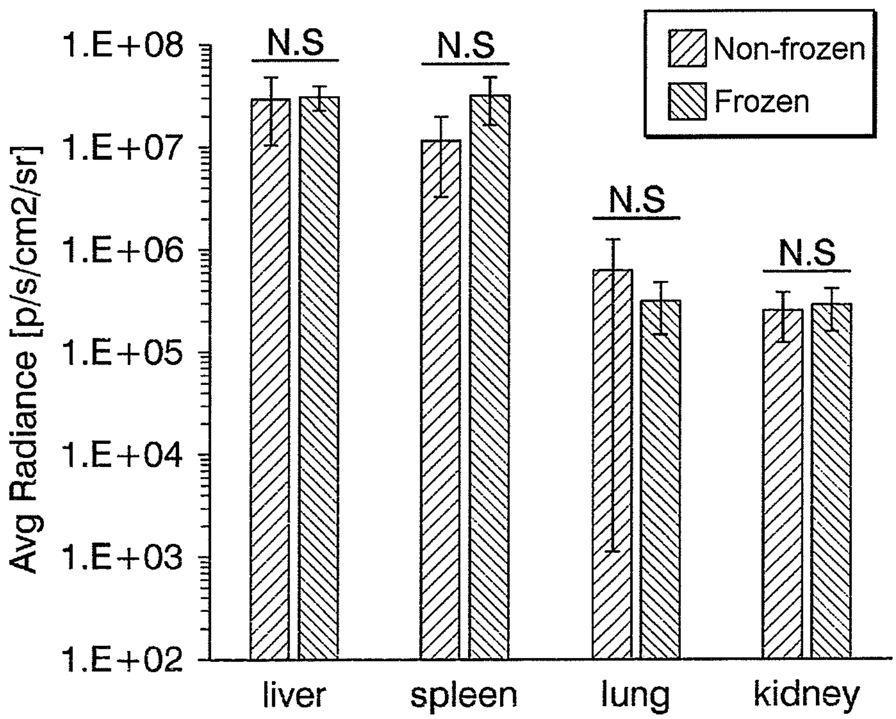


n=3, N.S: not significant, unpaired t-test

[FIG. 12]

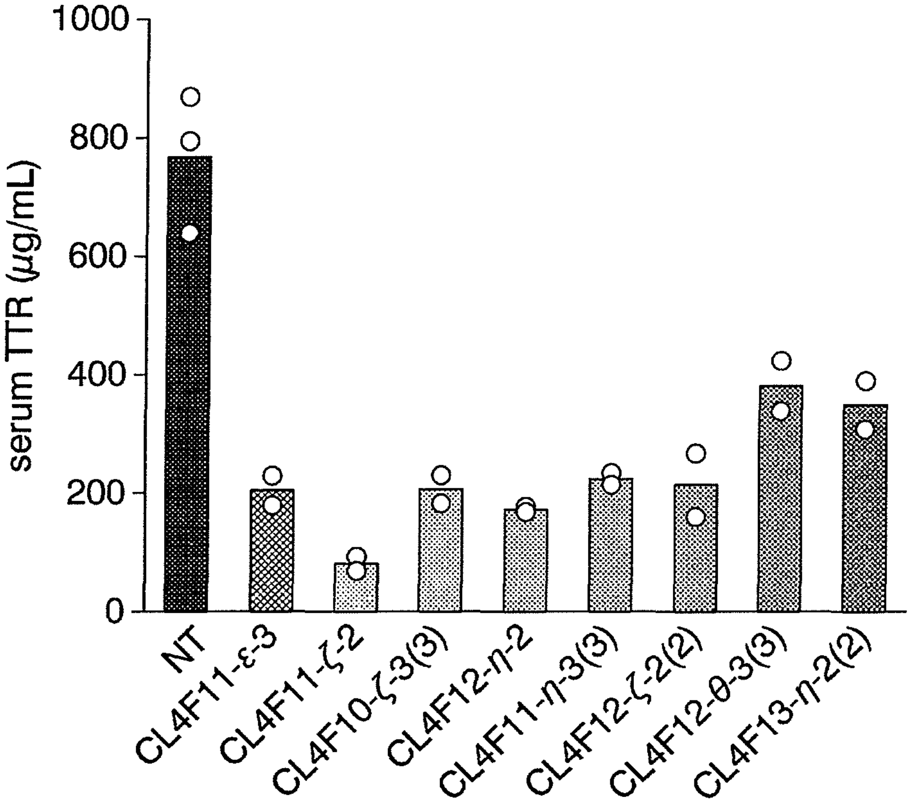


[FIG. 13]

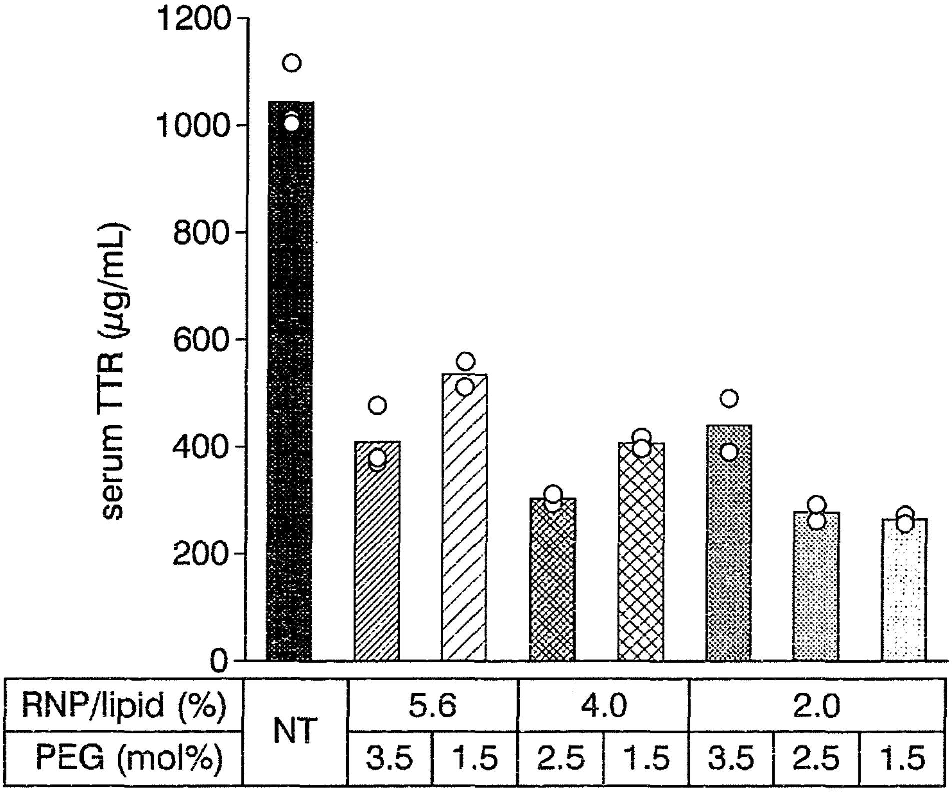


| RNP/lipid (%) | NT | 5.6 | | 4.0 | | 2.0 | | |
|---|---|---|---|---|---|---|---|---|
| PEG (mol%) | | 3.5 | 1.5 | 2.5 | 1.5 | 3.5 | 2.5 | 1.5 |

[FIG. 14]

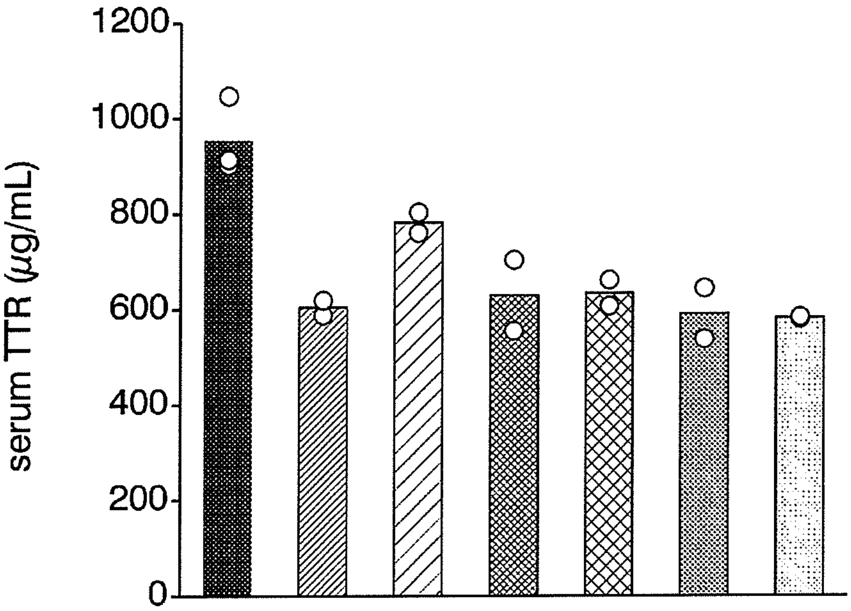


| CL (mol%) | NT | 40 | | 50 | | 60 | |
|---|---|---|---|---|---|---|---|
| PC (mol%) | | 5 | 10 | 5 | 10 | 5 | 10 |

[FIG. 15]

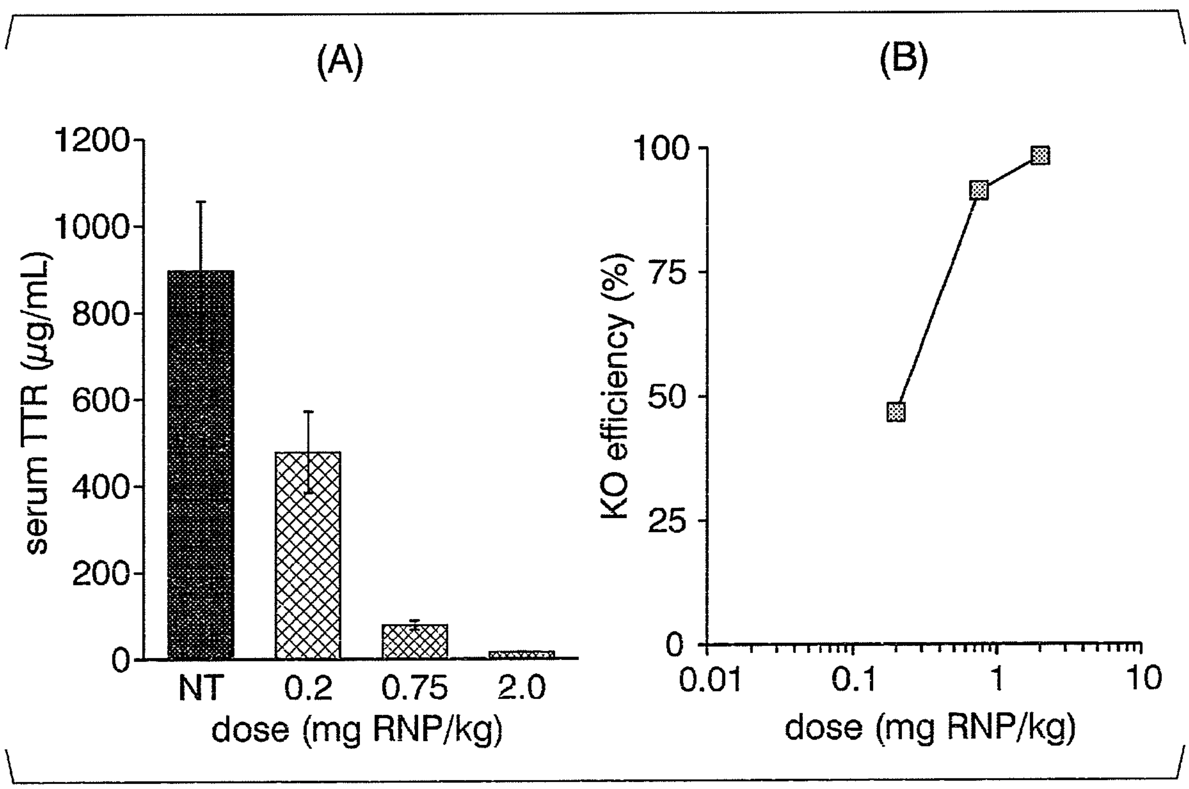

[FIG. 16]

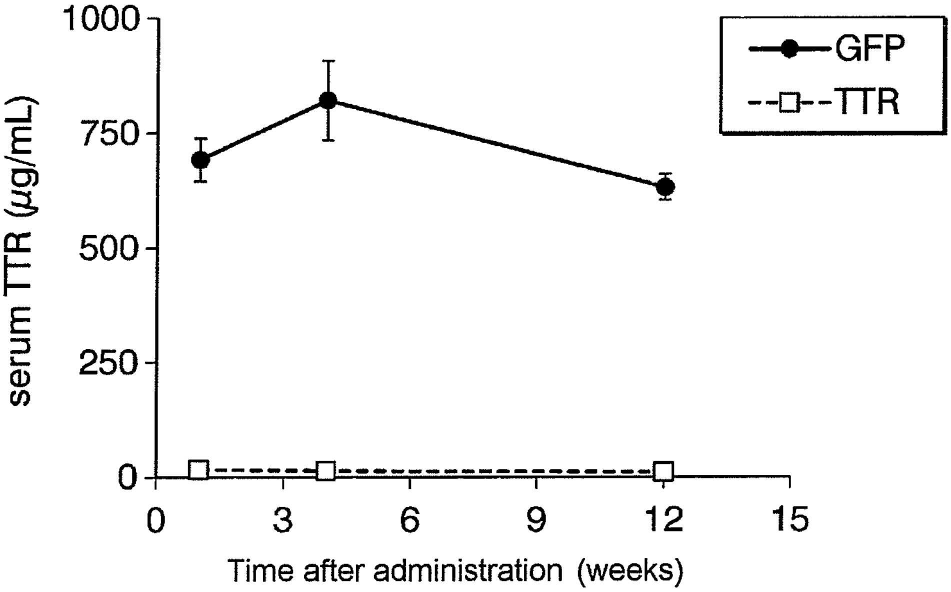


[FIG. 17]

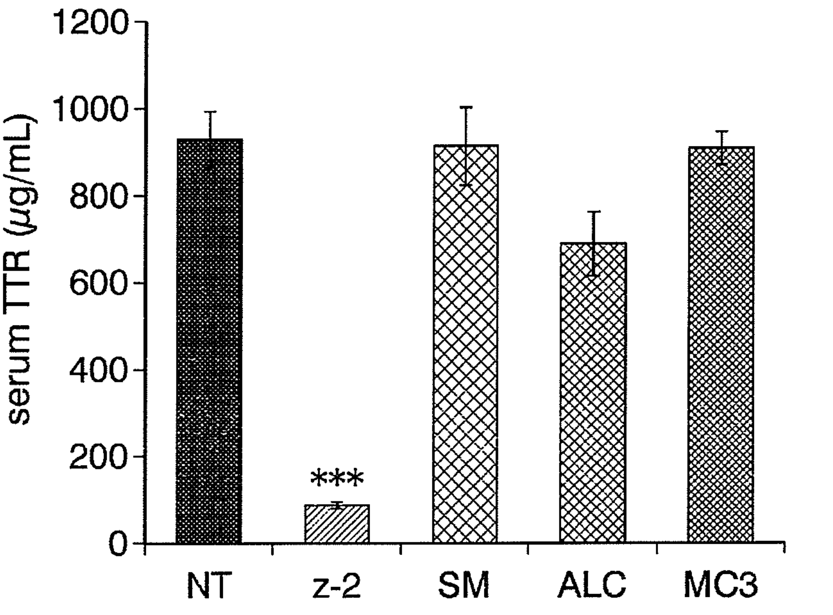


***p<0.001 (Tukey, vs. NT, SM, ALC, MC3)

[FIG. 18]

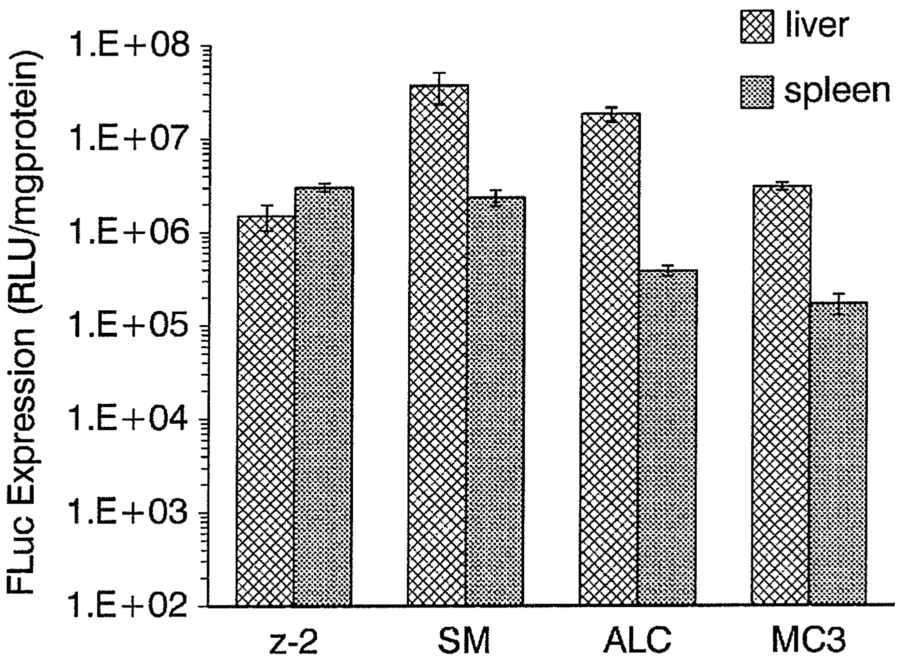


[FIG. 19]

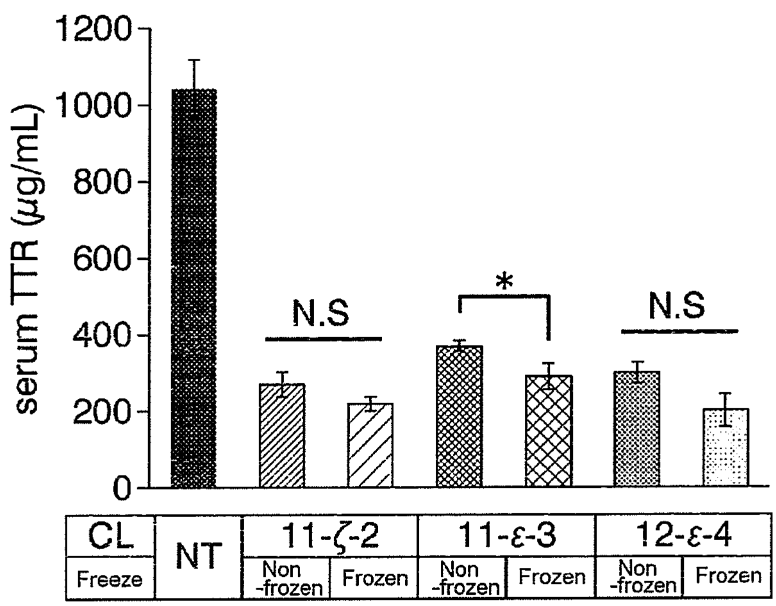


Unpaired Student's t-test, p* < 0.05, N.S: not significant

N=3

[FIG. 20]

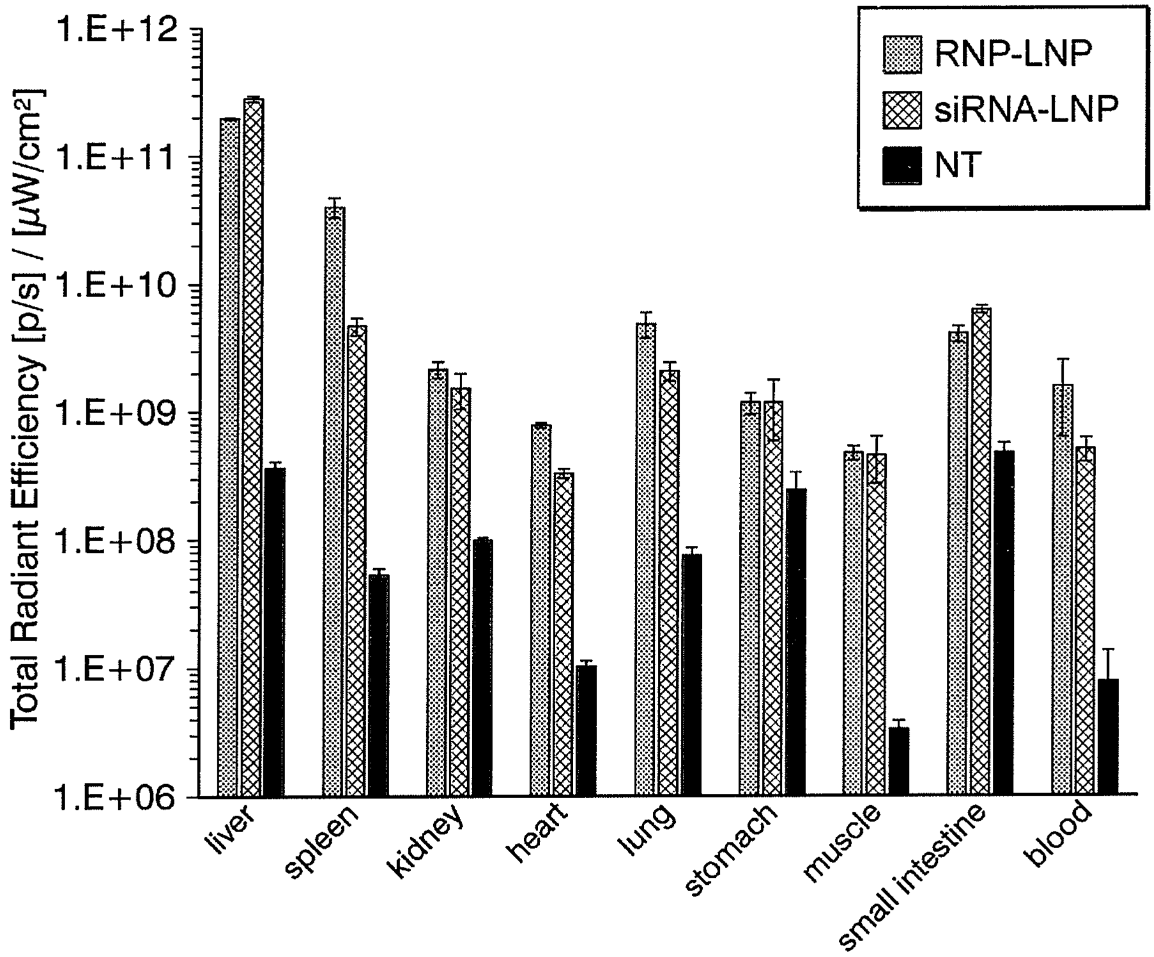

1.E+12
1.E+11
1.E+10
1.E+09
1.E+08
1.E+07
1.E+06
Total Radiant Efficiency [p/s] / [μW/cm²]
RNP-LNP
siRNA-LNP
NT
liver
spleen
kidney
heart
lung
stomach
muscle
small intestine
blood


[FIG. 21]

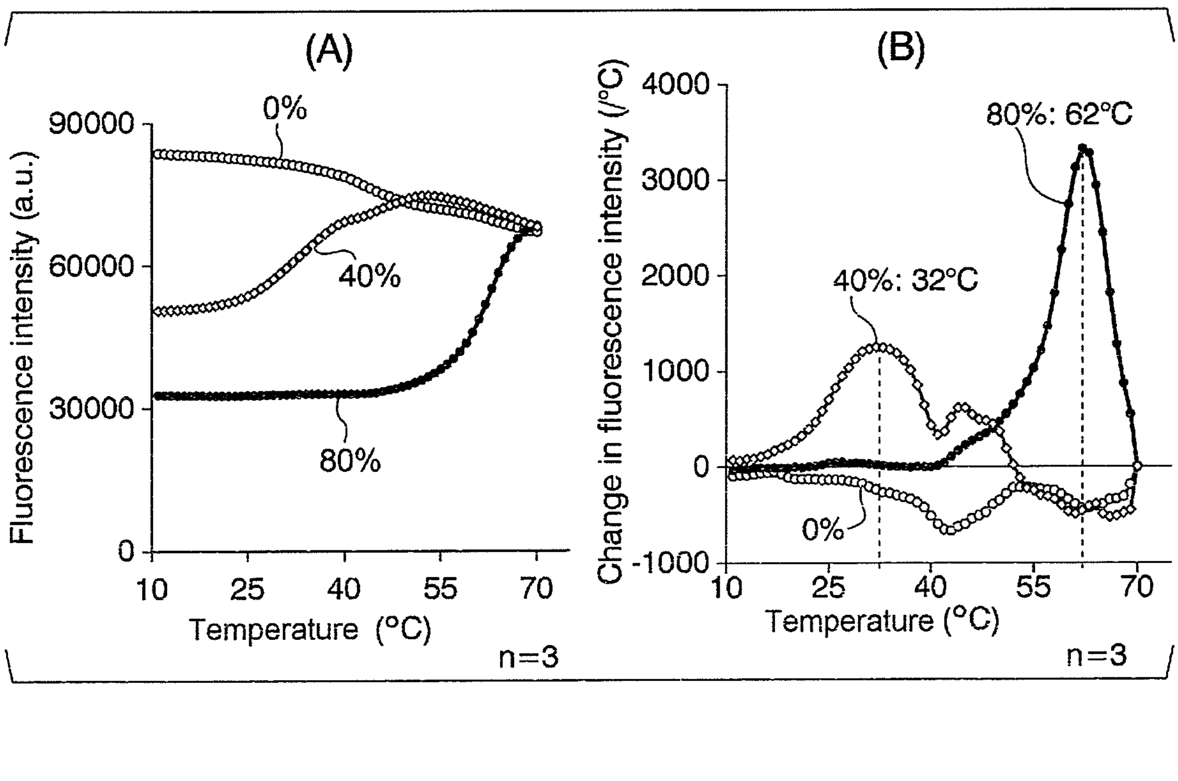

(A)
0%
40%
80%
90000
60000
30000
0
Fluorescence intensity (a.u.)
10
25
40
55
70
Temperature (°C)
n=3
(B)
4000
3000
2000
1000
0
-1000
Change in fluorescence intensity (/°C)
80%: 62°C
40%: 32°C
0%
10
25
40
55
70
Temperature (°C)
n=3

[FIG. 22]

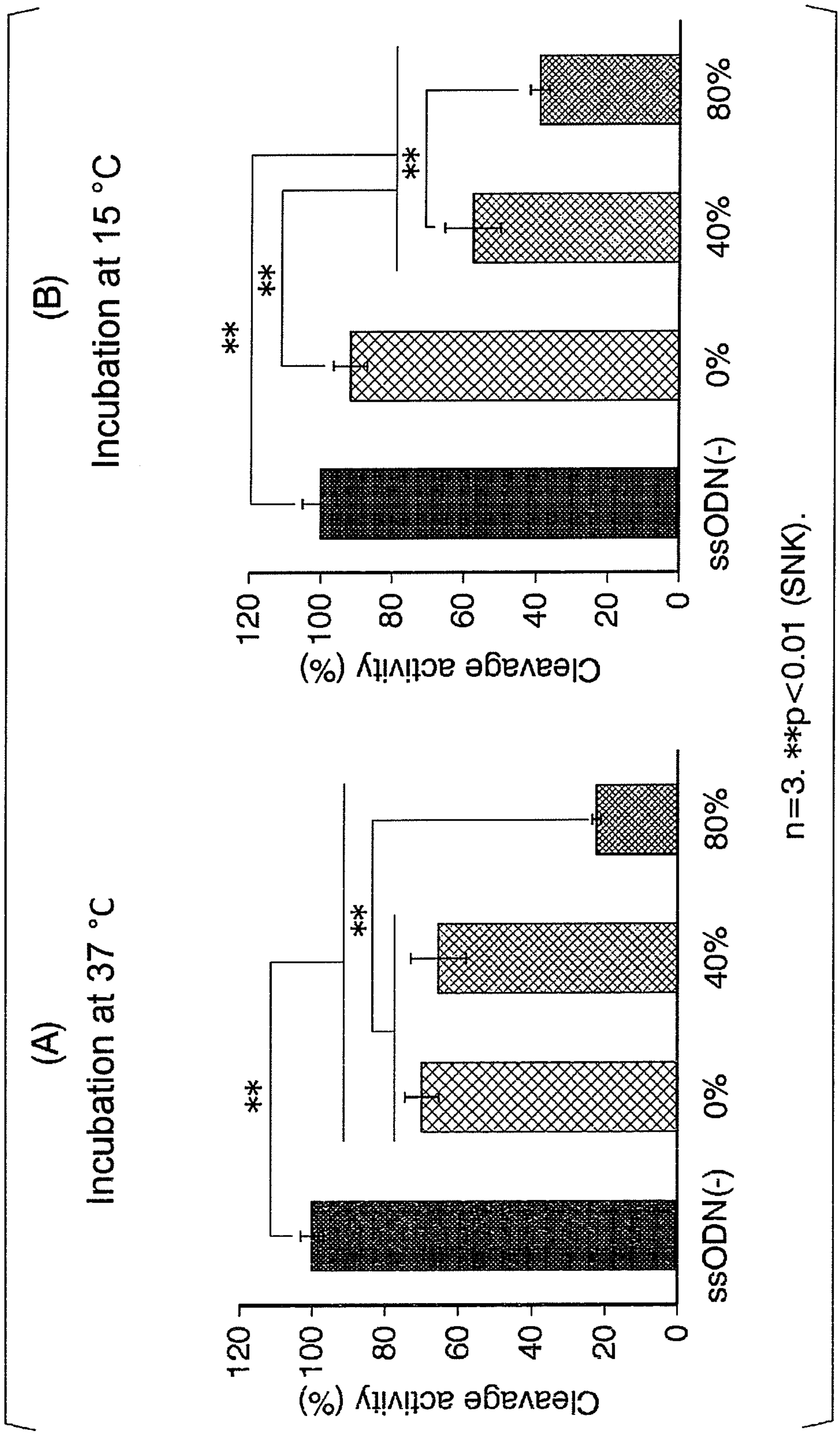
(A)
Incubation at 37 °C
Cleavage activity (%)
120
100
80
60
40
20
0
ssODN(-)
0%
40%
80%
**
**
(B)
Incubation at 15 °C
Cleavage activity (%)
120
100
80
60
40
20
0
ssODN(-)
0%
40%
80%
**
**
**
n=3. **p<0.01 (SNK).

[FIG. 23]

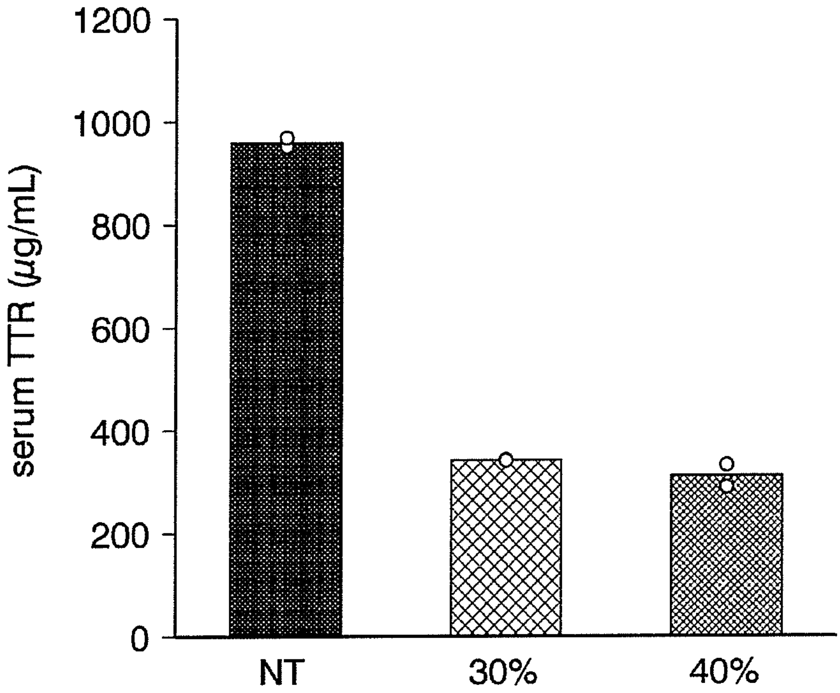

**INTERNATIONAL SEARCH REPORT**

International application No.
**PCT/JP2023/003844**

**A. CLASSIFICATION OF SUBJECT MATTER**

***A61K 47/18***(2017.01)i; ***A61K 9/127***(2006.01)i; ***A61K 9/51***(2006.01)i; ***A61K 31/7088***(2006.01)i; ***A61K 31/7105***(2006.01)i; ***A61K 31/713***(2006.01)i; ***A61K 48/00***(2006.01)i; ***A61P 1/16***(2006.01)i; ***A61P 43/00***(2006.01)i; ***C07C 219/06***(2006.01)i; ***C12N 15/09***(2006.01)i; ***C12N 15/113***(2010.01)i; ***C12N 15/88***(2006.01)i

FI: A61K47/18; C12N15/09 110; C12N15/88 Z; A61K9/51; A61K48/00; A61K31/7088; A61K31/7105; A61K31/713; A61P43/00 111; A61P43/00 105; A61P1/16; A61K9/127; C07C219/06; C12N15/113 Z ZNA

According to International Patent Classification (IPC) or to both national classification and IPC

**B. FIELDS SEARCHED**

Minimum documentation searched (classification system followed by classification symbols)

A61K47/18; A61K9/127; A61K9/51; A61K31/7088; A61K31/7105; A61K31/713; A61K48/00; A61P1/16; A61P43/00; C07C219/06; C12N15/09; C12N15/113; C12N15/88

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| P, A | 佐藤悠介他. II-PM-24 CRISPR/Cas9 RNP搭載脂質ナノ粒子によるin vivo遺伝子ノックアウト. 第38回日本DDS学会学術集会プログラム予稿集. 10 June 2022, p. 122, non-official translation (SATO, Yusuke et al. II-PM-24: In vivo Gene Knockout Using CRISPR/Cas9 RNP-Equipped Lipid Nanoparticles. Proceedings of the 38th Annual Meeting of the Japan Society of Drug Delivery System.) entire text | 1-16 |
| A | WO 2020/241679 A1 (HOKKAIDO UNIVERSITY) 03 December 2020 (2020-12-03) entire text | 1-16 |
| A | WO 2018/230710 A1 (HOKKAIDO UNIVERSITY) 20 December 2018 (2018-12-20) entire text | 1-16 |

☑ Further documents are listed in the continuation of Box C.

☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 March 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/003844**

**C. DOCUMENTS CONSIDERED TO BE RELEVANT**

| Category* | Citation of document, with indication, where appropriate, of the relevant passages | Relevant to claim No. |
|---|---|---|
| A | 佐藤悠介他. 2-C-17 CRISPR/Casタンパク質搭載脂質ナノ粒子の開発. 第36回日本DDS学会学術集会プログラム予稿集. 10 August 2020, p. 162, (SATO, Yusuke et al. 2-C-17: Development of CRISPR/Cas Protein-Equipped Lipid Nanoparticles. Proceedings of the 36th Annual Meeting of the Japan Society of Drug Delivery System.) entire text | 1-16 |
| A | 小沼はるの他. 1P-39 Cas RNP搭載脂質ナノ粒子による高効率なゲノム編集の実現. 日本核酸医薬学会第6回年会講演要旨集(CD-ROM). 2021, p. 121, (KONUMA, Haruno et al. 1P-39: Realization of High-Performance Genome Editing Using Cas RNP-Equipped Lipid Nanoparticles. Proceedings of the 6th Annual Meeting of the Nucleic Acids Therapeutics Society of Japan.) entire text | 1-16 |
| A | SUZUKI, Y. et al. Lipid nanoparticles loaded with ribonucleoprotein-oligonucleotide complexes synthesized using a microfluidic device exhibit robust genome editing and hepatitis B virus inhibition. Journal of Controlled Release. 2021, 330, pp. 61-71 entire text | 1-16 |

Form PCT/ISA/210 (second sheet) (January 2015)

**INTERNATIONAL SEARCH REPORT**

International application No.
**PCT/JP2023/003844**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "Annex C/ST.25 text file format" should be read as as "ST.26 format".

Form PCT/ISA/210 (continuation of first sheet) (January 2015)

**INTERNATIONAL SEARCH REPORT**
**Information on patent family members**

International application No.
**PCT/JP2023/003844**

| Patent document cited in search report | Publication date (day/month/year) | Patent family member(s) | Publication date (day/month/year) |
|---|---|---|---|
| WO 2020/241679 A1 | 03 December 2020 | US 2022/0213509 A1 | |
| | | EP 3967649 A1 | |
| | | KR 10-2022-0015385 A | |
| | | CN 114174522 A | |
| WO 2018/230710 A1 | 20 December 2018 | US 2020/0129431 A1 | |
| | | EP 3640237 A1 | |
| | | CN 110740985 A | |
| | | KR 10-2020-0018782 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022060960 A
- WO 2018230710 A
- WO 2018190423 A
- WO 2007102481 A


### Non-patent literature cited in the description


- **JAYARAMAN et al.** *Angewandte Chemie International Edition*, 2012, vol. 51, 8529-8533
- **WATANABE et al.** *Scientific Reports*, 2014, vol. 4, 4750
- **YAMAMOTO et al.** *Journal of Hepatology*, 2016, vol. 64, 547-555
- **SATO et al.** *Molecular Therapy*, 2016, vol. 24, 788-795
- **MAIER et al.** *Molecular Therapy*, 2013, vol. 21 (8), 1570-1578
- **WITTRUP et al.** *Nature Biotechnology*, 2015, vol. 33 (8), 870-876
- **XU et al.** *Molecular Pharmaceutics*, 2014, vol. 11, 1424-1434
- **GILLERON et al.** *Nature Biotechnology*, 2013, vol. 31 (7), 638-646
- **LEUNG et al.** *Journal of Physical Chemistry C Nanomater Interfaces*, 2012, vol. 116 (34), 18440-18450